(19) Europäisches Patentamt
European Patent Office
Office européen des brevets

(11) **EP 3 505 041 B1**

(12) **EUROPEAN PATENT SPECIFICATION**

(45) Date of publication and mention
of the grant of the patent:
**25.06.2025 Bulletin 2025/26**

(21) Application number: **18193605.5**

(22) Date of filing: **11.09.2018**

(51) International Patent Classification (IPC):
*A61B 1/00* (2006.01)    *A61B 1/06* (2006.01)
*H04N 13/214* (2018.01)   *G06T 7/521* (2017.01)
*H04N 13/25* (2018.01)    *G01B 9/02* (2022.01)
*A61B 1/05* (2006.01)    *H04N 1/00* (2006.01)
*G16H 30/20* (2018.01)

(52) Cooperative Patent Classification (CPC):
**A61B 1/000094; A61B 1/00181; A61B 1/00193;
A61B 1/05; A61B 1/0638; A61B 1/0655;
H04N 1/00167; G16H 30/20**

(54) **DUAL CMOS ARRAY IMAGING**

DUAL-CMOS-ARRAY-BILDGEBUNG

IMAGERIE À DOUBLE RÉSEAU CMOS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.12.2017  US 201762611340 P
28.12.2017  US 201762611341 P
28.12.2017  US 201762611339 P
28.03.2018  US 201862649291 P
29.03.2018  US 201815940742**

(43) Date of publication of application:
**03.07.2019 Bulletin 2019/27**

(73) Proprietor: **Ethicon LLC
Guaynabo 00969 (PR)**

(72) Inventors:
• **SHELTON, IV, Frederick E.**
**Cincinnati, OH 45242 (US)**
• **HARRIS, Jason L.**
**Cincinnati, OH 45242 (US)**
• **YATES, David C.**
**Cincinnati, OH 45242 (US)**
• **MORGAN, Jerome R.**
**Cincinnati, OH 45242 (US)**
• **PARFETT, Raymond E.**
**Cincinnati, OH 45242 (US)**

(74) Representative: **Carpmaels & Ransford LLP
One Southampton Row
London WC1B 5HA (GB)**

(56) References cited:
EP-A- 2 133 021       EP-A1- 2 241 244
EP-A1- 3 225 151      JP-A- 2005 334 647
US-A1- 2006 122 580   US-A1- 2017 079 530
US-B1- 8 027 710      US-B2- 9 232 883

• MATTHIJS DRAIJER ET AL: "Review of laser
speckle contrast techniques for visualizing
tissue perfusion", LASERS IN MEDICAL
SCIENCE, SPRINGER-VERLAG, LO, vol. 24, no.
4, 3 December 2008 (2008-12-03), pages 639 -
651, XP019663605, ISSN: 1435-604X

**Description**

BACKGROUND

The present disclosure relates to various surgical systems. Surgical procedures are typically performed in surgical operating theaters or rooms in a healthcare facility such as, for example, a hospital. A sterile field is typically created around the patient. The sterile field may include the scrubbed team members, who are properly attired, and all furniture and fixtures in the area. Various surgical devices and systems are utilized in performance of a surgical procedure. US 8027710 B1 describes an imaging system for use with minimally invasive surgery. The imaging system comprises a platform which is anchored to a wall of a patient cavity. The platform comprises a plurality of transceivers which transmit energy toward the surgical site and receive reflected energy. The transceivers transmit rasterized image information to a computer which then displays the image information in real time, enabling a surgeon to view the surgical site on a display device. The transceivers can transmit and receive different types of energy, and the computer can then combine image information from the various different energy types to provide overlaid images of the surgical site. In addition, the computer controls the relative positions of the transceivers so that the images provided to the surgeon will not be affected by movement of the platform or by movement of structures (i.e., organs) at the surgical site. EP3225151 A1 describes a surgical apparatus including an endoscope, a camera, a light source, and a structured light pattern source. The endoscope includes an elongate body having a plurality of segments manipulatable relative to one another. The camera, light source, and structured light pattern source cooperate to determine the topography of a surface within a patient. A method of performing surgery is also provided.

SUMMARY

The present invention provides minimally invasive image acquisition systems and non-transitory computer readable media according to the appended claims.

FIGURES

The features of various aspects are set forth with particularity in the appended claims. The various aspects, however, both as to organization and methods of operation, together with further objects and advantages thereof, may best be understood by reference to the following description, taken in conjunction with the accompanying drawings as follows.

FIG. 1 is a block diagram of a computer-implemented interactive surgical system.
FIG. 2 is a surgical system being used to perform a surgical procedure in an operating room.
FIG. 3 is a surgical hub paired with a visualization system, a robotic system, and an intelligent instrument.
FIG. 4 is a partial perspective view of a surgical hub enclosure, and of a combo generator module slidably receivable in a drawer of the surgical hub enclosure.
FIG. 5 is a perspective view of a combo generator module with bipolar, ultrasonic, and monopolar contacts and a smoke evacuation component.
FIG. 6 illustrates individual power bus attachments for a plurality of lateral docking ports of a lateral modular housing configured to receive a plurality of modules.
FIG. 7 illustrates a vertical modular housing configured to receive a plurality of modules.
FIG. 8 illustrates a surgical data network comprising a modular communication hub configured to connect modular devices located in one or more operating theaters of a healthcare facility, or any room in a healthcare facility specially equipped for surgical operations, to the cloud.
FIG. 9 illustrates a computer-implemented interactive surgical system.
FIG. 10 illustrates a surgical hub comprising a plurality of modules coupled to the modular control tower.
FIG. 11 illustrates one aspect of a Universal Serial Bus (USB) network hub device.
FIG. 12 illustrates a logic diagram of a control system of a surgical instrument or tool.
FIG. 13 illustrates a control circuit configured to control aspects of the surgical instrument or tool.
FIG. 14 illustrates a combinational logic circuit configured to control aspects of the surgical instrument or tool.
FIG. 15 illustrates a sequential logic circuit configured to control aspects of the surgical instrument or tool.
FIG. 16 illustrates a surgical instrument or tool comprising a plurality of motors which can be activated to perform various functions.
FIG. 17 is a schematic diagram of a robotic surgical instrument configured to operate a surgical tool described herein.
FIG. 18 illustrates a block diagram of a surgical instrument programmed to control the distal translation of a displacement member.
FIG. 19 is a schematic diagram of a surgical instrument configured to control various functions.
FIG. 20 is a simplified block diagram of a generator configured to provide inductorless tuning, among other benefits.
FIG. 21 illustrates an example of a generator, which is one form of the generator of FIG. 20.
FIG. 22A illustrates a visualization system that may be incorporated into a surgical system according to the invention.

FIG. 22B illustrates a top plan view of a hand unit of the visualization system of FIG. 22A.

FIG. 22C illustrates a side plan view of the hand unit depicted in FIG. 22A along with an imaging sensor disposed therein.

FIG. 22D illustrates a plurality of an imaging sensors a depicted in FIG. 22C.

FIG. 23A illustrates a plurality of laser emitters that may be incorporated in the visualization system of FIG. 22A.

FIG. 23B illustrates illumination of an image sensor having a Bayer pattern of color filters.

FIG. 23C illustrates a graphical representation of the operation of a pixel array for a plurality of frames.

FIG. 23D illustrates a schematic of an example of an operation sequence of chrominance and luminance frames.

FIG. 23E illustrates an example of sensor and emitter patterns.

FIG. 23F illustrates a graphical representation of the operation of a pixel array.

FIG. 24 illustrates a schematic of one example of instrumentation for NIR spectroscopy.

FIG. 25 illustrates schematically one example of instrumentation for determining NIRS based on Fourier transform infrared imaging.

FIGS. 26A-C illustrate a change in wavelength of light scattered from moving blood cells, in accordance with a system according to the invention.

FIG. 27 illustrates an aspect of instrumentation that may be used to detect a Doppler shift in laser light scattered from portions of a tissue, in accordance with a system according to the invention.

FIG. 28 illustrates schematically some optical effects on light impinging on a tissue having subsurface structures, in accordance with a system according to the invention.

FIG. 29 illustrates an example of the effects on a Doppler analysis of light impinging on a tissue sample having subsurface structures, in accordance with a system according to the invention.

FIGS. 30A-C illustrate schematically the detection of moving blood cells at a tissue depth based on a laser Doppler analysis at a variety of laser wavelengths, in accordance with a system according to the invention.

FIG. 30D illustrates the effect of illuminating a CMOS imaging sensor with a plurality of light wavelengths over time, in accordance with a system according to the invention.

FIG. 31 illustrates an example of a use of Doppler imaging to detect the present of subsurface blood vessels, in accordance with a system according to the invention.

FIG. 32 illustrates a method to identify a subsurface blood vessel based on a Doppler shift of blue light due to blood cells flowing therethrough, in accordance with a system according to the invention.

FIG. 33 illustrates schematically localization of a deep subsurface blood vessel, in accordance with a system according to the invention.

FIG. 34 illustrates schematically localization of a shallow subsurface blood vessel, in accordance with a system according to the invention.

FIG. 35 illustrates a composite image comprising a surface image and an image of a subsurface blood vessel, in accordance with a system according to the invention.

FIG. 36 is a flow chart of a method for determining a depth of a surface feature in a piece of tissue.

FIG. 37 illustrates the effect of the location and characteristics of non-vascular structures on light impinging on a tissue sample.

FIG. 38 schematically depicts one example of components used in a full field OCT device.

FIG. 39 illustrates schematically the effect of tissue anomalies on light reflected from a tissue sample.

FIG. 40 illustrates an image display derived from a combination of tissue visualization modalities.

FIGS. 41A-C illustrate several aspects of displays that may be provided to a surgeon for a visual identification of a combination of surface and sub-surface structures of a tissue in a surgical site.

FIG. 42 is a flow chart of a method for providing information related to a characteristic of a tissue to a smart surgical instrument.

FIGS. 43A and 43B illustrate a multi-pixel light sensor receiving by light reflected by a tissue illuminated by sequential exposure to red, green, blue, and infra red light, and red, green, blue, and ultraviolet laser light sources, respectively.

FIGS. 44A and 44B illustrate the distal end of an elongated camera probe having a single light sensor and two light sensors, respectively.

FIG. 44C illustrates a perspective view of an example of a monolithic sensor having a plurality of pixel arrays.

FIG. 45 illustrates one example of a pair of fields of view available to two image sensors of an elongated camera probe.

FIGS. 46A-D illustrate additional examples of a pair of fields of view available to two image sensors of an elongated camera probe.

FIGS. 47A-C illustrate an example of the use of an imaging system incorporating the features disclosed in FIG 46D.

FIGS. 48A and 48B depict another example of the use of a dual imaging system.

FIGS. 49A-C illustrate examples of a sequence of surgical steps which may benefit from the use of multi-image analysis at the surgical site.

FIG. 50 is a timeline depicting situational awareness of a surgical hub.

DESCRIPTION

Before explaining various aspects of surgical devices and generators in detail, it should be noted that the illustrative examples are not limited in application or use to the details of construction and arrangement of parts illustrated in the accompanying drawings and description. The illustrative examples may be implemented or incorporated in other aspects, variations and modifications, and may be practiced or carried out in various ways. Further, unless otherwise indicated, the terms and expressions employed herein have been chosen for the purpose of describing the illustrative examples for the convenience of the reader and are not for the purpose of limitation thereof.

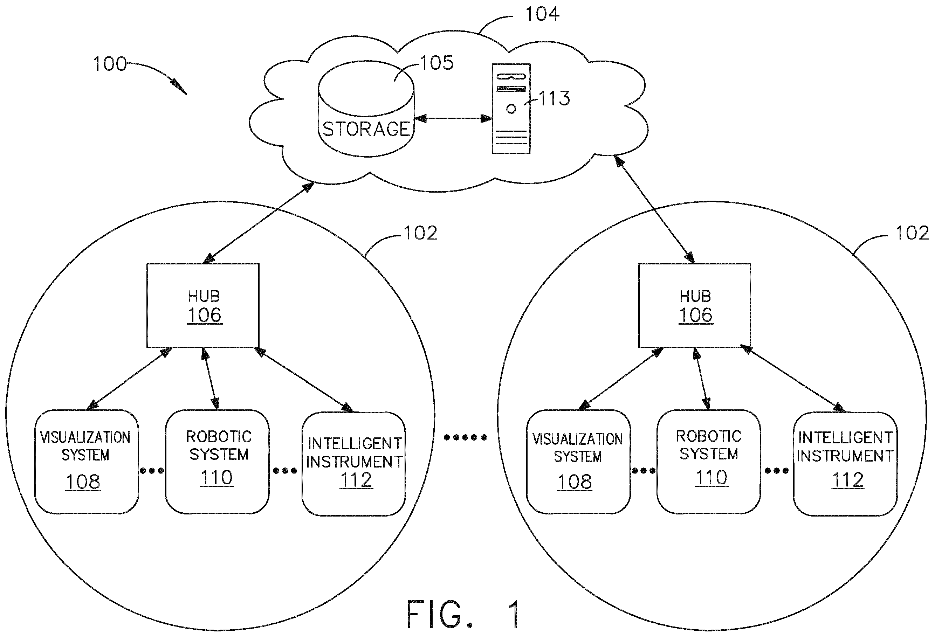

Referring to FIG. 1, a computer-implemented interactive surgical system 100 includes one or more surgical systems 102 and a cloud-based system (e.g., the cloud 104 that may include a remote server 113 coupled to a storage device 105). Each surgical system 102 includes at least one surgical hub 106 in communication with the cloud 104 that may include a remote server 113. As illustrated in FIG. 1, the surgical system 102 includes a visualization system 108, a robotic system 110, and a handheld intelligent surgical instrument 112, which are configured to communicate with one another and/or the hub 106. The surgical system 102 may include an M number of hubs 106, an N number of visualization systems 108, an O number of robotic systems 110, and a P number of handheld intelligent surgical instruments 112, where M, N, O, and P are integers greater than or equal to one.

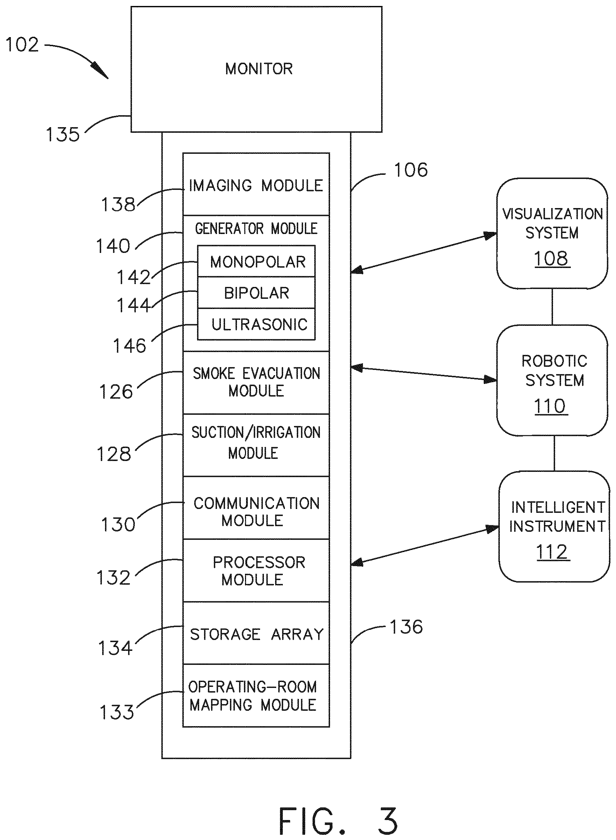

FIG. 3 depicts an example of a surgical system 102 being used to perform a surgical procedure on a patient who is lying down on an operating table 114 in a surgical operating room 116. A robotic system 110 is used in the surgical procedure as a part of the surgical system 102. The robotic system 110 includes a surgeon's console 118, a patient side cart 120 (surgical robot), and a surgical robotic hub 122. The patient side cart 120 manipulates at least one removably coupled surgical tool 117 through a minimally invasive incision in the body of the patient while the surgeon views the surgical site through the surgeon's console 118. An image of the surgical site is obtained by a medical imaging device 124, which is manipulated by the patient side cart 120 to orient the imaging device 124. The robotic hub 122 is used to process the images of the surgical site for subsequent display to the surgeon through the surgeon's console 118.

Other types of robotic systems can be readily adapted for use with the surgical system 102. Various examples of robotic systems and surgical tools that are suitable for use with the present disclosure are described in U.S. Provisional Patent Application Serial No. 62/611,339, titled ROBOT ASSISTED SURGICAL PLATFORM, filed December 28, 2017.

Various examples of cloud-based analytics that are performed by the cloud 104, and are suitable for use with the present disclosure, are described in U.S. Provisional Patent Application Serial No. 62/611,340, titled CLOUD-BASED MEDICAL ANALYTICS, filed December 28, 2017.

The imaging device 124 includes at least one image sensor and one or more optical components. Suitable image sensors include, but are not limited to, Charge-Coupled Device (CCD) sensors and Complementary Metal-Oxide Semiconductor (CMOS) sensors.

The optical components of the imaging device 124 include one or more illumination sources and/or one or more lenses. The one or more illumination sources are directed to illuminate portions of the surgical field. The one or more image sensors receive light reflected or refracted from the surgical field, including light reflected or refracted from tissue and/or surgical instruments.

The one or more illumination sources are configured to radiate electromagnetic energy in the visible spectrum as well as the invisible spectrum. The visible spectrum, sometimes referred to as the optical spectrum or luminous spectrum, is that portion of the electromagnetic spectrum that is visible to (i.e., can be detected by) the human eye and may be referred to as visible light or simply light. A typical human eye will respond to wavelengths in air that are from about 380 nm to about 750 nm.

The invisible spectrum (i.e., the non-luminous spectrum) is that portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

The imaging device 124 is configured for use in a minimally invasive procedure. Examples of imaging devices suitable for use with the present disclosure include, but not limited to, an arthroscope, angioscope, bronchoscope, choledochoscope, colonoscope, cytoscope, duodenoscope, enteroscope, esophagogastroduodenoscope (gastroscope), endoscope, laryngoscope, nasopharyngo-neproscope, sigmoidoscope, thoracoscope, and ureteroscope.

The imaging device employs multi-spectrum monitoring to discriminate topography and underlying structures. A multi-spectral image is one that captures image data within specific wavelength ranges across the electromagnetic spectrum. The wavelengths are separated by filters or by the use of instruments that are sensitive to particular wavelengths, including light from frequencies beyond the visible light range, e.g., IR and ultraviolet. Spectral imaging can allow extraction of additional information the human eye fails to capture with its receptors for red, green, and blue. The use of multi-spectral imaging is described in greater detail under the heading "Advanced Imaging Acquisition Module" in U.S. Provisional Patent Application Serial No. 62/611,341, titled INTERACTIVE SURGICAL PLATFORM, filed December 28, 2017. Multi-spectrum monitoring can be a useful tool in relocating a surgical field after a surgical task is completed to perform one or more of the previously described tests on the treated tissue.

It is axiomatic that strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field, including the imaging device 124 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

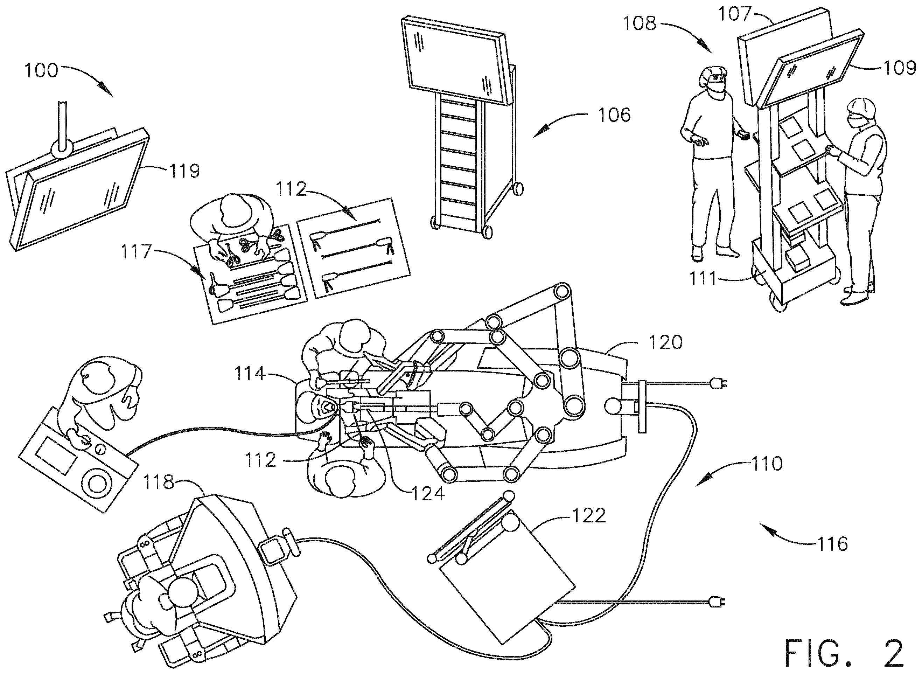

The visualization system 108 includes one or more imaging sensors, one or more image processing units, one or more storage arrays, and one or more displays that are strategically arranged with respect to the sterile field, as illustrated in FIG. 2. The visualization system 108 includes an interface for HL7, PACS, and EMR. Various components of the visualization system 108 are described under the heading "Advanced Imaging Acquisition Module" in U.S. Provisional Patent Application Serial No. 62/611,341, titled INTERACTIVE SURGICAL PLATFORM, filed December 28, 2017.

As illustrated in FIG. 2, a primary display 119 is positioned in the sterile field to be visible to an operator at the operating table 114. In addition, a visualization tower 111 is positioned outside the sterile field. The visualization tower 111 includes a first non-sterile display 107 and a second non-sterile display 109, which face away from each other. The visualization system 108, guided by the hub 106, is configured to utilize the displays 107, 109, and 119 to coordinate information flow to operators inside and outside the sterile field. For example, the hub 106 may cause the visualization system 108 to display a snap-shot of a surgical site, as recorded by an imaging device 124, on a non-sterile display 107 or 109, while maintaining a live feed of the surgical site on the primary display 119. The snap-shot on the non-sterile display 107 or 109 can permit a non-sterile operator to perform a diagnostic step relevant to the surgical procedure, for example.

The hub 106 is also configured to route a diagnostic input or feedback entered by a non-sterile operator at the visualization tower 111 to the primary display 119 within the sterile field, where it can be viewed by a sterile operator at the operating table. The input is in the form of a modification to the snap-shot displayed on the non-sterile display 107 or 109, which can be routed to the primary display 119 by the hub 106.

Referring to FIG. 2, a surgical instrument 112 is being used in the surgical procedure as part of the surgical system 102. The hub 106 is also configured to coordinate information flow to a display of the surgical instrument 112. For example, in U.S. Provisional Patent Application Serial No. 62/611,341, titled INTERACTIVE SURGICAL PLATFORM, filed December 28, 2017. A diagnostic input or feedback entered by a non-sterile operator at the visualization tower 111 is routed by the hub 106 to the surgical instrument display 115 within the sterile field, where it is viewed by the operator of the surgical instrument 112. Example surgical instruments that are suitable for use with the surgical system 102 are described under the heading "Surgical Instrument Hardware" and in U.S. Provisional Patent Application Serial No. 62/611,341, titled INTERACTIVE SURGICAL PLATFORM, filed December 28, 2017.

Referring now to FIG. 3, a hub 106 is depicted in communication with a visualization system 108, a robotic system 110, and a handheld intelligent surgical instrument 112. The hub 106 includes a hub display 135, an imaging module 138, a generator module 140, a communication module 130, a processor module 132, and a storage array 134. As illustrated in FIG. 3, the hub 106 further includes a smoke evacuation module 126 and/or a suction/irrigation module 128.

During a surgical procedure, energy application to tissue, for sealing and/or cutting, is generally associated with smoke evacuation, suction of excess fluid, and/or irrigation of the tissue. Fluid, power, and/or data lines from different sources are often entangled during the surgical procedure. Valuable time can be lost addressing this issue during a surgical procedure. Detangling the lines may necessitate disconnecting the lines from their respective modules, which may require resetting the modules. The hub modular enclosure 136 offers a unified environment for managing the power, data, and fluid lines, which reduces the frequency of entanglement between such lines.

A surgical hub is used in a surgical procedure that involves energy application to tissue at a surgical site. The surgical hub includes a hub enclosure and a combo generator module slidably receivable in a docking station of the hub enclosure. The docking station includes data and power contacts. The combo generator module includes two or more of an ultrasonic energy generator component, a bipolar RF energy generator component, and a monopolar RF energy generator component that are housed in a single unit. The combo generator module also includes a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component.

The fluid line is a first fluid line and a second fluid line extends from the remote surgical site to a suction and irrigation module slidably received in the hub enclosure. The hub enclosure comprises a fluid interface.

Certain surgical procedures may require the application of more than one energy type to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. A hub modular enclosure 136 is configured to accommodate different generators, and facilitate an interactive communication therebetween. One of the advantages of the hub modular enclosure 136 is enabling the quick removal and/or replacement of various modules.

A modular surgical enclosure is used in a surgical procedure that involves energy application to tissue. The modular surgical enclosure includes a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts.

The modular surgical enclosure also includes a second energy-generator module configured to generate a second energy, different than the first energy, for application to the tissue, and a second docking station comprising a second docking port that includes second data and power contacts, wherein the second energy-generator module is slidably movable into an electrical engagement with the power and data contacts, and wherein the second energy-generator module is slidably movable out of the electrical engagement with the second power and data contacts.

The modular surgical enclosure also includes a communication bus between the first docking port and the second docking port, configured to facilitate communication between the first energy-generator module and the second energy-generator module.

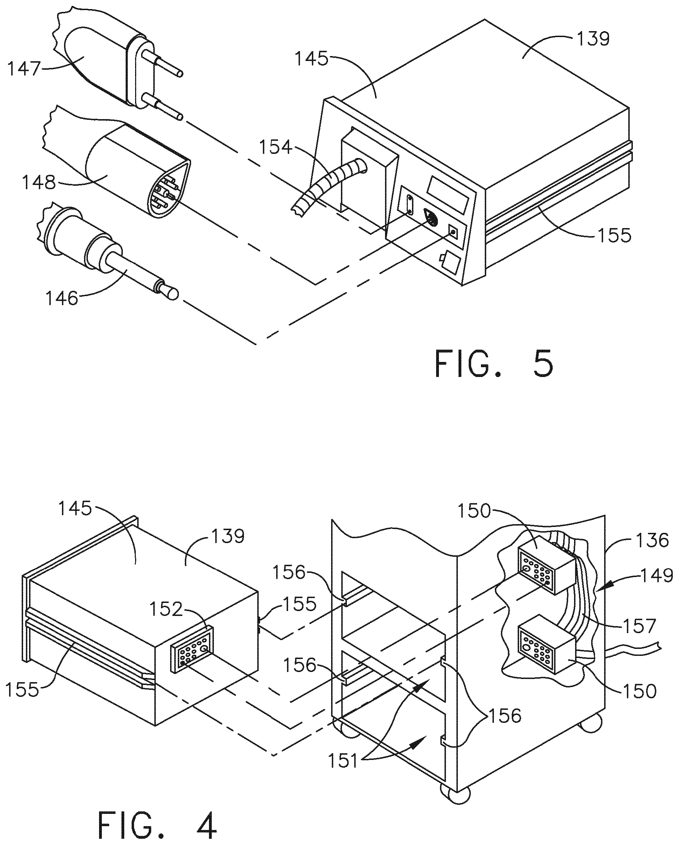

Referring to FIGS. 3-7, depicted is a hub modular enclosure 136 that allows the modular integration of a generator module 140, a smoke evacuation module 126, and a suction/irrigation module 128. The hub modular enclosure 136 further facilitates interactive communication between the modules 140, 126, 128. As illustrated in FIG. 5, the generator module 140 is a generator module with integrated monopolar, bipolar, and ultrasonic components supported in a single housing unit 139 slidably insertable into the hub modular enclosure 136. As illustrated in FIG. 5, the generator module 140 is configured to connect to a monopolar device 146, a bipolar device 147, and an ultrasonic device 148. Alternatively, the generator module 140 may comprise a series of monopolar, bipolar, and/or ultrasonic generator modules that interact through the hub modular enclosure 136. The hub modular enclosure 136 is configured to facilitate the insertion of multiple generators and interactive communication between the generators docked into the hub modular enclosure 136 so that the generators would act as a single generator.

The hub modular enclosure 136 comprises a modular power and communication backplane 149 with external and wireless communication headers to enable the removable attachment of the modules 140, 126, 128 and interactive communication therebetween.

The hub modular enclosure 136 includes docking stations, or drawers, 151, herein also referred to as drawers, which are configured to slidably receive the modules 140, 126, 128. FIG. 4 illustrates a partial perspective view of a surgical hub enclosure 136, and a combo generator module 145 slidably receivable in a docking station 151 of the surgical hub enclosure 136. A docking port 152 with power and data contacts on a rear side of the combo generator module 145 is configured to engage a corresponding docking port 150 with power and data contacts of a corresponding docking station 151 of the hub modular enclosure 136 as the combo generator module 145 is slid into position within the corresponding docking station 151 of the hub module enclosure 136. The combo generator module 145 includes a bipolar, ultrasonic, and monopolar module and a smoke evacuation module integrated together into a single housing unit 139, as illustrated in FIG. 5.

The smoke evacuation module 126 includes a fluid line 154 that conveys captured/collected smoke and/or fluid away from a surgical site and to, for example, the smoke evacuation module 126. Vacuum suction originating from the smoke evacuation module 126 can draw the smoke into an opening of a utility conduit at the surgical site. The utility conduit, coupled to the fluid line, is in the form of a flexible tube terminating at the smoke evacuation module 126. The utility conduit and the fluid line define a fluid path extending toward the smoke evacuation module 126 that is received in the hub enclosure 136.

The suction/irrigation module 128 is coupled to a surgical tool comprising an aspiration fluid line and a suction fluid line. The aspiration and suction fluid lines are in the form of flexible tubes extending from the surgical site toward the suction/irrigation module 128. One or more drive systems are configured to cause irrigation and aspiration of fluids to and from the surgical site.

The surgical tool includes a shaft having an end effector at a distal end thereof and at least one energy treatment associated with the end effector, an aspiration tube, and an irrigation tube. The aspiration tube has an inlet port at a distal end thereof and the aspiration tube extends through the shaft. Similarly, an irrigation tube extends through the shaft and has an inlet port in proximity to the energy deliver implement. The energy deliver implement is configured to deliver ultrasonic and/or RF energy to the surgical site and is coupled to the generator module 140 by a cable extending initially through the shaft.

The irrigation tube is in fluid communication with a fluid source, and the aspiration tube is in fluid communication with a vacuum source. The fluid source and/or the vacuum source is housed in the suction/irrigation module 128. In one example, the fluid source and/or the vacuum source is housed in the hub enclosure 136 separately from the suction/irrigation module 128. In such example, a fluid interface is configured to connect the suction/irrigation module 128 to the fluid source and/or the vacuum source.

The modules 140, 126, 128 and/or their corresponding docking stations on the hub modular enclosure 136 include alignment features that are configured to align the docking ports of the modules into engagement with their counterparts in the docking stations of the hub modular enclosure 136. For example, as illustrated in FIG. 4, the combo generator module 145 includes side brackets 155 that are configured to slidably engage with corresponding brackets 156 of the corresponding docking station 151 of the hub modular enclosure 136. The brackets cooperate to guide the docking port contacts of the combo generator module 145 into an electrical engagement with the docking port contacts of the hub modular enclosure 136.

The drawers 151 of the hub modular enclosure 136 are the same, or substantially the same size, and the modules are adjusted in size to be received in the drawers 151. The side brackets 155 and/or 156 are larger or smaller depending on the size of the module. Alternatively, the drawers 151 are different in size and are each designed to accommodate a particular module.

Furthermore, the contacts of a particular module can be keyed for engagement with the contacts of a particular drawer to avoid inserting a module into a drawer with mismatching contacts.

As illustrated in FIG. 4, the docking port 150 of one drawer 151 can be coupled to the docking port 150 of another drawer 151 through a communications link 157 to facilitate an interactive communication between the modules housed in the hub modular enclosure 136. The docking ports 150 of the hub modular enclosure 136 may alternatively, or additionally, facilitate a wireless interactive communication between the modules housed in the hub modular enclosure 136. Any suitable wireless communication can be employed, such as for example Air Titan-Bluetooth.

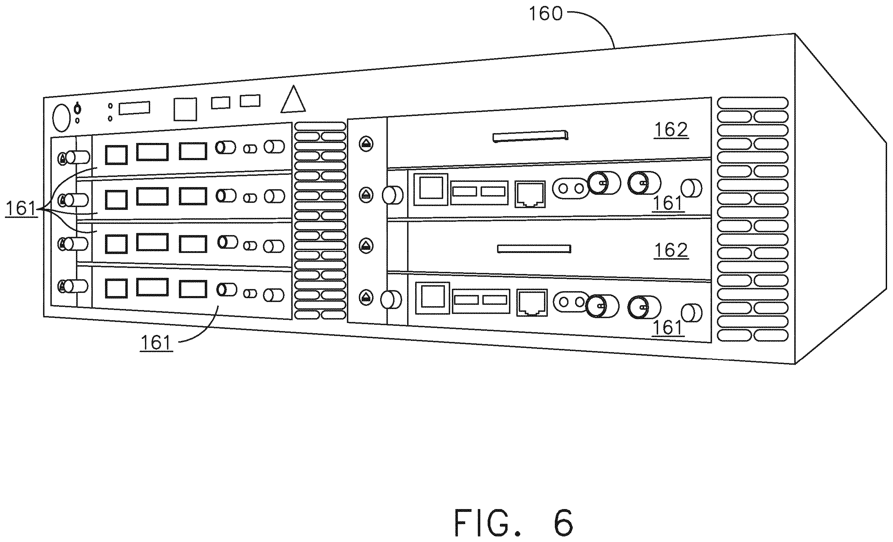

FIG. 6 illustrates individual power bus attachments for a plurality of lateral docking ports of a lateral modular housing 160 configured to receive a plurality of modules of a surgical hub 206. The lateral modular housing 160 is configured to laterally receive and interconnect the modules 161. The modules 161 are slidably inserted into docking stations 162 of lateral modular housing 160, which includes a backplane for interconnecting the modules 161. As illustrated in FIG. 6, the modules 161 are arranged laterally in the lateral modular housing 160. Alternatively, the modules 161 may be arranged vertically in a lateral modular housing.

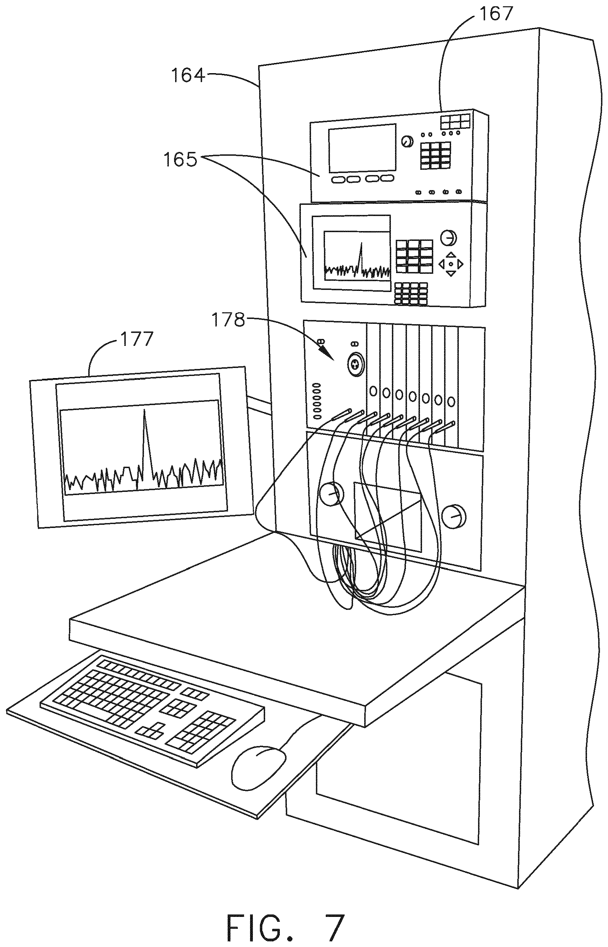

FIG. 7 illustrates a vertical modular housing 164 configured to receive a plurality of modules 165 of the surgical hub 106. The modules 165 are slidably inserted into docking stations, or drawers, 167 of vertical modular housing 164, which includes a backplane for interconnecting the modules 165. Although the drawers 167 of the vertical modular housing 164 are arranged vertically, a vertical modular housing 164 may include drawers that are arranged laterally. Furthermore, the modules 165 interact with one another through the docking ports of the vertical modular housing 164. In the example of FIG. 7, a display 177 is provided for displaying data relevant to the operation of the modules 165. In addition, the vertical modular housing 164 includes a master module 178 housing a plurality of sub-modules that are slidably received in the master module 178.

The imaging module 138 comprises an integrated video processor and a modular light source and is adapted for use with various imaging devices. The imaging device is comprised of a modular housing that can be assembled with a light source module and a camera module. The housing is a disposable housing. The disposable housing is removably coupled to a reusable controller, a light source module, and a camera module. The light source module and/or the camera module are selectively chosen depending on the type of surgical procedure. The camera module comprises a CCD sensor. The camera module comprises a CMOS sensor. The camera module is configured for scanned beam imaging. Likewise, the light source module is configured to deliver a white light or a different light, depending on the surgical procedure.

During a surgical procedure, removing a surgical device from the surgical field and replacing it with another surgical device that includes a different camera or a different light source can be inefficient. Temporarily losing sight of the surgical field may lead to undesirable consequences. The module imaging device of the present disclosure is configured to permit the replacement of a light source module or a camera module midstream during a surgical procedure, without having to remove the imaging device from the surgical field.

The imaging device comprises a tubular housing that includes a plurality of channels. A first channel is configured to slidably receive the camera module, which can be configured for a snap-fit engagement with the first channel. A second channel is configured to slidably receive the light source module, which can be configured for a snap-fit engagement with the second channel. The camera module and/or the light source module can be rotated into a final position within their respective channels. A threaded engagement can be employed in lieu of the snap-fit engagement.

Multiple imaging devices are placed at different positions in the surgical field to provide multiple views. The imaging module 138 are configured to switch between the imaging devices to provide an optimal view. The imaging module 138 is configured to integrate the images from the different imaging device.

Various image processors and imaging devices suitable for use with the present disclosure are described in U.S. Patent No. 7,995,045, titled COMBINED SBI AND CONVENTIONAL IMAGE PROCESSOR, which issued on August 9, 2011. In addition, U.S. Patent No. 7,982,776, titled SBI MOTION ARTIFACT REMOVAL APPARATUS AND METHOD, which issued on July 19, 2011, describes various systems for removing motion artifacts from image data. Such systems can be integrated with the imaging module 138. Furthermore, U.S. Patent Application Publication No. 2011/0306840, titled CONTROLLABLE MAGNETIC SOURCE TO FIXTURE INTRACORPOREAL APPARATUS, which published on December 15, 2011, and U.S. Patent Application Publication No. 2014/0243597, titled SYSTEM FOR PERFORMING A MINIMALLY INVASIVE SURGICAL PROCEDURE, which published on August 28, 2014.

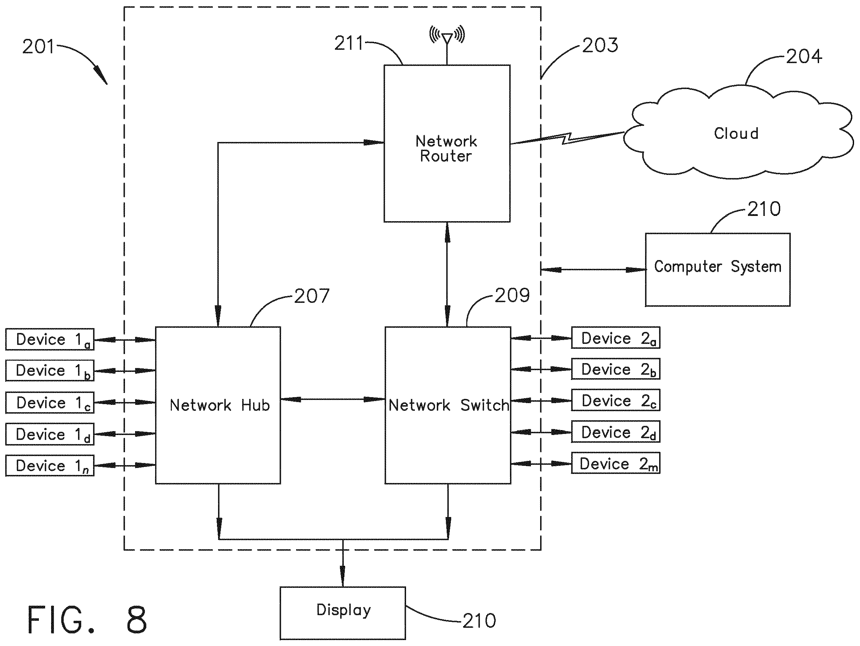

FIG. 8 illustrates a surgical data network 201 comprising a modular communication hub 203 configured to connect modular devices located in one or more operating theaters of a healthcare facility, or any room in a healthcare facility specially equipped for surgical operations, to a cloud-based system (e.g., the cloud 204 that includes a remote server 213 coupled to a storage device 205). The modular communication hub 203 comprises a network hub 207 and/or a network switch 209 in communication with a network router. The modular communication hub 203 also is coupled to a local computer system 210 to provide local computer processing and data manipulation. The surgical data network 201 may be configured as passive, intelligent, or switching. A passive surgical data network serves as a conduit for the data, enabling it to go from one device (or segment) to another and to the cloud computing resources. An intelligent surgical data network includes additional features to enable the traffic passing through the surgical data network to be monitored and to configure each port in the network hub 207 or network switch 209. An intelligent surgical data network is referred to as a manageable hub or switch. A switching hub reads the destination address of each packet and then forwards the packet to the correct port.

Modular devices 1a-1n located in the operating theater are coupled to the modular communication hub 203. The network hub 207 and/or the network switch 209 is/are coupled to a network router 211 to connect the devices 1a-1n to the cloud 204 or the local computer system 210. Data associated with the devices 1a-1n is transferred to cloud-based computers via the router for remote data processing and manipulation. Data associated with the devices 1a-1n is also be transferred to the local computer system 210 for local data processing and manipulation. Modular devices 2a-2m located in the same operating theater also are coupled to a network switch 209. The network switch 209 is coupled to the network hub 207 and/or the network router 211 to connect to the devices 2a-2m to the cloud 204. Data associated with the devices 2a-2n is transferred to the cloud 204 via the network router 211 for data processing and manipulation. Data associated with the devices 2a-2m is also be transferred to the local computer system 210 for local data processing and manipulation.

It will be appreciated that the surgical data network 201 can be expanded by interconnecting multiple network hubs 207 and/or multiple network switches 209 with multiple network routers 211. The modular communication hub 203 is contained in a modular control tower configured to receive multiple devices 1a-1n/2a-2m. The local computer system 210 also is contained in a modular control tower. The modular communication hub 203 is connected to a display 212 to display images obtained by some of the devices 1a-1n/2a-2m, for example during surgical procedures. The devices 1a-1n/2a-2m include, for example, various modules such as an imaging module 138 coupled to an endoscope, a generator module 140 coupled to an energy-based surgical device, a smoke evacuation module 126, a suction/irrigation module 128, a communication module 130, a processor module 132, a storage array 134, a surgical device coupled to a display, and/or a non-contact sensor module, among other modular devices that are connected to the modular communication hub 203 of the surgical data network 201.

The surgical data network 201 comprises a combination of network hub(s), network switch(es), and network router(s) connecting the devices 1a-1n/2a-2m to the cloud. Any one of or all of the devices 1a-1n/2a-2m coupled to the network hub or network switch collects data in real time and transfers the data to cloud computers for data processing and manipulation. It will be appreciated that cloud computing relies on sharing computing resources rather than having local servers or personal devices to handle software applications. The word "cloud" is used as a metaphor for "the Internet," although the term is not limited as such. Accordingly, the term "cloud computing" is also used herein to refer to "a type of Internet-based computing," where different services-such as servers, storage, and applications-are delivered to the modular communication hub 203 and/or computer system 210 located in the surgical theater (e.g., a fixed, mobile, temporary, or field operating room or space) and to devices connected to the modular communication hub 203 and/or computer system 210 through the Internet. The cloud infrastructure may be maintained by a cloud service provider. In this context, the cloud service provider is the entity that coordinates the usage and control of the devices 1a-1n/2a-2m located in one or more operating theaters. The cloud computing services can perform a large number of calculations based on the data gathered by smart surgical instruments, robots, and other computerized devices located in the operating theater. The hub hardware enables multiple devices or connections to be connected to a computer that communicates with the cloud computing resources and storage.

Applying cloud computer data processing techniques on the data collected by the devices 1a-1n/2a-2m, the surgical data network provides improved surgical outcomes, reduced costs, and improved patient satisfaction. At least some of the devices 1a-1n/2a-2m are employed to view tissue states to assess leaks or perfusion of sealed tissue after a tissue sealing and cutting procedure. At least some of the devices 1a-1n/2a-2m are employed to identify pathology, such as the effects of diseases, using the cloud-based computing to examine data including images of samples of body tissue for diagnostic purposes. This includes localization and margin confirmation of tissue and phenotypes. At least some of the devices 1a-1n/2a-2m are employed to identify anatomical structures of the body using a variety of sensors integrated with imaging devices and techniques such as overlaying images captured by multiple imaging devices. The data gathered by the devices 1a-1n/2a-2m, including image data, is transferred to the cloud 204 or the local computer system 210 or both for data processing and manipulation including image processing and manipulation. The data is analyzed to improve surgical procedure outcomes by determining if further treatment, such as the application of endoscopic intervention, emerging technologies, a targeted radiation, targeted intervention, and precise robotics to tissue-specific sites and conditions, is pursued. Such data analysis employs outcome analytics processing, and using standardized approaches may provide beneficial feedback to either confirm surgical treatments and the behavior of the surgeon or suggest modifications to surgical treatments and the behavior of the surgeon.

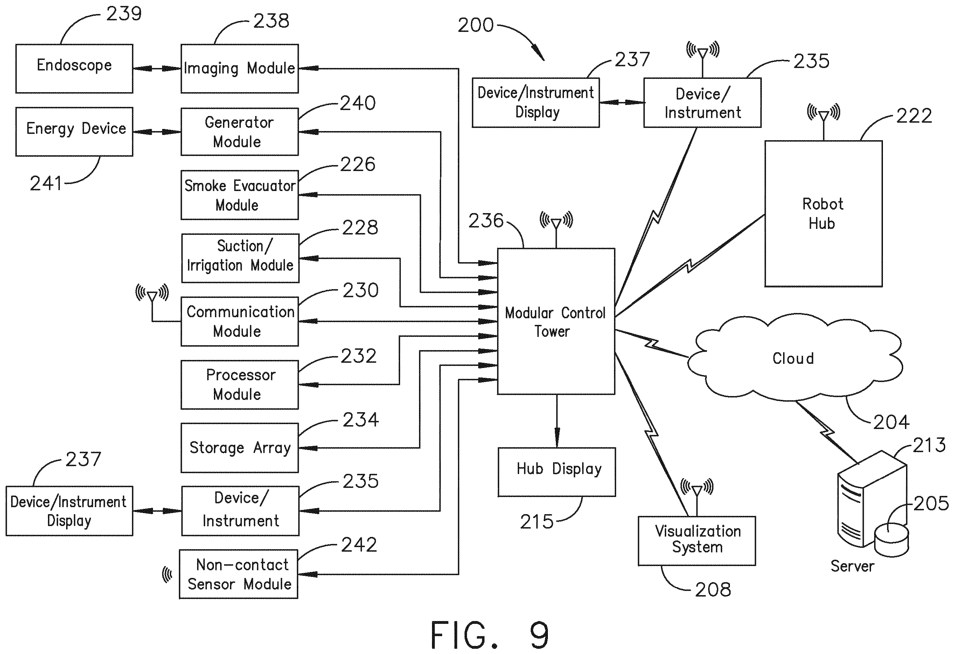

The operating theater devices 1a-1n are connected to the modular communication hub 203 over a wired channel or a wireless channel depending on the configuration of the devices 1a-1n to a network hub. The network hub 207 is implemented as a local network broadcast device that works on the physical layer of the Open System Interconnection (OSI) model. The network hub provides connectivity to the devices 1a-1n located in the same operating theater network. The network hub 207 collects data in the form of packets and sends them to the router in half duplex mode. The network hub 207 does not store any media access control/internet protocol (MAC/IP) to transfer the device data. Only one of the devices 1a-1n can send data at a time through the network hub 207. The network hub 207 has no routing tables or intelligence regarding where to send information and broadcasts all network data across each connection and to a remote server 213 (FIG. 9) over the cloud 204. The network hub 207 can detect basic network errors such as collisions, but having all information broadcast to multiple ports can be a security risk and cause bottlenecks.

The operating theater devices 2a-2m are connected to a network switch 209 over a wired channel or a wireless channel. The network switch 209 works in the data link layer of the OSI model. The network switch 209 is a multicast device for connecting the devices 2a-2m located in the same operating theater to the network. The network switch 209 sends data in the form of frames to the network router 211 and works in full duplex mode. Multiple devices 2a-2m can send data at the same time through the network switch 209. The network switch 209 stores and uses MAC addresses of the devices 2a-2m to transfer data.

The network hub 207 and/or the network switch 209 are coupled to the network router 211 for connection to the cloud 204. The network router 211 works in the network layer of the OSI model. The network router 211 creates a route for transmitting data packets received from the network hub 207 and/or network switch 211 to cloud-based computer resources for further processing and manipulation of the data collected by any one of or all the devices 1a-1n/2a-2m. The network router 211 is employed to connect two or more different networks located in different locations, such as, for example, different operating theaters of the same healthcare facility or different networks located in different operating theaters of different healthcare facilities. The network router 211 sends data in the form of packets to the cloud 204 and works in full duplex mode. Multiple devices can send data at the same time. The network router 211 uses IP addresses to transfer data.

The network hub 207 is implemented as a USB hub, which allows multiple USB devices to connect to a host computer. The USB hub expands a single USB port into several tiers so that there are more ports available to connect devices to the host system computer. The network hub 207 includes wired or wireless capabilities to receive information over a wired channel or a wireless channel. A wireless USB short-range, high-bandwidth wireless radio communication protocol is employed for communication between the devices 1a-1n and devices 2a-2m located in the operating theater.

The operating theater devices 1a-1n/2a-2m communicate to the modular communication hub 203 via Bluetooth wireless technology standard for exchanging data over short distances (using short-wavelength UHF radio waves in the ISM band from 2.4 to 2.485 GHz) from fixed and mobile devices and building personal area networks (PANs). The operating theater devices 1a-1n/2a-2m communicates to the modular communication hub 203 via a number of wireless or wired communication standards or protocols, including but not limited to Wi-Fi (IEEE 802.11 family), WiMAX (IEEE 802.16 family), IEEE 802.20, long-term evolution (LTE), and Ev-DO, HSPA+, HSDPA+, HSUPA+, EDGE, GSM, GPRS, CDMA, TDMA, DECT, and Ethernet derivatives thereof, as well as any other wireless and wired protocols that are designated as 3G, 4G, 5G, and beyond. The computing module includes a plurality of communication modules. For instance, a first communication module is dedicated to shorter-range wireless communications such as Wi-Fi and Bluetooth, and a second communication module is dedicated to longer-range wireless communications such as GPS, EDGE, GPRS, CDMA, Wi-MAX, LTE, Ev-DO, and others.

The modular communication hub 203 serves as a central connection for one or all of the operating theater devices 1a-1n/2a-2m and handles a data type known as frames. Frames carry the data generated by the devices 1a-1n/2a-2m. When a frame is received by the modular communication hub 203, it is amplified and transmitted to the network router 211, which transfers the data to the cloud computing resources by using a number of wireless or wired communication standards or protocols, as described herein.

The modular communication hub 203 can be used as a standalone device or be connected to compatible network hubs and network switches to form a larger network. The modular communication hub 203 is generally easy to install, configure, and maintain, making it a good option for networking the operating theater devices 1a-1n/2a-2m.

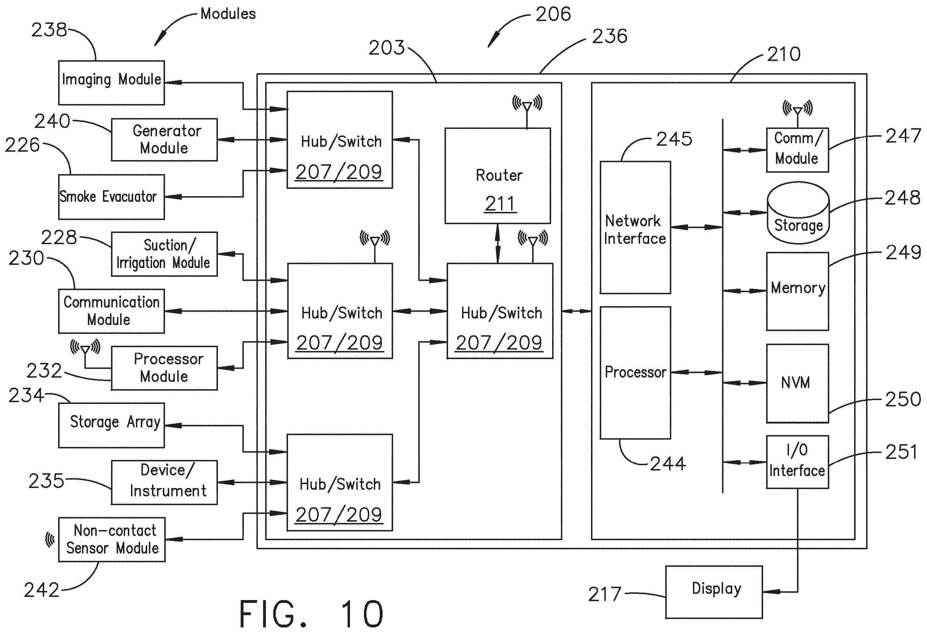

FIG. 9 illustrates a computer-implemented interactive surgical system 200. The computer-implemented interactive surgical system 200 is similar in many respects to the computer-implemented interactive surgical system 100. For example, the computer-implemented interactive surgical system 200 includes one or more surgical systems 202, which are similar in many respects to the surgical systems 102. Each surgical system 202 includes at least one surgical hub 206 in communication with a cloud 204 that may include a remote server 213. In one aspect, the computer-implemented interactive surgical system 200 comprises a modular control tower 236 connected to multiple operating theater devices such as, for example, intelligent surgical instruments, robots, and other computerized devices located in the operating theater. As shown in FIG. 10, the modular control tower 236 comprises a modular communication hub 203 coupled to a computer system 210. As illustrated in the example of FIG. 9, the modular control tower 236 is coupled to an imaging module 238 that is coupled to an endoscope 239, a generator module 240 that is coupled to an energy device 241, a smoke evacuator module 226, a suction/irrigation module 228, a communication module 230, a processor module 232, a storage array 234, a smart device/instrument 235 optionally coupled to a display 237, and a non-contact sensor module 242. The operating theater devices are coupled to cloud computing resources and data storage via the modular control tower 236. A robot hub 222 also is connected to the modular control tower 236 and to the cloud computing resources. The devices/instruments 235, visualization systems 208, among others, is coupled to the modular control tower 236 via wired or wireless communication standards or protocols, as described herein. The modular control tower 236 is coupled to a hub display 215 (e.g., monitor, screen) to display and overlay images received from the imaging module, device/instrument display, and/or other visualization systems 208. The hub display also displays data received from devices connected to the modular control tower in conjunction with images and overlaid images.

FIG. 10 illustrates a surgical hub 206 comprising a plurality of modules coupled to the modular control tower 236. The modular control tower 236 comprises a modular communication hub 203, e.g., a network connectivity device, and a computer system 210 to provide local processing, visualization, and imaging, for example. As shown in FIG. 10, the modular communication hub 203 is connected in a tiered configuration to expand the number of modules (e.g., devices) that may be connected to the modular communication hub 203 and transfer data associated with the modules to the computer system 210, cloud computing resources, or both. As shown in FIG. 10, each of the network hubs/switches in the modular communication hub 203 includes three downstream ports and one upstream port. The upstream network hub/switch is connected to a processor to provide a communication connection to the cloud computing resources and a local display 217. Communication to the cloud 204 is made either through a wired or a wireless communication channel.

The surgical hub 206 employs a non-contact sensor module 242 to measure the dimensions of the operating theater and generate a map of the surgical theater using either ultrasonic or laser-type non-contact measurement devices. An ultrasound-based non-contact sensor module scans the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, in which the sensor module is configured to determine the size of the operating theater and to adjust Bluetooth-pairing distance limits. A laser-based non-contact sensor module scans the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

The computer system 210 comprises a processor 244 and a network interface 245. The processor 244 is coupled to a communication module 247, storage 248, memory 249, non-volatile memory 250, and input/output interface 251 via a system bus. The system bus is any of several types of bus structure(s) including the memory bus or memory controller, a peripheral bus or external bus, and/or a local bus using any variety of available bus architectures including, but not limited to, 9-bit bus, Industrial Standard Architecture (ISA), Micro-Charmel Architecture (MSA), Extended ISA (EISA), Intelligent Drive Electronics (IDE), VESA Local Bus (VLB), Peripheral Component Interconnect (PCI), USB, Advanced Graphics Port (AGP), Personal Computer Memory Card International Association bus (PCMCIA), Small Computer Systems Interface (SCSI), or any other proprietary bus.

The processor 244 is any single-core or multicore processor such as those known under the trade name ARM Cortex by Texas Instruments. In one aspect, the processor may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle serial random access memory (SRAM), an internal read-only memory (ROM) loaded with StellarisWare® software, a 2 KB electrically erasable programmable read-only memory (EEPROM), and/or one or more pulse width modulation (PWM) modules, one or more quadrature encoder inputs (QEI) analogs, one or more 12-bit analog-to-digital converters (ADCs) with 12 analog input channels, details of which are available for the product datasheet.

In one aspect, the processor 244 comprises a safety controller comprising two controller-based families such as TMS570 and RM4x, known under the trade name Hercules ARM Cortex R4, also by Texas Instruments. The safety controller is configured specifically for IEC 61508 and ISO 26262 safety critical applications, among others, to provide advanced integrated safety features while delivering scalable performance, connectivity, and memory options.

The system memory includes volatile memory and non-volatile memory. The basic input/output system (BIOS), containing the basic routines to transfer information between elements within the computer system, such as during start-up, is stored in non-volatile memory. For example, the non-volatile memory can include ROM, programmable ROM (PROM), electrically programmable ROM (EPROM), EEPROM, or flash memory. Volatile memory includes random-access memory (RAM), which acts as external cache memory. Moreover, RAM is available in many forms such as SRAM, dynamic RAM (DRAM), synchronous DRAM (SDRAM), double data rate SDRAM (DDR SDRAM), enhanced SDRAM (ESDRAM), Synchlink DRAM (SLDRAM), and direct Rambus RAM (DRRAM).

The computer system 210 also includes removable/non-removable, volatile/non-volatile computer storage media, such as for example disk storage. The disk storage includes, but is not limited to, devices like a magnetic disk drive, floppy disk drive, tape drive, Jaz drive, Zip drive, LS-60 drive, flash memory card, or memory stick. In addition, the disk storage can include storage media separately or in combination with other storage media including, but not limited to, an optical disc drive such as a compact disc ROM device (CD-ROM), compact disc recordable drive (CD-R Drive), compact disc rewritable drive (CD-RW Drive), or a digital versatile disc ROM drive (DVD-ROM). To facilitate the connection of the disk storage devices to the system bus, a removable or non-removable interface may be employed.

It is to be appreciated that the computer system 210 includes software that acts as an intermediary between users and the basic computer resources described in a suitable operating environment. Such software includes an operating system. The operating system, which can be stored on the disk storage, acts to control and allocate resources of the computer system. System applications take advantage of the management of resources by the operating system through program modules and program data stored either in the system memory or on the disk storage. It is to be appreciated that various components described herein can be implemented with various operating systems or combinations of operating systems.

A user enters commands or information into the computer system 210 through input device(s) coupled to the I/O interface 251. The input devices include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processor through the system bus via interface port(s). The interface port(s) include, for example, a serial port, a parallel port, a game port, and a USB. The output device(s) use some of the same types of ports as input device(s). Thus, for example, a USB port is used to provide input to the computer system and to output information from the computer system to an output device. An output adapter is provided to illustrate that there are some output devices like monitors, displays, speakers, and printers, among other output devices that require special adapters. The output adapters include, by way of illustration and not limitation, video and sound cards that provide a means of connection between the output device and the system bus. It should be noted that other devices and/or systems of devices, such as remote computer(s), provide both input and output capabilities.

The computer system 210 operates in a networked environment using logical connections to one or more remote computers, such as cloud computer(s), or local computers. The remote cloud computer(s) can be a personal computer, server, router, network PC, workstation, microprocessor-based appliance, peer device, or other common network node, and the like, and typically includes many or all of the elements described relative to the computer system. For purposes of brevity, only a memory storage device is illustrated with the remote computer(s). The remote computer(s) is logically connected to the computer system through a network interface and then physically connected via a communication connection. The network interface encompasses communication networks such as local area networks (LANs) and wide area networks (WANs). LAN technologies include Fiber Distributed Data Interface (FDDI), Copper Distributed Data Interface (CDDI), Ethernet/IEEE 802.3, Token Ring/IEEE 802.5 and the like. WAN technologies include, but are not limited to, point-to-point links, circuit-switching networks like Integrated Services Digital Networks (ISDN) and variations thereon, packet-switching networks, and Digital Subscriber Lines (DSL).

The computer system 210 of FIG. 10, the imaging module 238 and/or visualization system 208, and/or the processor module 232 of FIGS. 9-10, comprises an image processor, image processing engine, media processor, or any specialized digital signal processor (DSP) used for the processing of digital images. The image processor employs parallel computing with single instruction, multiple data (SIMD) or multiple instruction, multiple data (MIMD) technologies to increase speed and efficiency. The digital image processing engine can perform a range of tasks. The image processor may be a system on a chip with multicore processor architecture.

The communication connection(s) refers to the hardware/software employed to connect the network interface to the bus. While the communication connection is shown for illustrative clarity inside the computer system, it can also be external to the computer system 210. The hardware/software necessary for connection to the network interface includes, for illustrative purposes only, internal and external technologies such as modems, including regular telephone-grade modems, cable modems, and DSL modems, ISDN adapters, and Ethernet cards.

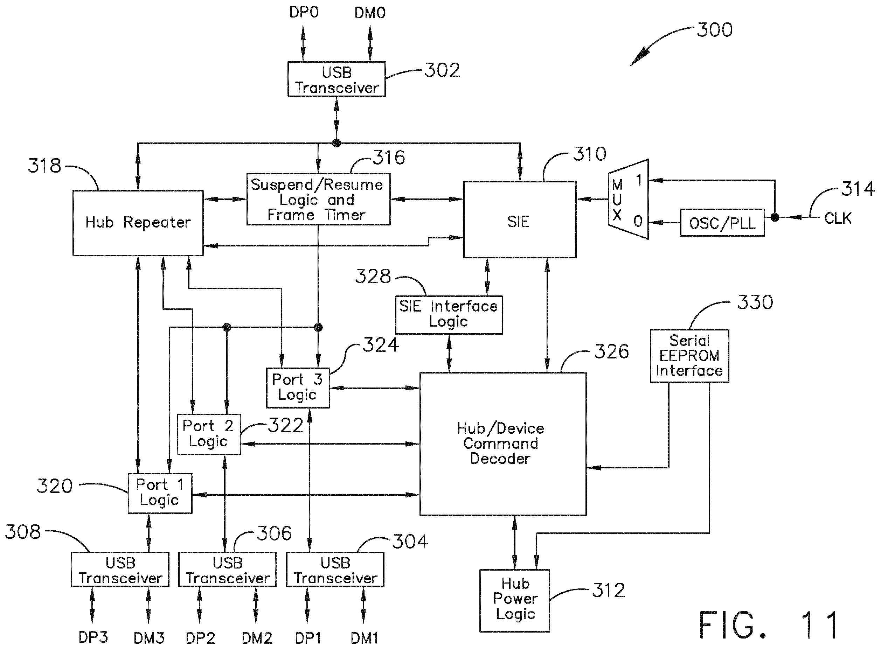

FIG. 11 illustrates a functional block diagram of a USB network hub 300 device. The USB network hub device 300 employs a TUSB2036 integrated circuit hub by Texas Instruments. The USB network hub 300 is a CMOS device that provides an upstream USB transceiver port 302 and up to three downstream USB transceiver ports 304, 306, 308 in compliance with the USB 2.0 specification. The upstream USB transceiver port 302 is a differential root data port comprising a differential data minus (DM0) input paired with a differential data plus (DP0) input. The three downstream USB transceiver ports 304, 306, 308 are differential data ports where each port includes differential data plus (DP1-DP3) outputs paired with differential data minus (DM1-DM3) outputs.

The USB network hub 300 device is implemented with a digital state machine instead of a microcontroller, and no firmware programming is required. Fully compliant USB transceivers are integrated into the circuit for the upstream USB transceiver port 302 and all downstream USB transceiver ports 304, 306, 308. The downstream USB transceiver ports 304, 306, 308 support both full-speed and low-speed devices by automatically setting the slew rate according to the speed of the device attached to the ports. The USB network hub 300 device may be configured either in bus-powered or self-powered mode and includes a hub power logic 312 to manage power.

The USB network hub 300 device includes a serial interface engine 310 (SIE). The SIE 310 is the front end of the USB network hub 300 hardware and handles most of the protocol described in chapter 8 of the USB specification. The SIE 310 typically comprehends signaling up to the transaction level. The functions that it handles include: packet recognition, transaction sequencing, SOP, EOP, RESET, and RESUME signal detection/generation, clock/data separation, non-return-to-zero invert (NRZI) data encoding/decoding and bit-stuffing, CRC generation and checking (token and data), packet ID (PID) generation and checking/decoding, and/or serial-parallel/parallel-serial conversion. The 310 receives a clock input 314 and is coupled to a suspend/resume logic and frame timer 316 circuit and a hub repeater circuit 318 to control communication between the upstream USB transceiver port 302 and the downstream USB transceiver ports 304, 306, 308 through port logic circuits 320, 322, 324. The SIE 310 is coupled to a command decoder 326 via interface logic to control commands from a serial EEPROM via a serial EEPROM interface 330.

The USB network hub 300 connects 127 functions configured in up to six logical layers (tiers) to a single computer. Further, the USB network hub 300 connects to all peripherals using a standardized four-wire cable that provides both communication and power distribution. The power configurations are bus-powered and self-powered modes. The USB network hub 300 is configured to support four modes of power management: a bus-powered hub, with either individual-port power management or ganged-port power management, and the self-powered hub, with either individual-port power management or ganged-port power management. Using a USB cable, the USB network hub 300, the upstream USB transceiver port 302 is plugged into a USB host controller, and the downstream USB transceiver ports 304, 306, 308 are exposed for connecting USB compatible devices, and so forth.

Surgical Instrument Hardware

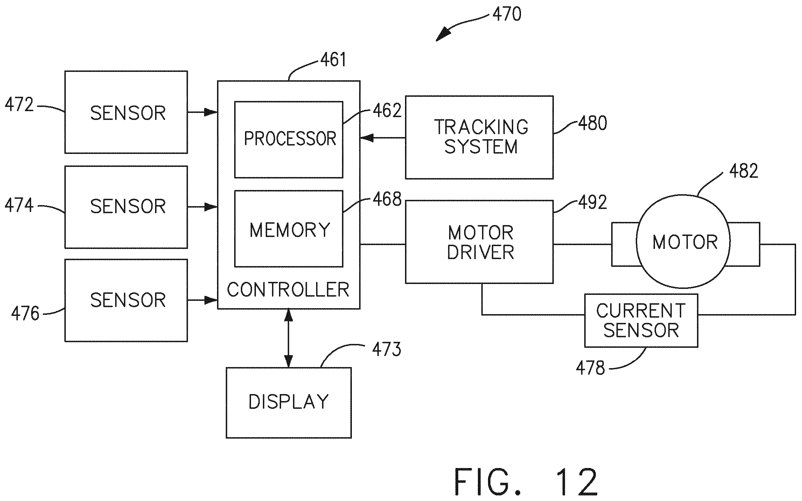

FIG. 12 illustrates a logic diagram of a control system 470 of a surgical instrument or tool. The system 470 comprises a control circuit. The control circuit includes a microcontroller 461 comprising a processor 462 and a memory 468. One or more of sensors 472, 474, 476, for example, provide real-time feedback to the processor 462. A motor 482, driven by a motor driver 492, operably couples a longitudinally movable displacement member to drive the I-beam knife element. A tracking system 480 is configured to determine the position of the longitudinally movable displacement member. The position information is provided to the processor 462, which is programmed or configured to determine the position of the longitudinally movable drive member as well as the position of a firing member, firing bar, and I-beam knife element. Additional motors are provided at the tool driver interface to control I-beam firing, closure tube travel, shaft rotation, and articulation. A display 473 displays a variety of operating conditions of the instruments and includes touch screen functionality for data input. Information displayed on the display 473 is overlaid with images acquired via endoscopic imaging modules.

The microcontroller 461 may be any single-core or multicore processor such as those known under the trade name ARM Cortex by Texas Instruments. The main microcontroller 461 may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle SRAM, and internal ROM loaded with StellarisWare® software, a 2 KB EEPROM, one or more PWM modules, one or more QEI analogs, and/or one or more 12-bit ADCs with 12 analog input channels, details of which are available for the product datasheet.

The microcontroller 461 comprises a safety controller comprising two controller-based families such as TMS570 and RM4x, known under the trade name Hercules ARM Cortex R4, also by Texas Instruments. The safety controller is configured specifically for IEC 61508 and ISO 26262 safety critical applications, among others, to provide advanced integrated safety features while delivering scalable performance, connectivity, and memory options.

The microcontroller 461 is programmed to perform various functions such as precise control over the speed and position of the knife and articulation systems. The microcontroller 461 includes a processor 462 and a memory 468. The electric motor 482 may be a brushed direct current (DC) motor with a gearbox and mechanical links to an articulation or knife system. A motor driver 492 is an A3941 available from Allegro Microsystems, Inc. Other motor drivers may be readily substituted for use in the tracking system 480 comprising an absolute positioning system. A detailed description of an absolute positioning system is described in U.S. Patent Application Publication No. 2017/0296213, titled SYSTEMS AND METHODS FOR CONTROLLING A SURGICAL STAPLING AND CUTTING INSTRUMENT, which published on October 19, 2017.

The microcontroller 461 is programmed to provide precise control over the speed and position of displacement members and articulation systems. The microcontroller 461 is configured to compute a response in the software of the microcontroller 461. The computed response is compared to a measured response of the actual system to obtain an "observed" response, which is used for actual feedback decisions. The observed response is a favorable, tuned value that balances the smooth, continuous nature of the simulated response with the measured response, which detects outside influences on the system.

The motor 482 is controlled by the motor driver 492 and is employed by the firing system of the surgical instrument or tool. The motor 482 is a brushed DC driving motor having a maximum rotational speed of approximately 25,000 RPM. Alernatively, the motor 482 includes a brushless motor, a cordless motor, a synchronous motor, a stepper motor, or any other suitable electric motor. The motor driver 492 comprises an H-bridge driver comprising field-effect transistors (FETs), for example. The motor 482 is powered by a power assembly releasably mounted to the handle assembly or tool housing for supplying control power to the surgical instrument or tool. The power assembly comprises a battery which includes a number of battery cells connected in series that can be used as the power source to power the surgical instrument or tool. In certain circumstances, the battery cells of the power assembly are replaceable and/or rechargeable. In at least one example, the battery cells can be lithium-ion batteries which are couplable to and separable from the power assembly.

The motor driver 492 may be an A3941 available from Allegro Microsystems, Inc. The A3941 492 is a full-bridge controller for use with external N-channel power metal-oxide semiconductor field-effect transistors (MOSFETs) specifically designed for inductive loads, such as brush DC motors. The driver 492 comprises a unique charge pump regulator that provides full (>10 V) gate drive for battery voltages down to 7 V and allows the A3941 to operate with a reduced gate drive, down to 5.5 V. A bootstrap capacitor is employed to provide the above battery supply voltage required for N-channel MOSFETs. An internal charge pump for the high-side drive allows DC (100% duty cycle) operation. The full bridge is driven in fast or slow decay modes using diode or synchronous rectification. In the slow decay mode, current recirculation can be through the high-side or the lowside FETs. The power FETs are protected from shoot-through by resistor-adjustable dead time. Integrated diagnostics provide indications of undervoltage, overtemperature, and power bridge faults and are configured to protect the power MOSFETs under most short circuit conditions. Other motor drivers are readily substituted for use in the tracking system 480 comprising an absolute positioning system.

The tracking system 480 comprises a controlled motor drive circuit arrangement comprising a position sensor 472 according to one aspect of this disclosure. The position sensor 472 for an absolute positioning system provides a unique position signal corresponding to the location of a displacement member. The displacement member represents a longitudinally movable drive member comprising a rack of drive teeth for meshing engagement with a corresponding drive gear of a gear reducer assembly. The displacement member represents the firing member, which could be adapted and configured to include a rack of drive teeth. The displacement member represents a firing bar or the I-beam, each of which can be adapted and configured to include a rack of drive teeth. Accordingly, as used herein, the term displacement member is used generically to refer to any movable member of the surgical instrument or tool such as the drive member, the firing member, the firing bar, the I-beam, or any element that can be displaced. The longitudinally movable drive member is coupled to the firing member, the firing bar, and the I-beam. Accordingly, the absolute positioning system can, in effect, track the linear displacement of the I-beam by tracking the linear displacement of the longitudinally movable drive member. The displacement member is coupled to any position sensor 472 suitable for measuring linear displacement. Thus, the longitudinally movable drive member, the firing member, the firing bar, or the I-beam, or combinations thereof, are coupled to any suitable linear displacement sensor. Linear displacement sensors include contact or non-contact displacement sensors. Linear displacement sensors comprise linear variable differential transformers (LVDT), differential variable reluctance transducers (DVRT), a slide potentiometer, a magnetic sensing system comprising a movable magnet and a series of linearly arranged Hall effect sensors, a magnetic sensing system comprising a fixed magnet and a series of movable, linearly arranged Hall effect sensors, an optical sensing system comprising a movable light source and a series of linearly arranged photo diodes or photo detectors, an optical sensing system comprising a fixed light source and a series of movable linearly, arranged photo diodes or photo detectors, or any combination thereof.

The electric motor 482 can include a rotatable shaft that operably interfaces with a gear assembly that is mounted in meshing engagement with a set, or rack, of drive teeth on the displacement member. A sensor element is operably coupled to a gear assembly such that a single revolution of the position sensor 472 element corresponds to some linear longitudinal translation of the displacement member. An arrangement of gearing and sensors is connected to the linear actuator, via a rack and pinion arrangement, or a rotary actuator, via a spur gear or other connection. A power source supplies power to the absolute positioning system and an output indicator may display the output of the absolute positioning system. The displacement member represents the longitudinally movable drive member comprising a rack of drive teeth formed thereon for meshing engagement with a corresponding drive gear of the gear reducer assembly. The displacement member represents the longitudinally movable firing member, firing bar, I-beam, or combinations thereof.

A single revolution of the sensor element associated with the position sensor 472 is equivalent to a longitudinal linear displacement d1 of the of the displacement member, where d1 is the longitudinal linear distance that the displacement member moves from point "a" to point "b" after a single revolution of the sensor element coupled to the displacement member. The sensor arrangement may be connected via a gear reduction that results in the position sensor 472 completing one or more revolutions for the full stroke of the displacement member. The position sensor 472 may complete multiple revolutions for the full stroke of the displacement member.

A series of switches, where n is an integer greater than one, is employed alone or in combination with a gear reduction to provide a unique position signal for more than one revolution of the position sensor 472. The state of the switches are fed back to the microcontroller 461 that applies logic to determine a unique position signal corresponding to the longitudinal linear displacement d1 + d2 + ... dn of the displacement member. The output of the position sensor 472 is provided to the microcontroller 461. The position sensor 472 of the sensor arrangement comprises a magnetic sensor, an analog rotary sensor like a potentiometer, or an array of analog Hall-effect elements, which output a unique combination of position signals or values.

The position sensor 472 comprisea any number of magnetic sensing elements, such as, for example, magnetic sensors classified according to whether they measure the total magnetic field or the vector components of the magnetic field. The techniques used to produce both types of magnetic sensors encompass many aspects of physics and electronics. The technologies used for magnetic field sensing include search coil, fluxgate, optically pumped, nuclear precession, SQUID, Hall-effect, anisotropic magnetoresistance, giant magnetoresistance, magnetic tunnel junctions, giant magnetoimpedance, magnetostrictive/piezoelectric composites, magnetodiode, magnetotransistor, fiber-optic, magneto-optic, and microelectromechanical systems-based magnetic sensors, among others.

The position sensor 472 for the tracking system 480 comprising an absolute positioning system comprises a magnetic rotary absolute positioning system. The position sensor 472 may be implemented as an AS5055EQFT single-chip magnetic rotary position sensor available from Austria Microsystems, AG. The position sensor 472 is interfaced with the microcontroller 461 to provide an absolute positioning system. The position sensor 472 is a low-voltage and low-power component and includes four Hall-effect elements in an area of the position sensor 472 that is located above a magnet. A high-resolution ADC and a smart power management controller are also provided on the chip. A coordinate rotation digital computer (CORDIC) processor, also known as the digit-by-digit method and Volder's algorithm, is provided to implement a simple and efficient algorithm to calculate hyperbolic and trigonometric functions that require only addition, subtraction, bitshift, and table lookup operations. The angle position, alarm bits, and magnetic field information are transmitted over a standard serial communication interface, such as a serial peripheral interface (SPI) interface, to the microcontroller 461. The position sensor 472 provides 12 or 14 bits of resolution. The position sensor 472 may be an AS5055 chip provided in a small QFN 16-pin 4x4x0.85mm package.

The tracking system 480 comprising an absolute positioning system comprises and/or is programmed to implement a feedback controller, such as a PID, state feedback, and adaptive controller. A power source converts the signal from the feedback controller into a physical input to the system: in this case the voltage. Other examples include a PWM of the voltage, current, and force. Other sensor(s) may be provided to measure physical parameters of the physical system in addition to the position measured by the position sensor 472. The other sensor(s) can include sensor arrangements such as those described in U.S. Patent No. 9,345,481, titled STAPLE CARTRIDGE TISSUE THICKNESS SENSOR SYSTEM, which issued on May 24, 2016; U.S. Patent Application Publication No. 2014/0263552, titled STAPLE CARTRIDGE TISSUE THICKNESS SENSOR SYSTEM, which published on September 18, 2014; and U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017. In a digital signal processing system, an absolute positioning system is coupled to a digital data acquisition system where the output of the absolute positioning system will have a finite resolution and sampling frequency. The absolute positioning system comprises a compare-and-combine circuit to combine a computed response with a measured response using algorithms, such as a weighted average and a theoretical control loop, that drive the computed response towards the measured response. The computed response of the physical system takes into account properties like mass, inertial, viscous friction, inductance resistance, etc., to predict what the states and outputs of the physical system will be by knowing the input.

The absolute positioning system provides an absolute position of the displacement member upon power-up of the instrument, without retracting or advancing the displacement member to a reset (zero or home) position as may be required with conventional rotary encoders that merely count the number of steps forwards or backwards that the motor 482 has taken to infer the position of a device actuator, drive bar, knife, or the like.

A sensor 474, such as, for example, a strain gauge or a micro-strain gauge, is configured to measure one or more parameters of the end effector, such as, for example, the amplitude of the strain exerted on the anvil during a clamping operation, which is indicative of the closure forces applied to the anvil. The measured strain is converted to a digital signal and provided to the processor 462. Alternatively, or in addition to the sensor 474, a sensor 476, such as, for example, a load sensor, measures the closure force applied by the closure drive system to the anvil. The sensor 476, such as, for example, a load sensor, can measure the firing force applied to an I-beam in a firing stroke of the surgical instrument or tool. The I-beam is configured to engage a wedge sled, which is configured to upwardly cam staple drivers to force out staples into deforming contact with an anvil. The I-beam also includes a sharpened cutting edge that is used to sever tissue as the I-beam is advanced distally by the firing bar. Alternatively, a current sensor 478 is employed to measure the current drawn by the motor 482. The force required to advance the firing member corresponds to the current drawn by the motor 482, for example. The measured force is converted to a digital signal and provided to the processor 462.

The strain gauge sensor 474 is used to measure the force applied to the tissue by the end effector. A strain gauge is coupled to the end effector to measure the force on the tissue being treated by the end effector. A system for measuring forces applied to the tissue grasped by the end effector comprises a strain gauge sensor 474, such as, for example, a micro-strain gauge, that is configured to measure one or more parameters of the end effector, for example. The strain gauge sensor 474 measures the amplitude or magnitude of the strain exerted on a jaw member of an end effector during a clamping operation, which is indicative of the tissue compression. The measured strain is converted to a digital signal and provided to a processor 462 of the microcontroller 461. A load sensor 476 measures the force used to operate the knife element, for example, to cut the tissue captured between the anvil and the staple cartridge. A magnetic field sensor is employed to measure the thickness of the captured tissue. The measurement of the magnetic field sensor also may be converted to a digital signal and provided to the processor 462.

The measurements of the tissue compression, the tissue thickness, and/or the force required to close the end effector on the tissue, as respectively measured by the sensors 474, 476, is used by the microcontroller 461 to characterize the selected position of the firing member and/or the corresponding value of the speed of the firing member. A memory 468 stores a technique, an equation, and/or a lookup table which is employed by the microcontroller 461 in the assessment.

The control system 470 of the surgical instrument or tool also comprises wired or wireless communication circuits to communicate with the modular communication hub as shown in FIGS. 8-11.

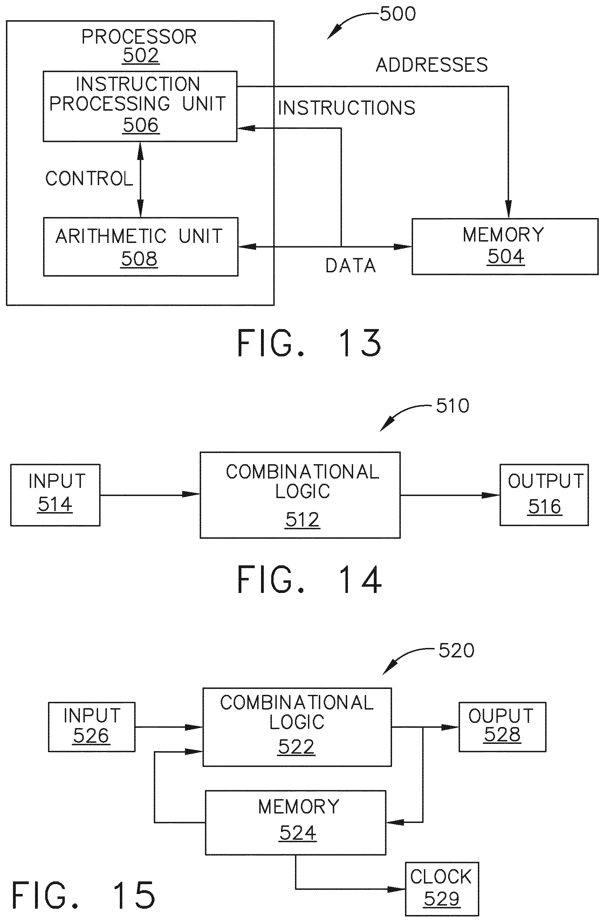

FIG. 13 illustrates a control circuit 500 configured to control aspects of the surgical instrument or tool. The control circuit 500 is configured to implement various processes described herein. The control circuit 500 comprises a microcontroller comprising one or more processors 502 (e.g., microprocessor, microcontroller) coupled to at least one memory circuit 504. The memory circuit 504 stores machine-executable instructions that, when executed by the processor 502, cause the processor 502 to execute machine instructions to implement various processes described herein. The processor 502 is any one of a number of single-core or multicore processors known in the art. The memory circuit 504 comprises volatile and non-volatile storage media. The processor 502 includes an instruction processing unit 506 and an arithmetic unit 508. The instruction processing unit is configured to receive instructions from the memory circuit 504 of this disclosure.

FIG. 14 illustrates a combinational logic circuit 510 configured to control aspects of the surgical instrument or tool. The combinational logic circuit 510 can be configured to implement various processes described herein. The combinational logic circuit 510 comprises a finite state machine comprising a combinational logic 512 configured to receive data associated with the surgical instrument or tool at an input 514, process the data by the combinational logic 512, and provide an output 516.

FIG. 15 illustrates a sequential logic circuit 520 configured to control aspects of the surgical instrument or tool. The sequential logic circuit 520 or the combinational logic 522 is configured to implement various processes described herein. The sequential logic circuit 520 comprises a finite state machine. The sequential logic circuit 520 comprises a combinational logic 522, at least one memory circuit 524, and a clock 529, for example. The at least one memory circuit 524 stores a current state of the finite state machine. The sequential logic circuit 520 is synchronous or asynchronous. The combinational logic 522 is configured to receive data associated with the surgical instrument or tool from an input 526, process the data by the combinational logic 522, and provide an output 528. The circuit comprises a combination of a processor (e.g., processor 502, FIG. 13) and a finite state machine to implement various processes herein. The finite state machine comprises a combination of a combinational logic circuit (e.g., combinational logic circuit 510, FIG. 14) and the sequential logic circuit 520.

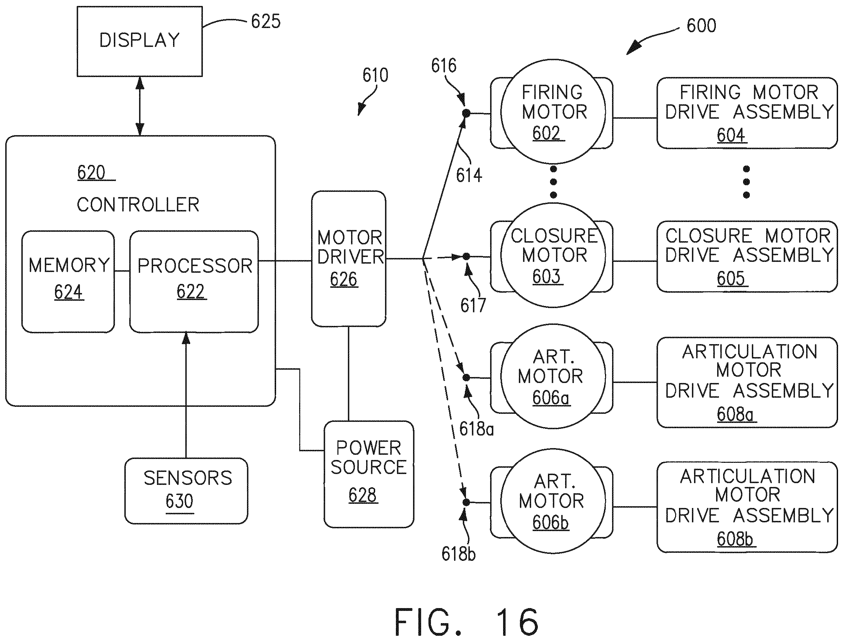

FIG. 16 illustrates a surgical instrument or tool comprising a plurality of motors which are activated to perform various functions. A first motor is activated to perform a first function, a second motor is activated to perform a second function, a third motor is activated to perform a third function, a fourth motor is activated to perform a fourth function, and so on. The plurality of motors of robotic surgical instrument 600 are individually activated to cause firing, closure, and/or articulation motions in the end effector. The firing, closure, and/or articulation motions are transmitted to the end effector through a shaft assembly, for example.

The surgical instrument system or tool includes a firing motor 602. The firing motor 602 is operably coupled to a firing motor drive assembly 604 which is configured to transmit firing motions, generated by the motor 602 to the end effector, in particular to displace the I-beam element. The firing motions generated by the motor 602 cause the staples to be deployed from the staple cartridge into tissue captured by the end effector and/or the cutting edge of the I-beam element to be advanced to cut the captured tissue, for example. The I-beam element is retracted by reversing the direction of the motor 602.

The surgical instrument or tool includes a closure motor 603. The closure motor 603 is operably coupled to a closure motor drive assembly 605 which is configured to transmit closure motions, generated by the motor 603 to the end effector, in particular to displace a closure tube to close the anvil and compress tissue between the anvil and the staple cartridge. The closure motions cause the end effector to transition from an open configuration to an approximated configuration to capture tissue, for example. The end effector is transitioned to an open position by reversing the direction of the motor 603.

The surgical instrument or tool includes one or more articulation motors 606a, 606b, for example. The motors 606a, 606b are operably coupled to respective articulation motor drive assemblies 608a, 608b, which are configured to transmit articulation motions generated by the motors 606a, 606b to the end effector. The articulation motions causes the end effector to articulate relative to the shaft, for example.

As described above, the surgical instrument or tool includes a plurality of motors which are configured to perform various independent functions. The plurality of motors of the surgical instrument or tool are individually or separately activated to perform one or more functions while the other motors remain inactive. For example, the articulation motors 606a, 606b are activated to cause the end effector to be articulated while the firing motor 602 remains inactive. Alternatively, the firing motor 602 is activated to fire the plurality of staples, and/or to advance the cutting edge, while the articulation motor 606 remains inactive. Furthermore the closure motor 603 is activated simultaneously with the firing motor 602 to cause the closure tube and the I-beam element to advance distally as described in more detail hereinbelow.

The surgical instrument or tool includes a common control module 610 which is employed with a plurality of motors of the surgical instrument or tool. The common control module 610 accommodates one of the plurality of motors at a time. For example, the common control module 610 is couplable to and separable from the plurality of motors of the robotic surgical instrument individually. A plurality of the motors of the surgical instrument or tool shares one or more common control modules such as the common control module 610. A plurality of motors of the surgical instrument or tool are individually and selectively engaged with the common control module 610. The common control module 610 is selectively switched from interfacing with one of a plurality of motors of the surgical instrument or tool to interfacing with another one of the plurality of motors of the surgical instrument or tool.

The common control module 610 is selectively switched between operable engagement with the articulation motors 606a, 606b and operable engagement with either the firing motor 602 or the closure motor 603. As illustrated in FIG. 16, a switch 614 is moved or transitioned between a plurality of positions and/or states. In a first position 616, the switch 614 electrically couples the common control module 610 to the firing motor 602; in a second position 617, the switch 614 electrically couples the common control module 610 to the closure motor 603; in a third position 618a, the switch 614 electrically couples the common control module 610 to the first articulation motor 606a; and in a fourth position 618b, the switch 614 electrically couples the common control module 610 to the second articulation motor 606b, for example. In certain instances, separate common control modules 610 are electrically coupled to the firing motor 602, the closure motor 603, and the articulations motor 606a, 606b at the same time. The switch 614 may be a mechanical switch, an electromechanical switch, a solid-state switch, or any suitable switching mechanism.

Each of the motors 602, 603, 606a, 606b comprises a torque sensor to measure the output torque on the shaft of the motor. The force on an end effector is sensed in any conventional manner, such as by force sensors on the outer sides of the jaws or by a torque sensor for the motor actuating the jaws.

As illustrated in FIG. 16, the common control module 610 comprises a motor driver 626 which comprises one or more H-Bridge FETs. The motor driver 626 modulates the power transmitted from a power source 628 to a motor coupled to the common control module 610 based on input from a microcontroller 620 (the "controller"), for example. The microcontroller 620 is employed to determine the current drawn by the motor while the motor is coupled to the common control module 610, as described above.

The microcontroller 620 includes a microprocessor 622 (the "processor") and one or more non-transitory computer-readable mediums or memory units 624 (the "memory"). The memory 624 stores various program instructions, which when executed cause the processor 622 to perform a plurality of functions and/or calculations described herein. One or more of the memory units 624 is coupled to the processor 622.

The power source 628 is employed to supply power to the microcontroller 620. The power source 628 may comprise a battery (or "battery pack" or "power pack"), such as a lithium-ion battery, for example. The battery pack is configured to be releasably mounted to a handle for supplying power to the surgical instrument 600. A number of battery cells connected in series is used as the power source 628. The power source 628 may be replaceable and/or rechargeable.

The processor 622 controls the motor driver 626 to control the position, direction of rotation, and/or velocity of a motor that is coupled to the common control module 610. The processor 622 signals the motor driver 626 to stop and/or disable a motor that is coupled to the common control module 610. It should be understood that the term "processor" as used herein includes any suitable microprocessor, microcontroller, or other basic computing device that incorporates the functions of a computer's central processing unit (CPU) on an integrated circuit or, at most, a few integrated circuits. The processor is a multipurpose, programmable device that accepts digital data as input, processes it according to instructions stored in its memory, and provides results as output. It is an example of sequential digital logic, as it has internal memory. Processors operate on numbers and symbols represented in the binary numeral system.

The processor 622 may be any single-core or multicore processor such as those known under the trade name ARM Cortex by Texas Instruments. The microcontroller 620 may be an LM 4F230H5QR, available from Texas Instruments. the Texas Instruments LM4F230H5QR is an ARM Cortex-M4F Processor Core comprising an on-chip memory of 256 KB single-cycle flash memory, or other non-volatile memory, up to 40 MHz, a prefetch buffer to improve performance above 40 MHz, a 32 KB single-cycle SRAM, an internal ROM loaded with StellarisWare® software, a 2 KB EEPROM, one or more PWM modules, one or more QEI analogs, one or more 12-bit ADCs with 12 analog input channels, among other features that are readily available for the product datasheet. Other microcontrollers may be readily substituted for use with the module 4410. Accordingly, the present disclosure should not be limited in this context.

The memory 624 includes program instructions for controlling each of the motors of the surgical instrument 600 that are couplable to the common control module 610. The memory 624 includes program instructions for controlling the firing motor 602, the closure motor 603, and the articulation motors 606a, 606b. Such program instructions cause the processor 622 to control the firing, closure, and articulation functions in accordance with inputs from algorithms or control programs of the surgical instrument or tool.

One or more mechanisms and/or sensors such as sensors 630 are employed to alert the processor 622 to the program instructions that should be used in a particular setting. The sensors 630 alert the processor 622 to use the program instructions associated with firing, closing, and articulating the end effector. The sensors 630 comprise position sensors which are be employed to sense the position of the switch 614. Accordingly, the processor 622 uses the program instructions associated with firing the I-beam of the end effector upon detecting, through the sensors 630, that the switch 614 is in the first position 616; the processor 622 uses the program instructions associated with closing the anvil upon detecting, through the sensors 630, that the switch 614 is in the second position 617; and the processor 622 uses the program instructions associated with articulating the end effector upon detecting, through the sensors 630, that the switch 614 is in the third or fourth position 618a, 618b.

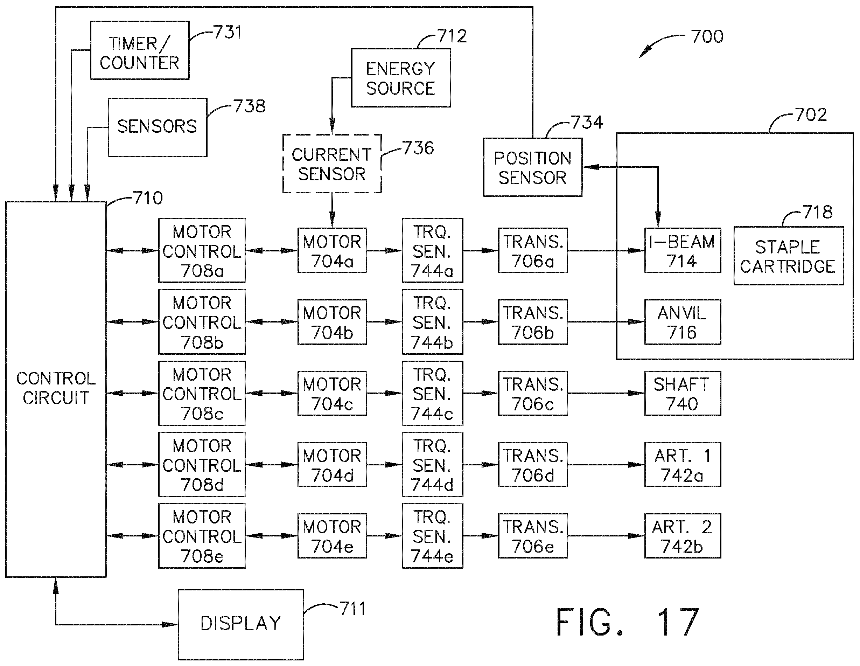

FIG. 17 is a schematic diagram of a robotic surgical instrument 700 configured to operate a surgical tool described herein . The robotic surgical instrument 700 is programmed or configured to control distal/proximal translation of a displacement member, distal/proximal displacement of a closure tube, shaft rotation, and articulation, either with single or multiple articulation drive links. The surgical instrument 700 is programmed or configured to individually control a firing member, a closure member, a shaft member, and/or one or more articulation members. The surgical instrument 700 comprises a control circuit 710 configured to control motor-driven firing members, closure members, shaft members, and/or one or more articulation members.

The robotic surgical instrument 700 comprises a control circuit 710 configured to control an anvil 716 and an I-beam 714 (including a sharp cutting edge) portion of an end effector 702, a removable staple cartridge 718, a shaft 740, and one or more articulation members 742a, 742b via a plurality of motors 704a-704e. A position sensor 734 is configured to provide position feedback of the I-beam 714 to the control circuit 710. Other sensors 738 are configured to provide feedback to the control circuit 710. A timer/counter 731 provides timing and counting information to the control circuit 710. An energy source 712 is provided to operate the motors 704a-704e, and a current sensor 736 provides motor current feedback to the control circuit 710. The motors 704a-704e are operated individually by the control circuit 710 in a open-loop or closed-loop feedback control.

The control circuit 710 comprises one or more microcontrollers, microprocessors, or other suitable processors for executing instructions that cause the processor or processors to perform one or more tasks. A timer/counter 731 provides an output signal, such as the elapsed time or a digital count, to the control circuit 710 to correlate the position of the I-beam 714 as determined by the position sensor 734 with the output of the timer/counter 731 such that the control circuit 710 can determine the position of the I-beam 714 at a specific time (t) relative to a starting position or the time (t) when the I-beam 714 is at a specific position relative to a starting position. The timer/counter 731 is configured to measure elapsed time, count external events, or time external events.

The control circuit 710 is programmed to control functions of the end effector 702 based on one or more tissue conditions. The control circuit 710 is programmed to sense tissue conditions, such as thickness, either directly or indirectly, as described herein. The control circuit 710 is programmed to select a firing control program or closure control program based on tissue conditions. A firing control program describes the distal motion of the displacement member. Different firing control programs are selected to better treat different tissue conditions. For example, when thicker tissue is present, the control circuit 710 is programmed to translate the displacement member at a lower velocity and/or with lower power. When thinner tissue is present, the control circuit 710 is programmed to translate the displacement member at a higher velocity and/or with higher power. A closure control program controls the closure force applied to the tissue by the anvil 716. Other control programs control the rotation of the shaft 740 and the articulation members 742a, 742b.

The control circuit 710 generates motor set point signals. The motor set point signals are provided to various motor controllers 708a-708e. The motor controllers 708a-708e comprise one or more circuits configured to provide motor drive signals to the motors 704a-704e to drive the motors 704a-704e as described herein. The motors 704a-704e may be brushed DC electric motors. The velocity of the motors 704a-704e are proportional to the respective motor drive signals. In some examples, the motors 704a-704e may be brushless DC electric motors, and the respective motor drive signals may comprise a PWM signal provided to one or more stator windings of the motors 704a-704e. Also, in some examples, the motor controllers 708a-708e may be omitted and the control circuit 710 may generate the motor drive signals directly.

The control circuit 710 initially operates each of the motors 704a-704e in an open-loop configuration for a first open-loop portion of a stroke of the displacement member. Based on the response of the robotic surgical instrument 700 during the open-loop portion of the stroke, the control circuit 710 selects a firing control program in a closed-loop configuration. The response of the instrument includes a translation distance of the displacement member during the open-loop portion, a time elapsed during the open-loop portion, the energy provided to one of the motors 704a-704e during the open-loop portion, a sum of pulse widths of a motor drive signal, etc. After the open-loop portion, the control circuit 710 implements the selected firing control program for a second portion of the displacement member stroke. For example, during a closed-loop portion of the stroke, the control circuit 710 modulates one of the motors 704a-704e based on translation data describing a position of the displacement member in a closed-loop manner to translate the displacement member at a constant velocity.

The motors 704a-704e receive power from an energy source 712. The energy source 712 is a DC power supply driven by a main alternating current power source, a battery, a super capacitor, or any other suitable energy source. The motors 704a-704e are mechanically coupled to individual movable mechanical elements such as the I-beam 714, anvil 716, shaft 740, articulation 742a, and articulation 742b via respective transmissions 706a-706e. The transmissions 706a-706e include one or more gears or other linkage components to couple the motors 704a-704e to movable mechanical elements. A position sensor 734 senses a position of the I-beam 714. The position sensor 734 includes any type of sensor that is capable of generating position data that indicate a position of the I-beam 714. The position sensor 734 includes an encoder configured to provide a series of pulses to the control circuit 710 as the I-beam 714 translates distally and proximally. The control circuit 710 tracks the pulses to determine the position of the I-beam 714. Other suitable position sensors may be used, including, for example, a proximity sensor. Other types of position sensors provide other signals indicating motion of the I-beam 714. Also, in some examples, the position sensor 734 may be omitted. Where any of the motors 704a-704e is a stepper motor, the control circuit 710 tracks the position of the I-beam 714 by aggregating the number and direction of steps that the motor 704 has been instructed to execute. The position sensor 734 is located in the end effector 702 or at any other portion of the instrument. The outputs of each of the motors 704a-704e include a torque sensor 744a-744e to sense force and have an encoder to sense rotation of the drive shaft.

The control circuit 710 is configured to drive a firing member such as the I-beam 714 portion of the end effector 702. The control circuit 710 provides a motor set point to a motor control 708a, which provides a drive signal to the motor 704a. The output shaft of the motor 704a is coupled to a torque sensor 744a. The torque sensor 744a is coupled to a transmission 706a which is coupled to the I-beam 714. The transmission 706a comprises movable mechanical elements such as rotating elements and a firing member to control the movement of the I-beam 714 distally and proximally along a longitudinal axis of the end effector 702. The motor 704a is coupled to the knife gear assembly, which includes a knife gear reduction set that includes a first knife drive gear and a second knife drive gear. A torque sensor 744a provides a firing force feedback signal to the control circuit 710. The firing force signal represents the force required to fire or displace the I-beam 714. A position sensor 734 is configured to provide the position of the I-beam 714 along the firing stroke or the position of the firing member as a feedback signal to the control circuit 710. The end effector 702 includes additional sensors 738 configured to provide feedback signals to the control circuit 710. When ready to use, the control circuit 710 provides a firing signal to the motor control 708a. In response to the firing signal, the motor 704a drives the firing member distally along the longitudinal axis of the end effector 702 from a proximal stroke start position to a stroke end position distal to the stroke start position. As the firing member translates distally, an I-beam 714, with a cutting element positioned at a distal end, advances distally to cut tissue located between the staple cartridge 718 and the anvil 716.

The control circuit 710 is configured to drive a closure member such as the anvil 716 portion of the end effector 702. The control circuit 710 provides a motor set point to a motor control 708b, which provides a drive signal to the motor 704b. The output shaft of the motor 704b is coupled to a torque sensor 744b. The torque sensor 744b is coupled to a transmission 706b which is coupled to the anvil 716. The transmission 706b comprises movable mechanical elements such as rotating elements and a closure member to control the movement of the anvil 716 from the open and closed positions. The motor 704b is coupled to a closure gear assembly, which includes a closure reduction gear set that is supported in meshing engagement with the closure spur gear. The torque sensor 744b provides a closure force feedback signal to the control circuit 710. The closure force feedback signal represents the closure force applied to the anvil 716. The position sensor 734 is configured to provide the position of the closure member as a feedback signal to the control circuit 710. Additional sensors 738 in the end effector 702 provide the closure force feedback signal to the control circuit 710. The pivotable anvil 716 is positioned opposite the staple cartridge 718. When ready to use, the control circuit 710 provides a closure signal to the motor control 708b. In response to the closure signal, the motor 704b advances a closure member to grasp tissue between the anvil 716 and the staple cartridge 718.

The control circuit 710 is configured to rotate a shaft member such as the shaft 740 to rotate the end effector 702. The control circuit 710 provides a motor set point to a motor control 708c, which provides a drive signal to the motor 704c. The output shaft of the motor 704c is coupled to a torque sensor 744c. The torque sensor 744c is coupled to a transmission 706c which is coupled to the shaft 740. The transmission 706c comprises movable mechanical elements such as rotating elements to control the rotation of the shaft 740 clockwise or counterclockwise up to and over 360°. The motor 704c is coupled to the rotational transmission assembly, which includes a tube gear segment that is formed on (or attached to) the proximal end of the proximal closure tube for operable engagement by a rotational gear assembly that is operably supported on the tool mounting plate. The torque sensor 744c provides a rotation force feedback signal to the control circuit 710. The rotation force feedback signal represents the rotation force applied to the shaft 740. The position sensor 734 is configured to provide the position of the closure member as a feedback signal to the control circuit 710. Additional sensors 738 such as a shaft encoder may provide the rotational position of the shaft 740 to the control circuit 710.

The control circuit 710 is configured to articulate the end effector 702. The control circuit 710 provides a motor set point to a motor control 708d, which provides a drive signal to the motor 704d. The output shaft of the motor 704d is coupled to a torque sensor 744d. The torque sensor 744d is coupled to a transmission 706d which is coupled to an articulation member 742a. The transmission 706d comprises movable mechanical elements such as articulation elements to control the articulation of the end effector 702 ±65°. The motor 704d is coupled to an articulation nut, which is rotatably journaled on the proximal end portion of the distal spine portion and is rotatably driven thereon by an articulation gear assembly. The torque sensor 744d provides an articulation force feedback signal to the control circuit 710. The articulation force feedback signal represents the articulation force applied to the end effector 702. Sensors 738, such as an articulation encoder, may provide the articulation position of the end effector 702 to the control circuit 710.

The articulation function of the robotic surgical system 700 comprises two articulation members, or links, 742a, 742b. These articulation members 742a, 742b are driven by separate disks on the robot interface (the rack) which are driven by the two motors 708d, 708e. When the separate firing motor 704a is provided, each of articulation links 742a, 742b can be antagonistically driven with respect to the other link in order to provide a resistive holding motion and a load to the head when it is not moving and to provide an articulation motion as the head is articulated. The articulation members 742a, 742b attach to the head at a fixed radius as the head is rotated. Accordingly, the mechanical advantage of the push-and-pull link changes as the head is rotated. This change in the mechanical advantage may be more pronounced with other articulation link drive systems.

The one or more motors 704a-704e comprises a brushed DC motor with a gearbox and mechanical links to a firing member, closure member, or articulation member. Another example includes electric motors 704a-704e that operate the movable mechanical elements such as the displacement member, articulation links, closure tube, and shaft. An outside influence is an unmeasured, unpredictable influence of things like tissue, surrounding bodies, and friction on the physical system. Such outside influence can be referred to as drag, which acts in opposition to one of electric motors 704a-704e. The outside influence, such as drag, may cause the operation of the physical system to deviate from a desired operation of the physical system.

The position sensor 734 is implemented as an absolute positioning system. The position sensor 734 comprises a magnetic rotary absolute positioning system implemented as an AS5055EQFT single-chip magnetic rotary position sensor available from Austria Microsystems, AG. The position sensor 734 interfaces with the control circuit 710 to provide an absolute positioning system. The position includes multiple Hall-effect elements located above a magnet and coupled to a CORDIC processor, also known as the digit-by-digit method and Volder's algorithm, that is provided to implement a simple and efficient algorithm to calculate hyperbolic and trigonometric functions that require only addition, subtraction, bitshift, and table lookup operations.

The control circuit 710 is in communication with one or more sensors 738. The sensors 738 are positioned on the end effector 702 and adapted to operate with the robotic surgical instrument 700 to measure the various derived parameters such as the gap distance versus time, tissue compression versus time, and anvil strain versus time. The sensors 738 comprise a magnetic sensor, a magnetic field sensor, a strain gauge, a load cell, a pressure sensor, a force sensor, a torque sensor, an inductive sensor such as an eddy current sensor, a resistive sensor, a capacitive sensor, an optical sensor, and/or any other suitable sensor for measuring one or more parameters of the end effector 702. The sensors 738 include one or more sensors. The sensors 738 are be located on the staple cartridge 718 deck to determine tissue location using segmented electrodes. The torque sensors 744a-744e are configured to sense force such as firing force, closure force, and/or articulation force, among others. Accordingly, the control circuit 710 can sense (1) the closure load experienced by the distal closure tube and its position, (2) the firing member at the rack and its position, (3) what portion of the staple cartridge 718 has tissue on it, and (4) the load and position on both articulation rods.

The one or more sensors 738 comprise a strain gauge, such as a micro-strain gauge, configured to measure the magnitude of the strain in the anvil 716 during a clamped condition. The strain gauge provides an electrical signal whose amplitude varies with the magnitude of the strain. The sensors 738 comprise a pressure sensor configured to detect a pressure generated by the presence of compressed tissue between the anvil 716 and the staple cartridge 718. The sensors 738 are configured to detect impedance of a tissue section located between the anvil 716 and the staple cartridge 718 that is indicative of the thickness and/or fullness of tissue located therebetween.

The sensors 738 are implemented as one or more limit switches, electromechanical devices, solid-state switches, Hall-effect devices, magneto-resistive (MR) devices, giant magneto-resistive (GMR) devices, magnetometers, among others. The sensors 738 may be implemented as solid-state switches that operate under the influence of light, such as optical sensors, IR sensors, ultraviolet sensors, among others. Still, the switches may be solid-state devices such as transistors (e.g., FET, junction FET, MOSFET, bipolar, and the like). The sensors 738 may include electrical conductorless switches, ultrasonic switches, accelerometers, and inertial sensors, among others.

The sensors 738 are configured to measure forces exerted on the anvil 716 by the closure drive system. For example, one or more sensors 738 are at an interaction point between the closure tube and the anvil 716 to detect the closure forces applied by the closure tube to the anvil 716. The forces exerted on the anvil 716 can be representative of the tissue compression experienced by the tissue section captured between the anvil 716 and the staple cartridge 718. The one or more sensors 738 are positioned at various interaction points along the closure drive system to detect the closure forces applied to the anvil 716 by the closure drive system. The one or more sensors 738 are sampled in real time during a clamping operation by the processor of the control circuit 710. The control circuit 710 receives real-time sample measurements to provide and analyze time-based information and assess, in real time, closure forces applied to the anvil 716.

A current sensor 736 is employed to measure the current drawn by each of the motors 704a-704e. The force required to advance any of the movable mechanical elements such as the I-beam 714 corresponds to the current drawn by one of the motors 704a-704e. The force is converted to a digital signal and provided to the control circuit 710. The control circuit 710 is configured to simulate the response of the actual system of the instrument in the software of the controller. A displacement member is actuated to move an I-beam 714 in the end effector 702 at or near a target velocity. The robotic surgical instrument 700 includes a feedback controller, which can be one of any feedback controllers, including, but not limited to a PID, a state feedback, a linear-quadratic (LQR), and/or an adaptive controller, for example. The robotic surgical instrument 700 includes a power source to convert the signal from the feedback controller into a physical input such as case voltage, PWM voltage, frequency modulated voltage, current, torque, and/or force, for example. Additional details are disclosed in U.S. Patent Application Serial No. 15/636,829, titled CLOSED LOOP VELOCITY CONTROL TECHNIQUES FOR ROBOTIC SURGICAL INSTRUMENT, filed June 29, 2017.

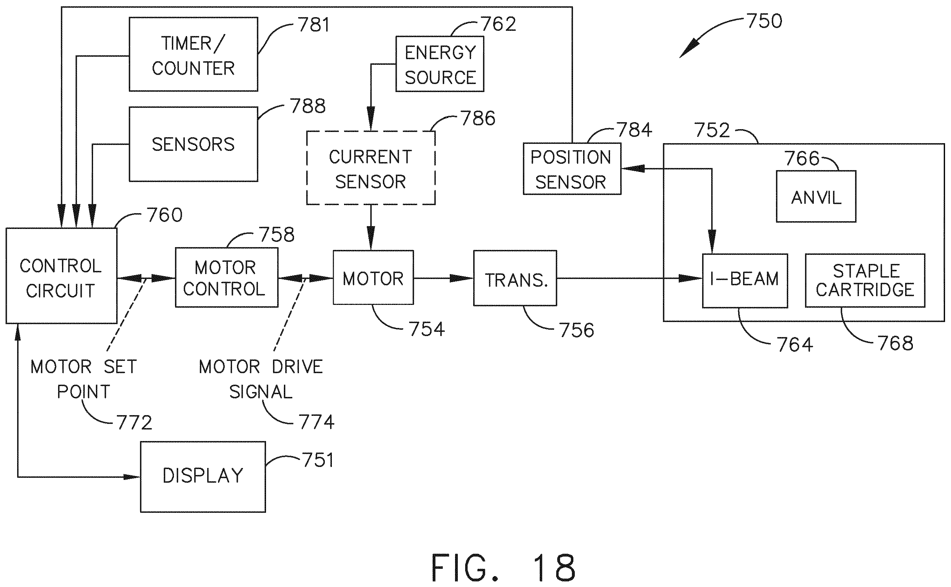

FIG. 18 illustrates a block diagram of a surgical instrument 750 programmed to control the distal translation of a displacement member. The surgical instrument 750 is programmed to control the distal translation of a displacement member such as the I-beam 764. The surgical instrument 750 comprises an end effector 752 that comprises an anvil 766, an I-beam 764 (including a sharp cutting edge), and a removable staple cartridge 768.

The position, movement, displacement, and/or translation of a linear displacement member, such as the I-beam 764, is measured by an absolute positioning system, sensor arrangement, and position sensor 784. Because the I-beam 764 is coupled to a longitudinally movable drive member, the position of the I-beam 764 is determined by measuring the position of the longitudinally movable drive member employing the position sensor 784. Accordingly, in the following description, the position, displacement, and/or translation of the I-beam 764 is achieved by the position sensor 784 as described herein. A control circuit 760 is programmed to control the translation of the displacement member, such as the I-beam 764. The control circuit 760, in some examples, comprises one or more microcontrollers, microprocessors, or other suitable processors for executing instructions that cause the processor or processors to control the displacement member, e.g., the I-beam 764, in the manner described. A timer/counter 781 provides an output signal, such as the elapsed time or a digital count, to the control circuit 760 to correlate the position of the I-beam 764 as determined by the position sensor 784 with the output of the timer/counter 781 such that the control circuit 760 can determine the position of the I-beam 764 at a specific time (t) relative to a starting position. The timer/counter 781 is configured to measure elapsed time, count external events, or time external events.

The control circuit 760 generates a motor set point signal 772. The motor set point signal 772 is provided to a motor controller 758. The motor controller 758 comprises one or more circuits configured to provide a motor drive signal 774 to the motor 754 to drive the motor 754 as described herein. In some examples, the motor 754 is a brushed DC electric motor. The velocity of the motor 754 is proportional to the motor drive signal 774. The motor 754 is a brushless DC electric motor and the motor drive signal 774 comprises a PWM signal provided to one or more stator windings of the motor 754. The motor controller 758 may be omitted, and the control circuit 760 generates the motor drive signal 774 directly.

The motor 754 receives power from an energy source 762. The energy source 762 may be or include a battery, a super capacitor, or any other suitable energy source. The motor 754 is mechanically coupled to the I-beam 764 via a transmission 756. The transmission 756 includes one or more gears or other linkage components to couple the motor 754 to the I-beam 764. A position sensor 784 senses a position of the I-beam 764. The position sensor 784 includes any type of sensor that is capable of generating position data that indicate a position of the I-beam 764. The position sensor 784 includes an encoder configured to provide a series of pulses to the control circuit 760 as the I-beam 764 translates distally and proximally. The control circuit 760 may track the pulses to determine the position of the I-beam 764. Other suitable position sensors are used, including, for example, a proximity sensor. Other types of position sensors provide other signals indicating motion of the I-beam 764. The position sensor 784 may be omitted. Where the motor 754 is a stepper motor, the control circuit 760 tracks the position of the I-beam 764 by aggregating the number and direction of steps that the motor 754 has been instructed to execute. The position sensor 784 is located in the end effector 752 or at any other portion of the instrument.

The control circuit 760 is in communication with one or more sensors 788. The sensors 788 are positioned on the end effector 752 and adapted to operate with the surgical instrument 750 to measure the various derived parameters such as gap distance versus time, tissue compression versus time, and anvil strain versus time. The sensors 788 comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 752. The sensors 788 include one or more sensors.

The one or more sensors 788 comprise a strain gauge, such as a micro-strain gauge, configured to measure the magnitude of the strain in the anvil 766 during a clamped condition. The strain gauge provides an electrical signal whose amplitude varies with the magnitude of the strain. The sensors 788 comprise a pressure sensor configured to detect a pressure generated by the presence of compressed tissue between the anvil 766 and the staple cartridge 768. The sensors 788 are configured to detect impedance of a tissue section located between the anvil 766 and the staple cartridge 768 that is indicative of the thickness and/or fullness of tissue located therebetween.

The sensors 788 are configured to measure forces exerted on the anvil 766 by a closure drive system. For example, one or more sensors 788 can be at an interaction point between a closure tube and the anvil 766 to detect the closure forces applied by a closure tube to the anvil 766. The forces exerted on the anvil 766 can be representative of the tissue compression experienced by the tissue section captured between the anvil 766 and the staple cartridge 768. The one or more sensors 788 are positioned at various interaction points along the closure drive system to detect the closure forces applied to the anvil 766 by the closure drive system. The one or more sensors 788 are sampled in real time during a clamping operation by a processor of the control circuit 760. The control circuit 760 receives real-time sample measurements to provide and analyze time-based information and assess, in real time, closure forces applied to the anvil 766.

A current sensor 786 is employed to measure the current drawn by the motor 754. The force required to advance the I-beam 764 corresponds to the current drawn by the motor 754. The force is converted to a digital signal and provided to the control circuit 760.

The control circuit 760 is configured to simulate the response of the actual system of the instrument in the software of the controller. A displacement member is actuated to move an I-beam 764 in the end effector 752 at or near a target velocity. The surgical instrument 750 includes a feedback controller, which can be one of any feedback controllers, including, but not limited to a PID, a state feedback, LQR, and/or an adaptive controller, for example. The surgical instrument 750 includes a power source to convert the signal from the feedback controller into a physical input such as case voltage, PWM voltage, frequency modulated voltage, current, torque, and/or force, for example.

The actual drive system of the surgical instrument 750 is configured to drive the displacement member, cutting member, or I-beam 764, by a brushed DC motor with gearbox and mechanical links to an articulation and/or knife system. Another example is the electric motor 754 that operates the displacement member and the articulation driver, for example, of an interchangeable shaft assembly. An outside influence is an unmeasured, unpredictable influence of things like tissue, surrounding bodies and friction on the physical system. Such outside influence can be referred to as drag which acts in opposition to the electric motor 754. The outside influence, such as drag, causes the operation of the physical system to deviate from a desired operation of the physical system.

A surgical instrument 750 comprises an end effector 752 with motor-driven surgical stapling and cutting implements. A motor 754 drives a displacement member distally and proximally along a longitudinal axis of the end effector 752. The end effector 752 comprises a pivotable anvil 766 and, when configured for use, a staple cartridge 768 positioned opposite the anvil 766. A clinician grasps tissue between the anvil 766 and the staple cartridge 768, as described herein. When ready to use the instrument 750, the clinician provides a firing signal, for example by depressing a trigger of the instrument 750. In response to the firing signal, the motor 754 drives the displacement member distally along the longitudinal axis of the end effector 752 from a proximal stroke begin position to a stroke end position distal of the stroke begin position. As the displacement member translates distally, an I-beam 764 with a cutting element positioned at a distal end, cuts the tissue between the staple cartridge 768 and the anvil 766.

The surgical instrument 750 comprises a control circuit 760 programmed to control the distal translation of the displacement member, such as the I-beam 764, for example, based on one or more tissue conditions. The control circuit 760 is programmed to sense tissue conditions, such as thickness, either directly or indirectly, as described herein. The control circuit 760 is programmed to select a firing control program based on tissue conditions. A firing control program may describe the distal motion of the displacement member. Different firing control programs are selected to better treat different tissue conditions. For example, when thicker tissue is present, the control circuit 760 is programmed to translate the displacement member at a lower velocity and/or with lower power. When thinner tissue is present, the control circuit 760 is programmed to translate the displacement member at a higher velocity and/or with higher power.

The control circuit 760 initially operates the motor 754 in an open loop configuration for a first open loop portion of a stroke of the displacement member. Based on a response of the instrument 750 during the open loop portion of the stroke, the control circuit 760 selects a firing control program. The response of the instrument includes, a translation distance of the displacement member during the open loop portion, a time elapsed during the open loop portion, energy provided to the motor 754 during the open loop portion, a sum of pulse widths of a motor drive signal, etc. After the open loop portion, the control circuit 760 implements the selected firing control program for a second portion of the displacement member stroke. For example, during the closed loop portion of the stroke, the control circuit 760 modulates the motor 754 based on translation data describing a position of the displacement member in a closed loop manner to translate the displacement member at a constant velocity. Additional details are disclosed in U.S. Patent Application Serial No. 15/720,852, titled SYSTEM AND METHODS FOR CONTROLLING A DISPLAY OF A SURGICAL INSTRUMENT, filed September 29, 2017, .

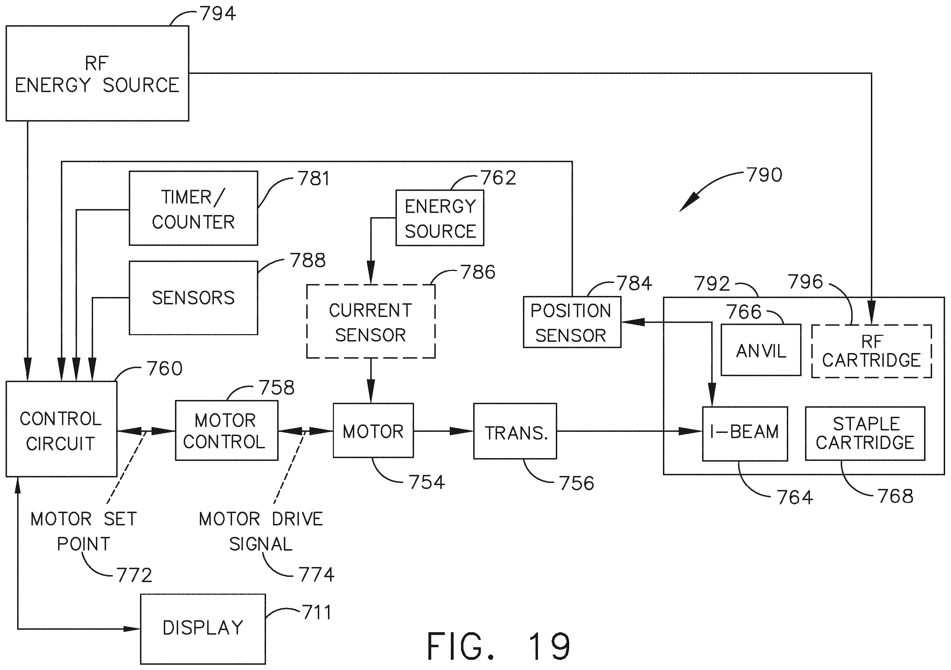

FIG. 19 is a schematic diagram of a surgical instrument 790 configured to control various functions .

The surgical instrument 790 is programmed to control distal translation of a displacement member such as the I-beam 764. The surgical instrument 790 comprises an end effector 792 that comprises an anvil 766, an I-beam 764, and a removable staple cartridge 768 which may be interchanged with an RF cartridge 796 (shown in dashed line).

Sensors 788 are implemented as a limit switch, electromechanical device, solid-state switches, Hall-effect devices, MR devices, GMR devices, magnetometers, among others. The sensors 638 are solid-state switches that operate under the influence of light, such as optical sensors, IR sensors, ultraviolet sensors, among others. Still, the switches are solid-state devices such as transistors (e.g., FET, junction FET, MOSFET, bipolar, and the like). The sensors 788 include electrical conductorless switches, ultrasonic switches, accelerometers, and inertial sensors, among others.

The position sensor 784 is implemented as an absolute positioning system comprising a magnetic rotary absolute positioning system implemented as an AS5055EQFT single-chip magnetic rotary position sensor available from Austria Microsystems, AG. The position sensor 784 interfaces with the control circuit 760 to provide an absolute positioning system. The position includes multiple Hall-effect elements located above a magnet and coupled to a CORDIC processor, also known as the digit-by-digit method and Volder's algorithm, that is provided to implement a simple and efficient algorithm to calculate hyperbolic and trigonometric functions that require only addition, subtraction, bitshift, and table lookup operations.

The I-beam 764 is implemented as a knife member comprising a knife body that operably supports a tissue cutting blade thereon and further includes anvil engagement tabs or features and channel engagement features or a foot. The staple cartridge 768 is implemented as a standard (mechanical) surgical fastener cartridge. The RF cartridge 796 is implemented as an RF cartridge. These and other sensors arrangements are described in commonly-owned U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017.

The position, movement, displacement, and/or translation of a linear displacement member, such as the I-beam 764, is measured by an absolute positioning system, sensor arrangement, and position sensor represented as position sensor 784. Because the I-beam 764 is coupled to the longitudinally movable drive member, the position of the I-beam 764 is determined by measuring the position of the longitudinally movable drive member employing the position sensor 784. Accordingly, in the following description, the position, displacement, and/or translation of the I-beam 764 is achieved by the position sensor 784 as described herein. A control circuit 760 is programmed to control the translation of the displacement member, such as the I-beam 764, as described herein. The control circuit 760, in some examples, comprises one or more microcontrollers, microprocessors, or other suitable processors for executing instructions that cause the processor or processors to control the displacement member, e.g., the I-beam 764, in the manner described. A timer/counter 781 provides an output signal, such as the elapsed time or a digital count, to the control circuit 760 to correlate the position of the I-beam 764 as determined by the position sensor 784 with the output of the timer/counter 781 such that the control circuit 760 can determine the position of the I-beam 764 at a specific time (t) relative to a starting position. The timer/counter 781 is configured to measure elapsed time, count external events, or time external events.

The control circuit 760 generates a motor set point signal 772. The motor set point signal 772 is provided to a motor controller 758. The motor controller 758 comprises one or more circuits configured to provide a motor drive signal 774 to the motor 754 to drive the motor 754 as described herein. The motor 754 is a brushed DC electric motor. The velocity of the motor 754 is proportional to the motor drive signal 774. In some examples, the motor 754 is a brushless DC electric motor and the motor drive signal 774 may comprise a PWM signal provided to one or more stator windings of the motor 754. Also, in some examples, the motor controller 758 is omitted, and the control circuit 760 generates the motor drive signal 774 directly.

The motor 754 receives power from an energy source 762. The energy source 762 may be or include a battery, a super capacitor, or any other suitable energy source. The motor 754 is mechanically coupled to the I-beam 764 via a transmission 756. The transmission 756 includes one or more gears or other linkage components to couple the motor 754 to the I-beam 764. A position sensor 784 senses a position of the I-beam 764. The position sensor 784 includes any type of sensor that is capable of generating position data that indicate a position of the I-beam 764. The position sensor 784 includes an encoder configured to provide a series of pulses to the control circuit 760 as the I-beam 764 translates distally and proximally. The control circuit 760 tracks the pulses to determine the position of the I-beam 764. Other suitable position sensors are used, including, for example, a proximity sensor. Other types of position sensors provide other signals indicating motion of the I-beam 764. Also, in some examples, the position sensor 784 is omitted. Where the motor 754 is a stepper motor, the control circuit 760 tracks the position of the I-beam 764 by aggregating the number and direction of steps that the motor has been instructed to execute. The position sensor 784 is located in the end effector 792 or at any other portion of the instrument.

The control circuit 760 is in communication with one or more sensors 788. The sensors 788 are positioned on the end effector 792 and adapted to operate with the surgical instrument 790 to measure the various derived parameters such as gap distance versus time, tissue compression versus time, and anvil strain versus time. The sensors 788 comprise a magnetic sensor, a magnetic field sensor, a strain gauge, a pressure sensor, a force sensor, an inductive sensor such as an eddy current sensor, a resistive sensor, a capacitive sensor, an optical sensor, and/or any other suitable sensor for measuring one or more parameters of the end effector 792. The sensors 788 include one or more sensors.

The one or more sensors 788 comprise a strain gauge, such as a micro-strain gauge, configured to measure the magnitude of the strain in the anvil 766 during a clamped condition. The strain gauge provides an electrical signal whose amplitude varies with the magnitude of the strain. The sensors 788 comprise a pressure sensor configured to detect a pressure generated by the presence of compressed tissue between the anvil 766 and the staple cartridge 768. The sensors 788 are configured to detect impedance of a tissue section located between the anvil 766 and the staple cartridge 768 that is indicative of the thickness and/or fullness of tissue located therebetween.

The sensors 788 are configured to measure forces exerted on the anvil 766 by the closure drive system. For example, one or more sensors 788 is at an interaction point between a closure tube and the anvil 766 to detect the closure forces applied by a closure tube to the anvil 766. The forces exerted on the anvil 766 are representative of the tissue compression experienced by the tissue section captured between the anvil 766 and the staple cartridge 768. The one or more sensors 788 are positioned at various interaction points along the closure drive system to detect the closure forces applied to the anvil 766 by the closure drive system. The one or more sensors 788 are sampled in real time during a clamping operation by a processor portion of the control circuit 760. The control circuit 760 receives real-time sample measurements to provide and analyze time-based information and assess, in real time, closure forces applied to the anvil 766.

A current sensor 786 is employed to measure the current drawn by the motor 754. The force required to advance the I-beam 764 corresponds to the current drawn by the motor 754. The force is converted to a digital signal and provided to the control circuit 760.

An RF energy source 794 is coupled to the end effector 792 and is applied to the RF cartridge 796 when the RF cartridge 796 is loaded in the end effector 792 in place of the staple cartridge 768. The control circuit 760 controls the delivery of the RF energy to the RF cartridge 796.

Additional details are disclosed in U.S. Patent Application Serial No. 15/636,096, titled SURGICAL SYSTEM COUPLABLE WITH STAPLE CARTRIDGE AND RADIO FREQUENCY CARTRIDGE, AND METHOD OF USING SAME, filed June 28, 2017.

Generator Hardware

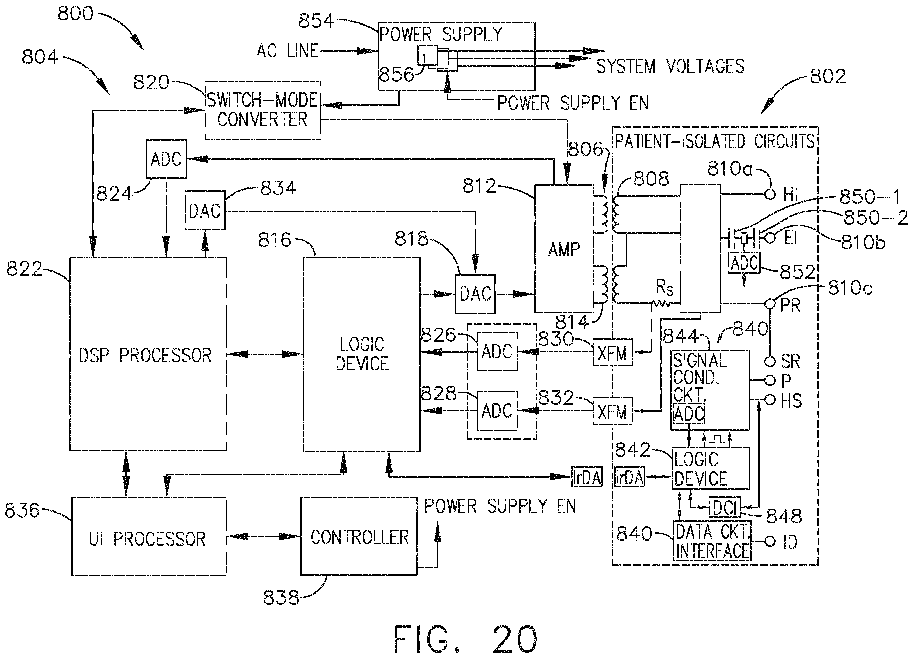

FIG. 20 is a simplified block diagram of a generator 800 configured to provide inductorless tuning, among other benefits. Additional details of the generator 800 are described in U.S. Patent No. 9,060,775, titled SURGICAL GENERATOR FOR ULTRASONIC AND ELECTROSURGICAL DEVICES, which issued on June 23, 2015, . The generator 800 comprises a patient isolated stage 802 in communication with a non-isolated stage 804 via a power transformer 806. A secondary winding 808 of the power transformer 806 is contained in the isolated stage 802 and comprises a tapped configuration (e.g., a center-tapped or a non-center-tapped configuration) to define drive signal outputs 810a, 810b, 810c for delivering drive signals to different surgical instruments, such as, for example, an ultrasonic surgical instrument, an RF electrosurgical instrument, and a multifunction surgical instrument which includes ultrasonic and RF energy modes that can be delivered alone or simultaneously. In particular, drive signal outputs 810a, 810c output an ultrasonic drive signal (e.g., a 420V root-mean-square (RMS) drive signal) to an ultrasonic surgical instrument, and drive signal outputs 810b, 810c output an RF electrosurgical drive signal (e.g., a 100V RMS drive signal) to an RF electrosurgical instrument, with the drive signal output 810b corresponding to the center tap of the power transformer 806.

The ultrasonic and electrosurgical drive signals are provided simultaneously to distinct surgical instruments and/or to a single surgical instrument, such as the multifunction surgical instrument, having the capability to deliver both ultrasonic and electrosurgical energy to tissue. It will be appreciated that the electrosurgical signal, provided either to a dedicated electrosurgical instrument and/or to a combined multifunction ultrasonic/electrosurgical instrument is either a therapeutic or sub-therapeutic level signal where the sub-therapeutic signal is used, for example, to monitor tissue or instrument conditions and provide feedback to the generator. For example, the ultrasonic and RF signals are delivered separately or simultaneously from a generator with a single output port in order to provide the desired output signal to the surgical instrument, as will be discussed in more detail below. Accordingly, the generator combines the ultrasonic and electrosurgical RF energies and deliver the combined energies to the multifunction ultrasonic/electrosurgical instrument. Bipolar electrodes are placed on one or both jaws of the end effector. One jaw may be driven by ultrasonic energy in addition to electrosurgical RF energy, working simultaneously. The ultrasonic energy is employed to dissect tissue, while the electrosurgical RF energy is employed for vessel sealing.

The non-isolated stage 804 comprises a power amplifier 812 having an output connected to a primary winding 814 of the power transformer 806. The power amplifier 812 comprises a push-pull amplifier. The non-isolated stage 804 further comprises a logic device 816 for supplying a digital output to a digital-to-analog converter (DAC) circuit 818, which in turn supplies a corresponding analog signal to an input of the power amplifier 812. In certain forms, the logic device 816 comprises a programmable gate array (PGA), a FPGA, programmable logic device (PLD), among other logic circuits, for example. The logic device 816, by virtue of controlling the input of the power amplifier 812 via the DAC circuit 818, therefore controls any of a number of parameters (e.g., frequency, waveform shape, waveform amplitude) of drive signals appearing at the drive signal outputs 810a, 810b, 810c. As discussed below, the logic device 816, in conjunction with a processor (e.g., a DSP discussed below), implements a number of DSP-based and/or other control algorithms to control parameters of the drive signals output by the generator 800.

Power is supplied to a power rail of the power amplifier 812 by a switch-mode regulator 820, e.g., a power converter. The switch-mode regulator 820 comprises an adjustable buck regulator. The non-isolated stage 804 further comprises a first processor 822, which in one form comprises a DSP processor such as an Analog Devices ADSP-21469 SHARC DSP, available from Analog Devices, Norwood, MA, for example, although in various forms any suitable processor is employed. The DSP processor 822 controls the operation of the switch-mode regulator 820 responsive to voltage feedback data received from the power amplifier 812 by the DSP processor 822 via an ADC circuit 824. , The DSP processor 822 receives as input, via the ADC circuit 824, the waveform envelope of a signal (e.g., an RF signal) being amplified by the power amplifier 812. The DSP processor 822 then controls the switch-mode regulator 820 (e.g., via a PWM output) such that the rail voltage supplied to the power amplifier 812 tracks the waveform envelope of the amplified signal. By dynamically modulating the rail voltage of the power amplifier 812 based on the waveform envelope, the efficiency of the power amplifier 812 is significantly improved relative to a fixed rail voltage amplifier schemes.

The logic device 816, in conjunction with the DSP processor 822, implements a digital synthesis circuit such as a direct digital synthesizer control scheme to control the waveform shape, frequency, and/or amplitude of drive signals output by the generator 800. The logic device 816 implements a DDS control algorithm by recalling waveform samples stored in a dynamically updated lookup table (LUT), such as a RAM LUT, which are embedded in an FPGA. This control algorithm is particularly useful for ultrasonic applications in which an ultrasonic transducer, such as an ultrasonic transducer, is driven by a clean sinusoidal current at its resonant frequency. Because other frequencies may excite parasitic resonances, minimizing or reducing the total distortion of the motional branch current may correspondingly minimize or reduce undesirable resonance effects. Because the waveform shape of a drive signal output by the generator 800 is impacted by various sources of distortion present in the output drive circuit (e.g., the power transformer 806, the power amplifier 812), voltage and current feedback data based on the drive signal is input into an algorithm, such as an error control algorithm implemented by the DSP processor 822, which compensates for distortion by suitably pre-distorting or modifying the waveform samples stored in the LUT on a dynamic, ongoing basis (e.g., in real time). In one form, the amount or degree of pre-distortion applied to the LUT samples is based on the error between a computed motional branch current and a desired current waveform shape, with the error being determined on a sample-by-sample basis. In this way, the pre-distorted LUT samples, when processed through the drive circuit, results in a motional branch drive signal having the desired waveform shape (e.g., sinusoidal) for optimally driving the ultrasonic transducer. In such forms, the LUT waveform samples will therefore not represent the desired waveform shape of the drive signal, but rather the waveform shape that is required to ultimately produce the desired waveform shape of the motional branch drive signal when distortion effects are taken into account.

The non-isolated stage 804 further comprises a first ADC circuit 826 and a second ADC circuit 828 coupled to the output of the power transformer 806 via respective isolation transformers 830, 832 for respectively sampling the voltage and current of drive signals output by the generator 800. In certain forms, the ADC circuits 826, 828 are configured to sample at high speeds (e.g., 80 mega samples per second (MSPS)) to enable oversampling of the drive signals. In one form, for example, the sampling speed of the ADC circuits 826, 828 enable approximately 200x (depending on frequency) oversampling of the drive signals. In certain forms, the sampling operations of the ADC circuit 826, 828 are performed by a single ADC circuit receiving input voltage and current signals via a two-way multiplexer. The use of highspeed sampling in forms of the generator 800 enable, among other things, calculation of the complex current flowing through the motional branch (which is used in certain forms to implement DDS-based waveform shape control described above), accurate digital filtering of the sampled signals, and calculation of real power consumption with a high degree of precision. Voltage and current feedback data output by the ADC circuits 826, 828 is received and processed (e.g., first-in-first-out (FIFO) buffer, multiplexer) by the logic device 816 and stored in data memory for subsequent retrieval by, for example, the DSP processor 822. As noted above, voltage and current feedback data is used as input to an algorithm for pre-distorting or modifying LUT waveform samples on a dynamic and ongoing basis. This requires each stored voltage and current feedback data pair to be indexed based on, or otherwise associated with, a corresponding LUT sample that was output by the logic device 816 when the voltage and current feedback data pair was acquired. Synchronization of the LUT samples and the voltage and current feedback data in this manner contributes to the correct timing and stability of the pre-distortion algorithm.

The voltage and current feedback data is used to control the frequency and/or amplitude (e.g., current amplitude) of the drive signals. In one form, for example, voltage and current feedback data is used to determine impedance phase. The frequency of the drive signal may then be controlled to minimize or reduce the difference between the determined impedance phase and an impedance phase setpoint (e.g., 0°), thereby minimizing or reducing the effects of harmonic distortion and correspondingly enhancing impedance phase measurement accuracy. The determination of phase impedance and a frequency control signal is implemented in the DSP processor 822, for example, with the frequency control signal being supplied as input to a DDS control algorithm implemented by the logic device 816.

The current feedback data is monitored in order to maintain the current amplitude of the drive signal at a current amplitude setpoint. The current amplitude setpoint is specified directly or determined indirectly based on specified voltage amplitude and power setpoints. Control of the current amplitude is implemented by control algorithm, such as, for example, a proportional-integral-derivative (PID) control algorithm, in the DSP processor 822. Variables controlled by the control algorithm to suitably control the current amplitude of the drive signal include, for example, the scaling of the LUT waveform samples stored in the logic device 816 and/or the full-scale output voltage of the DAC circuit 818 (which supplies the input to the power amplifier 812) via a DAC circuit 834.

The non-isolated stage 804 further comprises a second processor 836 for providing, among other things user interface (UI) functionality. The UI processor 836 may comprise an Atmel AT91SAM9263 processor having an ARM 926EJ-S core, available from Atmel Corporation, San Jose, California, for example. Examples of UI functionality supported by the UI processor 836 include audible and visual user feedback, communication with peripheral devices (e.g., via a USB interface), communication with a foot switch, communication with an input device (e.g., a touch screen display) and communication with an output device (e.g., a speaker). The UI processor 836 communicates with the DSP processor 822 and the logic device 816 (e.g., via SPI buses). Although the UI processor 836 may primarily support UI functionality, it also coordinates with the DSP processor 822 to implement hazard mitigation in certain forms. For example, the UI processor 836 may be programmed to monitor various aspects of user input and/or other inputs (e.g., touch screen inputs, foot switch inputs, temperature sensor inputs) and disables the drive output of the generator 800 when an erroneous condition is detected.

Both the DSP processor 822 and the UI processor 836 determine and monitor the operating state of the generator 800. For the DSP processor 822, the operating state of the generator 800 dictates, for example, which control and/or diagnostic processes are implemented by the DSP processor 822. For the UI processor 836, the operating state of the generator 800 dictates, for example, which elements of a UI (e.g., display screens, sounds) are presented to a user. The respective DSP and UI processors 822, 836 independently maintain the current operating state of the generator 800 and recognize and evaluate possible transitions out of the current operating state. The DSP processor 822 functions as the master in this relationship and determine when transitions between operating states are to occur. The UI processor 836 is aware of valid transitions between operating states and confirms if a particular transition is appropriate. For example, when the DSP processor 822 instructs the UI processor 836 to transition to a specific state, the UI processor 836 verifies that requested transition is valid. In the event that a requested transition between states is determined to be invalid by the UI processor 836, the UI processor 836 causes the generator 800 to enter a failure mode.

The non-isolated stage 804 further comprises a controller 838 for monitoring input devices (e.g., a capacitive touch sensor used for turning the generator 800 on and off, a capacitive touch screen). The controller 838 comprises at least one processor and/or other controller device in communication with the UI processor 836. In one form, for example, the controller 838 comprises a processor (e.g., a Meg168 8-bit controller available from Atmel) configured to monitor user input provided via one or more capacitive touch sensors. The controller 838 comprises a touch screen controller (e.g., a QT5480 touch screen controller available from Atmel) to control and manage the acquisition of touch data from a capacitive touch screen.

When the generator 800 is in a "power off" state, the controller 838 continues to receive operating power (e.g., via a line from a power supply of the generator 800, such as the power supply 854 discussed below). In this way, the controller 838 continues to monitor an input device (e.g., a capacitive touch sensor located on a front panel of the generator 800) for turning the generator 800 on and off. When the generator 800 is in the power off state, the controller 838 wakes the power supply (e.g., enable operation of one or more DC/DC voltage converters 856 of the power supply 854) if activation of the "on/off" input device by a user is detected. The controller 838 therefore initiates a sequence for transitioning the generator 800 to a "power on" state. Conversely, the controller 838 may initiate a sequence for transitioning the generator 800 to the power off state if activation of the "on/off" input device is detected when the generator 800 is in the power on state. The controller 838 reports activation of the "on/off" input device to the UI processor 836, which in turn implements the necessary process sequence for transitioning the generator 800 to the power off state. The controller 838 has no independent ability for causing the removal of power from the generator 800 after its power on state has been established.

The controller 838 causes the generator 800 to provide audible or other sensory feedback for alerting the user that a power on or power off sequence has been initiated. Such an alert is provided at the beginning of a power on or power off sequence and prior to the commencement of other processes associated with the sequence.

The isolated stage 802 comprises an instrument interface circuit 840 to, for example, provide a communication interface between a control circuit of a surgical instrument (e.g., a control circuit comprising handpiece switches) and components of the non-isolated stage 804, such as, for example, the logic device 816, the DSP processor 822, and/or the UI processor 836. The instrument interface circuit 840 exchanges information with components of the non-isolated stage 804 via a communication link that maintains a suitable degree of electrical isolation between the isolated and non-isolated stages 802, 804, such as, for example, an IR-based communication link. Power is supplied to the instrument interface circuit 840 using, for example, a low-dropout voltage regulator powered by an isolation transformer driven from the non-isolated stage 804.

The instrument interface circuit 840 comprises a logic circuit 842 (e.g., logic circuit, programmable logic circuit, PGA, FPGA, PLD) in communication with a signal conditioning circuit 844. The signal conditioning circuit 844 is configured to receive a periodic signal from the logic circuit 842 (e.g., a 2 kHz square wave) to generate a bipolar interrogation signal having an identical frequency. The interrogation signal is generated, for example, using a bipolar current source fed by a differential amplifier. The interrogation signal is communicated to a surgical instrument control circuit (e.g., by using a conductive pair in a cable that connects the generator 800 to the surgical instrument) and monitored to determine a state or configuration of the control circuit. The control circuit comprises a number of switches, resistors, and/or diodes to modify one or more characteristics (e.g., amplitude, rectification) of the interrogation signal such that a state or configuration of the control circuit is uniquely discernable based on the one or more characteristics. In one form, for example, the signal conditioning circuit 844 comprises an ADC circuit for generating samples of a voltage signal appearing across inputs of the control circuit resulting from passage of interrogation signal therethrough. The logic circuit 842 (or a component of the non-isolated stage 804) then determines the state or configuration of the control circuit based on the ADC circuit samples.

The instrument interface circuit 840 comprises a first data circuit interface 846 to enable information exchange between the logic circuit 842 (or other element of the instrument interface circuit 840) and a first data circuit disposed in or otherwise associated with a surgical instrument. A first data circuit is disposed in a cable integrally attached to a surgical instrument handpiece or in an adaptor for interfacing a specific surgical instrument type or model with the generator 800. The first data circuit is implemented in any suitable manner and communicates with the generator according to any suitable protocol, including, for example, as described herein with respect to the first data circuit. The first data circuit comprise a non-volatile storage device, such as an EEPROM device. The first data circuit interface 846 is implemented separately from the logic circuit 842 and comprise suitable circuitry (e.g., discrete logic devices, a processor) to enable communication between the logic circuit 842 and the first data circuit. The first data circuit interface 846 is integral with the logic circuit 842.

The first data circuit stores information pertaining to the particular surgical instrument with which it is associated. Such information includes, for example, a model number, a serial number, a number of operations in which the surgical instrument has been used, and/or any other type of information. This information is read by the instrument interface circuit 840 (e.g., by the logic circuit 842), transferred to a component of the non-isolated stage 804 (e.g., to logic device 816, DSP processor 822, and/or UI processor 836) for presentation to a user via an output device and/or for controlling a function or operation of the generator 800. Additionally, any type of information is communicated to the first data circuit for storage therein via the first data circuit interface 846 (e.g., using the logic circuit 842). Such information comprises, for example, an updated number of operations in which the surgical instrument has been used and/or dates and/or times of its usage.

As discussed previously, a surgical instrument is detachable from a handpiece (e.g., the multifunction surgical instrument may be detachable from the handpiece) to promote instrument interchangeability and/or disposability. In such cases, conventional generators are limited in their ability to recognize particular instrument configurations being used and to optimize control and diagnostic processes accordingly. The addition of readable data circuits to surgical instruments to address this issue is problematic from a compatibility standpoint, however. For example, designing a surgical instrument to remain backwardly compatible with generators that lack the requisite data reading functionality may be impractical due to, for example, differing signal schemes, design complexity, and cost. Forms of instruments discussed herein address these concerns by using data circuits that are implemented in existing surgical instruments economically and with minimal design changes to preserve compatibility of the surgical instruments with current generator platforms.

Additionally, forms of the generator 800 enable communication with instrument-based data circuits. For example, the generator 800 is configured to communicate with a second data circuit contained in an instrument (e.g., the multifunction surgical instrument). In some forms, the second data circuit is implemented in a many similar to that of the first data circuit described herein. The instrument interface circuit 840 comprises a second data circuit interface 848 to enable this communication. The second data circuit interface 848 comprises a tri-state digital interface, although other interfaces may also be used. In certain forms, the second data circuit is generally any circuit for transmitting and/or receiving data. The second data circuit stores information pertaining to the particular surgical instrument with which it is associated. Such information includes, for example, a model number, a serial number, a number of operations in which the surgical instrument has been used, and/or any other type of information.

The second data circuit stores information about the electrical and/or ultrasonic properties of an associated ultrasonic transducer, end effector, or ultrasonic drive system. For example, the first data circuit indicates a burn-in frequency slope, as described herein. Additionally or alternatively, any type of information is communicated to second data circuit for storage therein via the second data circuit interface 848 (e.g., using the logic circuit 842). Such information comprises, for example, an updated number of operations in which the instrument has been used and/or dates and/or times of its usage. In certain forms, the second data circuit may transmit data acquired by one or more sensors (e.g., an instrument-based temperature sensor). In certain forms, the second data circuit receives data from the generator 800 and provide an indication to a user (e.g., a light emitting diode indication or other visible indication) based on the received data.

The second data circuit and the second data circuit interface 848 is configured such that communication between the logic circuit 842 and the second data circuit can be effected without the need to provide additional conductors for this purpose (e.g., dedicated conductors of a cable connecting a handpiece to the generator 800). Information is communicated to and from the second data circuit using a one-wire bus communication scheme implemented on existing cabling, such as one of the conductors used transmit interrogation signals from the signal conditioning circuit 844 to a control circuit in a handpiece. In this way, design changes or modifications to the surgical instrument that might otherwise be necessary are minimized or reduced. Moreover, because different types of communications implemented over a common physical channel can be frequency-band separated, the presence of a second data circuit is "invisible" to generators that do not have the requisite data reading functionality, thus enabling backward compatibility of the surgical instrument.

The isolated stage 802 comprises at least one blocking capacitor 850-1 connected to the drive signal output 810b to prevent passage of DC current to a patient. A single blocking capacitor is required to comply with medical regulations or standards, for example. While failure in single-capacitor designs is relatively uncommon, such failure nonetheless has negative consequences. A second blocking capacitor 850-2 is provided in series with the blocking capacitor 850-1, with current leakage from a point between the blocking capacitors 850-1, 850-2 being monitored by an ADC circuit 852 for sampling a voltage induced by leakage current. The samples are received by the logic circuit 842, for example. Based changes in the leakage current (as indicated by the voltage samples), the generator 800 determines when at least one of the blocking capacitors 850-1, 850-2 has failed, thus providing a benefit over single-capacitor designs having a single point of failure.

In certain forms, the non-isolated stage 804 comprises a power supply 854 for delivering DC power at a suitable voltage and current. The power supply comprises, for example, a 400 W power supply for delivering a 48 VDC system voltage. The power supply 854 further comprises one or more DC/DC voltage converters 856 for receiving the output of the power supply to generate DC outputs at the voltages and currents required by the various components of the generator 800. As discussed above in connection with the controller 838, one or more of the DC/DC voltage converters 856 may receive an input from the controller 838 when activation of the "on/off" input device by a user is detected by the controller 838 to enable operation of, or wake, the DC/DC voltage converters 856.

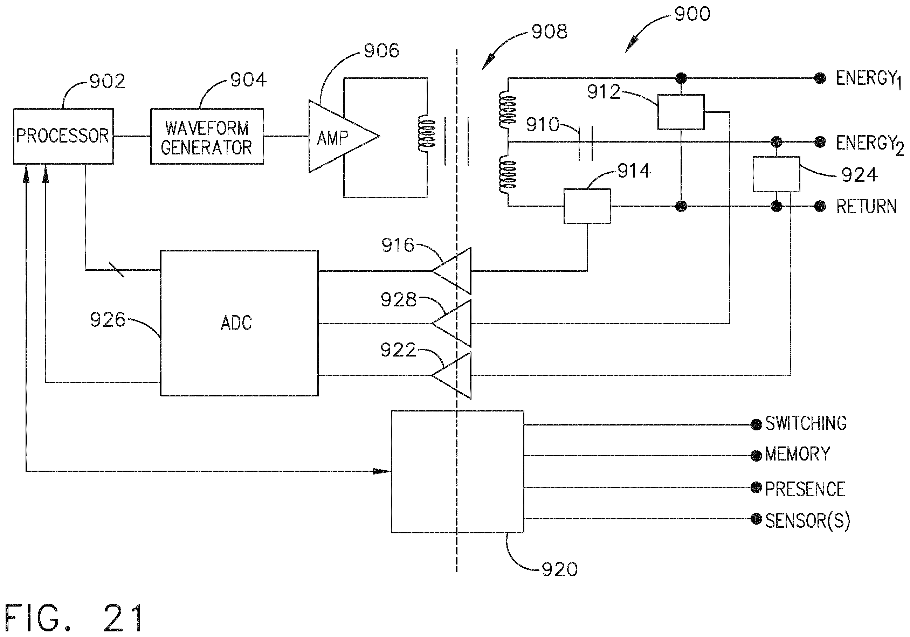

FIG. 21 illustrates an example of a generator 900, which is one form of the generator 800 (FIG. 20). The generator 900 is configured to deliver multiple energy modalities to a surgical instrument. The generator 900 provides RF and ultrasonic signals for delivering energy to a surgical instrument either independently or simultaneously. The RF and ultrasonic signals are provided alone or in combination and are provided simultaneously. As noted above, at least one generator output can deliver multiple energy modalities (e.g., ultrasonic, bipolar or monopolar RF, irreversible and/or reversible electroporation, and/or microwave energy, among others) through a single port, and these signals can be delivered separately or simultaneously to the end effector to treat tissue.

The generator 900 comprises a processor 902 coupled to a waveform generator 904. The processor 902 and waveform generator 904 are configured to generate a variety of signal waveforms based on information stored in a memory coupled to the processor 902, not shown for clarity of disclosure. The digital information associated with a waveform is provided to the waveform generator 904 which includes one or more DAC circuits to convert the digital input into an analog output. The analog output is fed to an amplifier 1106 for signal conditioning and amplification. The conditioned and amplified output of the amplifier 906 is coupled to a power transformer 908. The signals are coupled across the power transformer 908 to the secondary side, which is in the patient isolation side. A first signal of a first energy modality is provided to the surgical instrument between the terminals labeled ENERGY1 and RETURN. A second signal of a second energy modality is coupled across a capacitor 910 and is provided to the surgical instrument between the terminals labeled ENERGY2 and RETURN. It will be appreciated that more than two energy modalities may be output and thus the subscript "n" may be used to designate that up to n ENERGYn terminals may be provided, where n is a positive integer greater than 1. It also will be appreciated that up to "n" return paths RETURNn may be provided without departing from the scope of the present disclosure.

A first voltage sensing circuit 912 is coupled across the terminals labeled ENERGY1 and the RETURN path to measure the output voltage therebetween. A second voltage sensing circuit 924 is coupled across the terminals labeled ENERGY2 and the RETURN path to measure the output voltage therebetween. A current sensing circuit 914 is disposed in series with the RETURN leg of the secondary side of the power transformer 908 as shown to measure the output current for either energy modality. If different return paths are provided for each energy modality, then a separate current sensing circuit should be provided in each return leg. The outputs of the first and second voltage sensing circuits 912, 924 are provided to respective isolation transformers 916, 922 and the output of the current sensing circuit 914 is provided to another isolation transformer 918. The outputs of the isolation transformers 916, 928, 922 in the on the primary side of the power transformer 908 (non-patient isolated side) are provided to a one or more ADC circuit 926. The digitized output of the ADC circuit 926 is provided to the processor 902 for further processing and computation. The output voltages and output current feedback information can be employed to adjust the output voltage and current provided to the surgical instrument and to compute output impedance, among other parameters. Input/output communications between the processor 902 and patient isolated circuits is provided through an interface circuit 920. Sensors also may be in electrical communication with the processor 902 by way of the interface circuit 920.

The impedance is determined by the processor 902 by dividing the output of either the first voltage sensing circuit 912 coupled across the terminals labeled ENERGY1/RETURN or the second voltage sensing circuit 924 coupled across the terminals labeled ENERGY2/RETURN by the output of the current sensing circuit 914 disposed in series with the RETURN leg of the secondary side of the power transformer 908. The outputs of the first and second voltage sensing circuits 912, 924 are provided to separate isolations transformers 916, 922 and the output of the current sensing circuit 914 is provided to another isolation transformer 916. The digitized voltage and current sensing measurements from the ADC circuit 926 are provided the processor 902 for computing impedance. As an example, the first energy modality ENERGY1 is ultrasonic energy and the second energy modality ENERGY2 is RF energy. Nevertheless, in addition to ultrasonic and bipolar or monopolar RF energy modalities, other energy modalities include irreversible and/or reversible electroporation and/or microwave energy, among others. Also, although the example illustrated in FIG. 21 shows a single return path RETURN is provided for two or more energy modalities, multiple return paths RETURNn may be provided for each energy modality ENERGYn. Thus, as described herein, the ultrasonic transducer impedance is measured by dividing the output of the first voltage sensing circuit 912 by the current sensing circuit 914 and the tissue impedance is measured by dividing the output of the second voltage sensing circuit 924 by the current sensing circuit 914.

As shown in FIG. 21, the generator 900 comprising at least one output port can include a power transformer 908 with a single output and with multiple taps to provide power in the form of one or more energy modalities, such as ultrasonic, bipolar or monopolar RF, irreversible and/or reversible electroporation, and/or microwave energy, among others, for example, to the end effector depending on the type of treatment of tissue being performed. For example, the generator 900 can deliver energy with higher voltage and lower current to drive an ultrasonic transducer, with lower voltage and higher current to drive RF electrodes for sealing tissue, or with a coagulation waveform for spot coagulation using either monopolar or bipolar RF electrosurgical electrodes. The output waveform from the generator 900 can be steered, switched, or filtered to provide the frequency to the end effector of the surgical instrument. The connection of an ultrasonic transducer to the generator 900 output would be preferably located between the output labeled ENERGY1 and RETURN as shown in FIG. 21. In one example, a connection of RF bipolar electrodes to the generator 900 output would be preferably located between the output labeled ENERGY2 and RETURN. In the case of monopolar output, the preferred connections would be active electrode (e.g., pencil or other probe) to the ENERGY2 output and a suitable return pad connected to the RETURN output.

Additional details are disclosed in U.S. Patent Application Publication No. 2017/0086914, titled TECHNIQUES FOR OPERATING GENERATOR FOR DIGITALLY GENERATING ELECTRICAL SIGNAL WAVEFORMS AND SURGICAL INSTRUMENTS, which published on March 30, 2017.

As used throughout this description, the term "wireless" and its derivatives is used to describe circuits, devices, systems, methods, techniques, communications channels, etc., that communicate data through the use of modulated electromagnetic radiation through a non-solid medium. The term does not imply that the associated devices do not contain any wires, although in some aspects they might not. The communication module implements any of a number of wireless or wired communication standards or protocols, including but not limited to Wi-Fi (IEEE 802.11 family), WiMAX (IEEE 802.16 family), IEEE 802.20, long term evolution (LTE), Ev-DO, HSPA+, HSDPA+, HSUPA+, EDGE, GSM, GPRS, CDMA, TDMA, DECT, Bluetooth, Ethernet derivatives thereof, as well as any other wireless and wired protocols that are designated as 3G, 4G, 5G, and beyond. The computing module includes a plurality of communication modules. A first communication module is dedicated to shorter range wireless communications such as Wi-Fi and Bluetooth and a second communication module is dedicated to longer range wireless communications such as GPS, EDGE, GPRS, CDMA, Wi-MAX, LTE, Ev-DO, and others.

As used herein a processor or processing unit is an electronic circuit which performs operations on some external data source, usually memory or some other data stream. The term is used herein to refer to the central processor (central processing unit) in a system or computer systems (especially systems on a chip (SoCs)) that combine a number of specialized "processors."

As used herein, a system on a chip or system on chip (SoC or SOC) is an integrated circuit (also known as an "IC" or "chip") that integrates all components of a computer or other electronic systems. It contains digital, analog, mixed-signal, and often radio-frequency functions-all on a single substrate. A SoC integrates a microcontroller (or microprocessor) with advanced peripherals like graphics processing unit (GPU), Wi-Fi module, or coprocessor. A SoC may contain built-in memory.

As used herein, a microcontroller or controller is a system that integrates a microprocessor with peripheral circuits and memory. A microcontroller (or MCU for microcontroller unit) is implemented as a small computer on a single integrated circuit. It is similar to a SoC; an SoC includes a microcontroller as one of its components. A microcontroller contains one or more core processing units (CPUs) along with memory and programmable input/output peripherals. Program memory in the form of Ferroelectric RAM, NOR flash or OTP ROM is also often included on chip, as well as a small amount of RAM. Microcontrollers are employed for embedded applications, in contrast to the microprocessors used in personal computers or other general purpose applications consisting of various discrete chips.

As used herein, the term controller or microcontroller is a stand-alone IC or chip device that interfaces with a peripheral device. This is a link between two parts of a computer or a controller on an external device that manages the operation of (and connection with) that device.

Any of the processors or microcontrollers described herein, are implemented by any single core or multicore processor such as those known under the trade name ARM Cortex by Texas Instruments. The processor may be an LM4F230H5QR ARM Cortex-M4F Processor Core, available from Texas Instruments comprising on-chip memory of 256 KB single-cycle flash memory, or other non-volatile memory, up to 40 MHz, a prefetch buffer to improve performance above 40 MHz, a 32 KB single-cycle serial random access memory (SRAM), internal read-only memory (ROM) loaded with Stellaris-Ware® software, 2 KB electrically erasable programmable read-only memory (EEPROM), one or more pulse width modulation (PWM) modules, one or more quadrature encoder inputs (QEI) analog, one or more 12-bit Analog-to-Digital Converters (ADC) with 12 analog input channels, details of which are available for the product datasheet.

The processor comprises a safety controller comprising two controller-based families such as TMS570 and RM4x known under the trade name Hercules ARM Cortex R4, also by Texas Instruments. The safety controller is configured specifically for IEC 61508 and ISO 26262 safety critical applications, among others, to provide advanced integrated safety features while delivering scalable performance, connectivity, and memory options.

Modular devices include the modules (as described in connection with FIGS. 3 and 9, for example) that are receivable within a surgical hub and the surgical devices or instruments that can be connected to the various modules in order to connect or pair with the corresponding surgical hub. The modular devices include, for example, intelligent surgical instruments, medical imaging devices, suction/irrigation devices, smoke evacuators, energy generators, ventilators, insufflators, and displays. The modular devices described herein are controlled by control algorithms. The control algorithms can be executed on the modular device itself, on the surgical hub to which the particular modular device is paired, or on both the modular device and the surgical hub (e.g., via a distributed computing architecture). In some exemplifications, the modular devices' control algorithms control the devices based on data sensed by the modular device itself (i.e., by sensors in, on, or connected to the modular device). This data is related to the patient being operated on (e.g., tissue properties or insufflation pressure) or the modular device itself (e.g., the rate at which a knife is being advanced, motor current, or energy levels). For example, a control algorithm for a surgical stapling and cutting instrument can control the rate at which the instrument's motor drives its knife through tissue according to resistance encountered by the knife as it advances.

Visualization System

During a surgical procedure, a surgeon is required to manipulate tissues to effect a desired medical outcome. The actions of the surgeon are limited by what is visually observable in the surgical site. Thus, the surgeon may not be aware, for example, of the disposition of vascular structures that underlie the tissues being manipulated during the procedure. Since the surgeon is unable to visualize the vasculature beneath a surgical site, the surgeon may accidentally sever one or more critical blood vessels during the procedure. The solution is a surgical visualization system that can acquire imaging data of the surgical site for presentation to a surgeon, in which the presentation can include information related to the presence and depth of vascular structures located beneath the surface of a surgical site.

The surgical hub 106 incorporates a visualization system according to the invention to acquire imaging data during a surgical procedure. The specific visualization system 108 includes one or more illumination sources and one or more light sensors. The one or more illumination sources and one or more light sensors are incorporated together into a single device or may comprise one or more separate devices. The one or more illumination sources are directed to illuminate portions of the surgical field. The one or more light sensors receive light reflected or refracted from the surgical field including light reflected or refracted from tissue and/or surgical instruments. The following description includes all of the hardware and software processing techniques disclosed above.

The visualization system 108 may be integrated into a surgical system 100 as disclosed above and depicted in FIGS. 1 and 2. In addition to the visualization system 108, the surgical system 100 includes one or more handheld intelligent instruments 112, a multi-functional robotic system 110, one or more visualization systems 108, and a centralized surgical hub system 106, among other components. The centralized surgical hub system 106 controls several functions a disclosed above and also depicted in FIG 3. In one non-limiting example, such functions include supplying and controlling power to any number of powered surgical devices. In another non-limiting example, such functions include controlling fluid supplied to and evacuated from the surgical site. The centralized surgical hub system 106 also is configured to manage and analyze data received from any of the surgical system components as well as communicate data and other information among and between the components of the surgical system. The centralized surgical hub system 106 also is in data communication with a cloud computing system 104 as disclosed above and depicted, for example, in FIG. 1.

Imaging data generated by the visualization system 108 is analyzed by on-board computational components of the visualization system 108, and analysis results are communicated to the centralized surgical hub 106. Alternatively , the imaging data generated by the visualization system 108 is communicated directly to the centralized surgical hub 106 where the data is analyzed by computational components in the hub system 106. The centralized surgical hub 106 communicates the image analysis results to any one or more of the other components of the surgical system. The centralized surgical hub communicates the image data and/or the image analysis results to the cloud computing system 104.

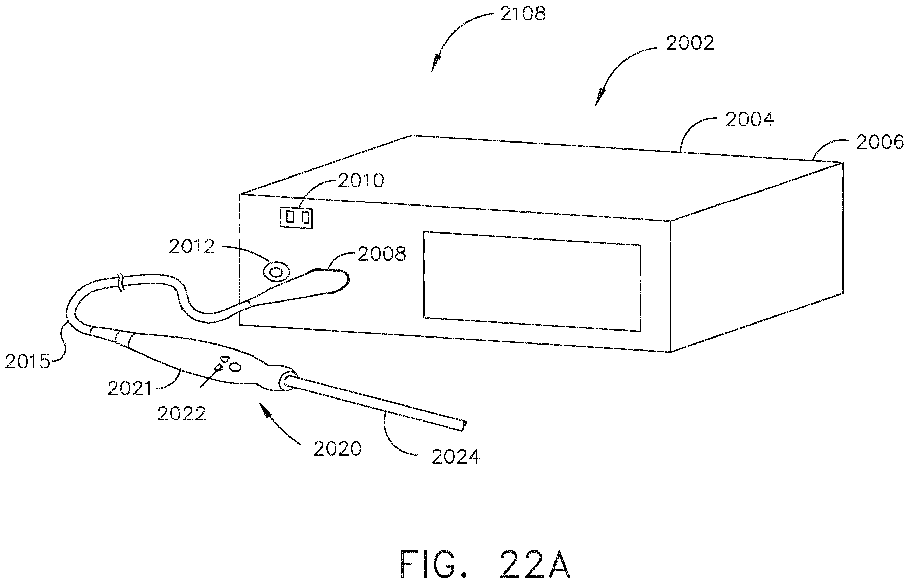

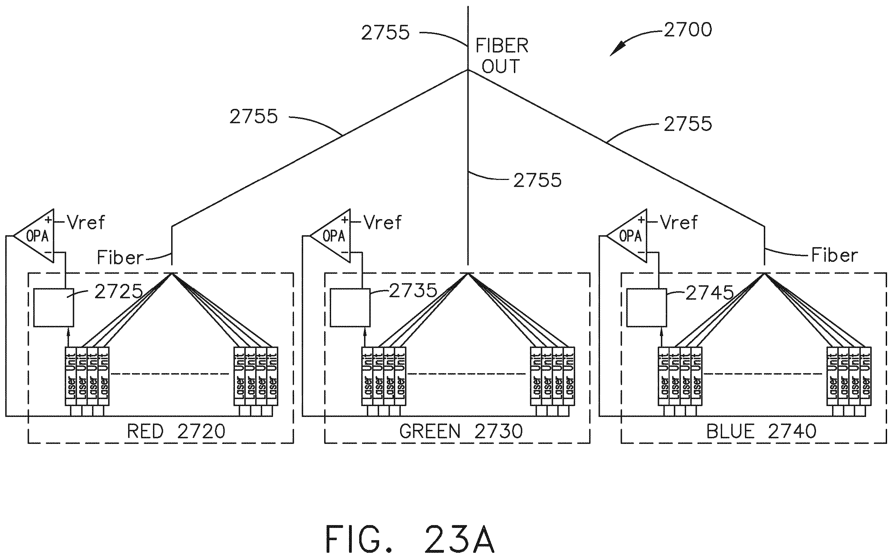

FIGS. 22A-D and FIGS. 23A-F depict one example of a visualization system 2108 that may be incorporated into a surgical system according to the invention. The visualization system 2108 includes an imaging control unit 2002 and a hand unit 2020. The imaging control unit 2002 includes one or more illumination sources, a power supply for the one or more illumination sources, one or more types of data communication interfaces (including USB, Ethernet, or wireless interfaces 2004), and one or more a video outputs 2006. The imaging control unit 2002 further includes an interface, such as a USB interface 2010, configured to transmit integrated video and image capture data to a USB enabled device. The imaging control unit 2002 also includes one or more computational components including, without limitation, a processor unit, a transitory memory unit, a non-transitory memory unit, an image processing unit, a bus structure to form data links among the computational components, and any interface (e.g. input and/or output) devices necessary to receive information from and transmit information to components not included in the imaging control unit. The non-transitory memory further contains instructions that when executed by the processor unit, performs any number of manipulations of data that may be received from the hand unit 2020 and/or computational devices not included in the imaging control unit.

The illumination sources includes a white light source 2012 and one or more laser light sources. The imaging control unit 2002 includes one or more optical and/or electrical interfaces for optical and/or electrical communication with the hand unit 2020. The one or more laser light sources include, any one or more of a red laser light source, a green laser light source, a blue laser light source, an infra red laser light source, and an ultraviolet laser light source. The red laser light source has source illumination having a peak wavelength that ranges between 635 nm and 660 nm, inclusive. A red laser peak wavelength includes about 635 nm, about 640 nm, about 645 nm, about 650 nm, about 655 nm, about 660 nm, or any value or range of values therebetween. The green laser light source has source illumination having a peak wavelength that ranges between 520 nm and 532 nm, inclusive. A green laser peak wavelength includes about 520 nm, about 522 nm, about 524 nm, about 526 nm, about 528 nm, about 530 nm, about 532 nm, or any value or range of values therebetween The blue laser light source has source illumination having a peak wavelength that ranges between 405 nm and 445 nm, inclusive. A blue laser peak wavelength includeS about 405 nm, about 410 nm, about 415 nm, about 420 nm, about 425 nm, about 430 nm, about 435 nm, about 440 nm, about 445 nm, or any value or range of values therebetween. The infra red laser light source source illumination has a peak wavelength that ranges between 750 nm and 3000 nm, inclusive. Infra red laser peak wavelength includes about 750 nm, about 1000 nm, about 1250 nm, about 1500 nm, about 1750 nm, about 2000 nm, about 2250 nm, about 2500 nm, about 2750 nm, 3000 nm, or any value or range of values therebetween. The ultraviolet laser light source has source illumination having a peak wavelength that ranges between 200 nm and 360 nm, inclusive. Ultraviolet laser peak wavelength includes about 200 nm, about 220 nm, about 240 nm, about 260 nm, about 280 nm, about 300 nm, about 320 nm, about 340 nm, about 360 nm, or any value or range of values therebetween.

The hand unit 2020 includes a body 2021, a camera scope cable 2015 attached to the body 2021, and an elongated camera probe 2024. The body 2021 of the hand unit 2020 includes hand unit control buttons 2022 or other controls to permit a health professional using the hand unit 2020 to control the operations of the hand unit 2020 or other components of the imaging control unit 2002, including, for example, the light sources. The camera scope cable 2015 includes one or more electrical conductors and one or more optical fibers. The camera scope cable 2015 terminates with a camera head connector 2008 at a proximal end in which the camera head connector 2008 is configured to mate with the one or more optical and/or electrical interfaces of the imaging control unit 2002. The electrical conductors supply power to the hand unit 2020, including the body 2021 and the elongated camera probe 2024, and/or to any electrical components internal to the hand unit 2020 including the body 2021 and/or elongated camera probe 2024. The electrical conductors also serve to provide bi-directional data communication between any one or more components the hand unit 2020 and the imaging control unit 2002. The one or more optical fibers conduct illumination from the one or more illumination sources in the imaging control unit 2002 through the hand unit body 2021 and to a distal end of the elongated camera probe 2024. The one or more optical fibers also conduct light reflected or refracted from the surgical site to one or more optical sensors disposed in the elongated camera probe 2024, the hand unit body 2021, and/or the imaging control unit 2002.

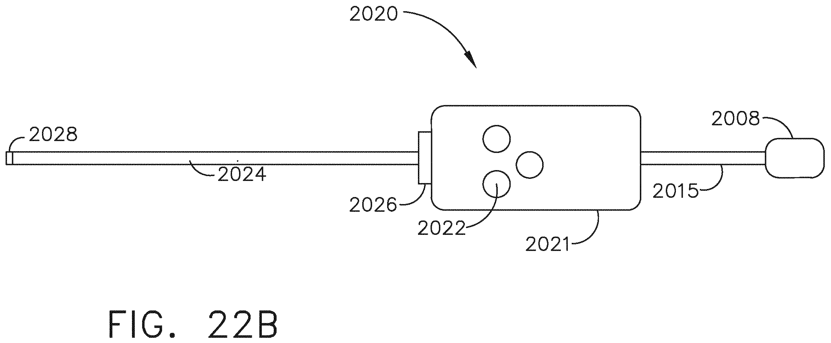

FIG. 22B (a top plan view) depicts in more detail some aspects of a hand unit 2020 of the visualization system 2108. The hand unit body 2021 is constructed of a plastic material. The hand unit control buttons 2022 or other controls has a rubber overmolding to protect the controls while permitting them to be manipulated by the surgeon. The camera scope cable 2015 has optical fibers integrated with electrical conductors, and the camera scope cable 2015 has a protective and flexible overcoating such as PVC. The camera scope cable 2015 is about 10 ft. long to permit ease of use during a surgical procedure. The length of the camera scope cable 2015 ranges from about 5 ft. to about 15 ft. A length of the camera scope cable 2015 may be about 5 ft., about 6 ft., about 7 ft., about 8 ft., about 9 ft., about 10 ft., about 11 ft., about 12 ft., about 13 ft., about 14 ft., about 15 ft., or any length or range of lengths therebetween. The elongated camera probe 2024 is fabricated from a rigid material such as stainless steel. The elongated camera probe 2024 is joined with the hand unit body 2021 via a rotatable collar 2026. The rotatable collar 2026 permits the elongated camera probe 2024 to be rotated with respect to the hand unit body 2021. The elongated camera probe 2024 terminates at a distal end with a plastic window 2028 sealed with epoxy.

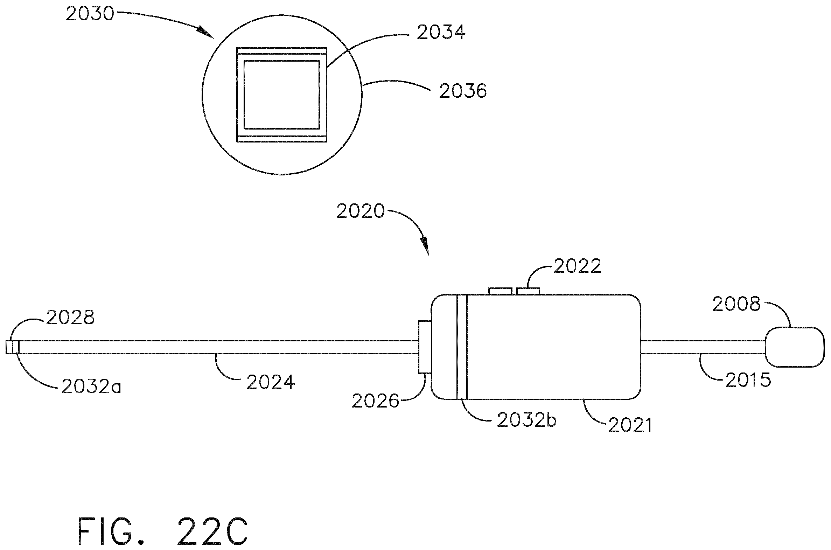

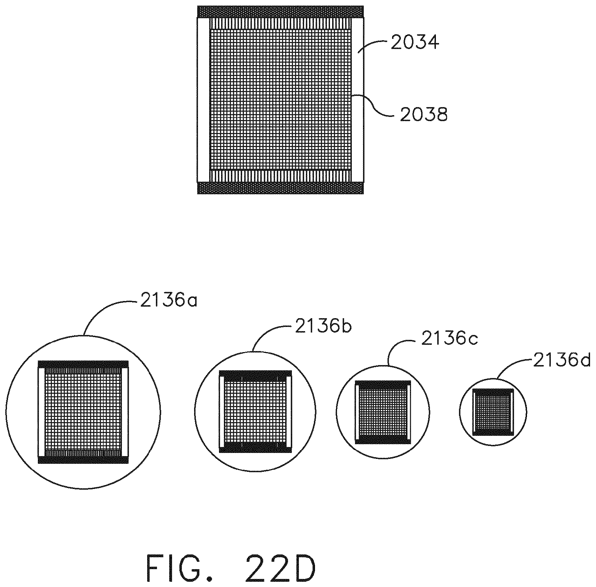

The side plan view of the hand unit, depicted in FIG. 22C illustrates that a light or image sensor 2030 is disposed at a distal end 2032a of the elongated camera probe or within the hand unit body 2032b. The light or image sensor 2030 is disposed with additional optical elements in the imaging control unit 2002. FIG. 22C further depicts an example of a light sensor 2030 comprising a CMOS image sensor 2034 disposed within a mount 2036 having a radius of about 4 mm. FIG. 22D illustrates aspects of the CMOS image sensor 2034, depicting the active area 2038 of the image sensor. Although the CMOS image sensor in FIG. 22C is depicted to be disposed within a mount 2036 having a radius of about 4 mm, it is recognized that such a sensor and mount combination is of any useful size to be disposed within the elongated camera probe 2024, the hand unit body 2021, or in the image control unit 2002. Alternative mounts include a 5.5 mm mount 2136a, a 4 mm mount 2136b, a 2.7 mm mount 2136c, and a 2 mm mount 2136d. It may be recognized that the image sensor also comprises a CCD image sensor. The CMOS or CCD sensor comprises an array of individual light sensing elements (pixels).

FIGS. 23A-23F depict illumination sources and their control that may be incorporated into the visualization system 2108.

FIG. 23A illustrates a laser illumination system having a plurality of laser bundles emitting a plurality of wavelengths of electromagnetic energy. As can be seen in the figure, the illumination system 2700 comprises a red laser bundle 2720, a green laser bundle 2730, and a blue laser bundle 2740 that are all optically coupled together though fiber optics 2755. As can be seen in the figure, each of the laser bundles has a corresponding light sensing element or electromagnetic sensor 2725, 2735, 2745 respectively, for sensing the output of the specific laser bundle or wavelength.

Additional disclosures regarding the laser illumination system depicted in FIG. 23A for use in a surgical visualization system 2108 may be found in U.S. Patent Application Publication No. 2014/0268860, titled CONTROLLING THE INTEGRAL LIGHT ENERGY OF A LASER PULSE filed on March 15, 2014, which issued on October 3, 2017 as U.S. Patent No. 9,777,913.

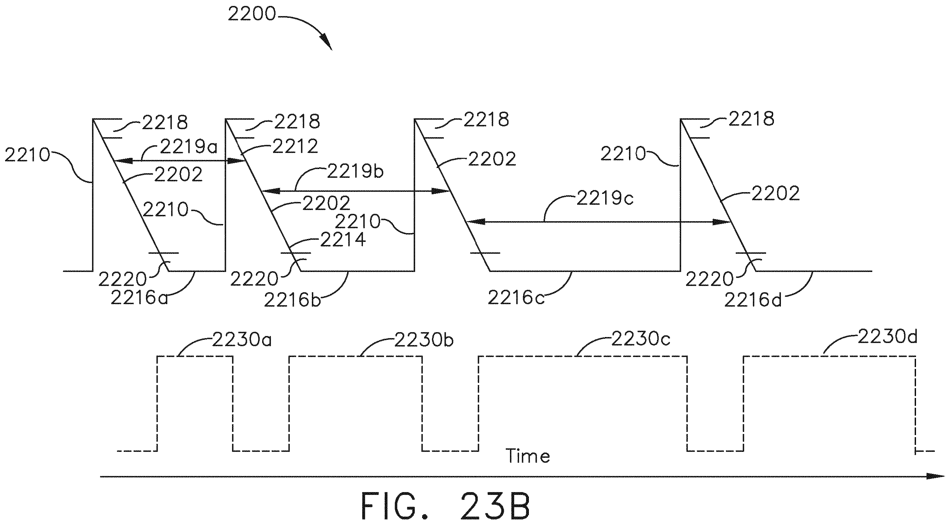

FIG. 23B illustrates the operational cycles of a sensor used in rolling readout mode. It will be appreciated that the x direction corresponds to time and the diagonal lines 2202 indicate the activity of an internal pointer that reads out each frame of data, one line at time. The same pointer is responsible for resetting each row of pixels for the next exposure period. The net integration time for each row 2219a-c is equivalent, but they are staggered in time with respect to one another due to the rolling reset and read process. Therefore, for any scenario in which adjacent frames are required to represent different constitutions of light, the only option for having each row be consistent is to pulse the light between the readout cycles 2230a-c. More specifically, the maximum available period corresponds to the sum of the blanking time plus any time during which optical black or optically blind (OB) rows (2218, 2220) are serviced at the start or end of the frame.

FIG. 23B illustrates the operational cycles of a sensor used in rolling readout mode or during the sensor readout 2200. The frame readout starts at and may be represented by vertical line 2210. The read out period is represented by the diagonal or slanted line 2202. The sensor is read out on a row by row basis, the top of the downwards slanted edge being the sensor top row 2212 and the bottom of the downwards slanted edge being the sensor bottom row 2214. The time between the last row readout and the next readout cycle is called the blanking time 2216a-d. It is understood that the blanking time 2216a-d is the same between success readout cycles or it may differ between success readout cycles. It should be noted that some of the sensor pixel rows might be covered with a light shield (e.g., a metal coating or any other substantially black layer of another material type). These covered pixel rows are referred to as optical black rows 2218 and 2220. Optical black rows 2218 and 2220 are used as input for correction algorithms.

As shown in FIG. 23B, these optical black rows 2218 and 2220 are located on the top of the pixel array or at the bottom of the pixel array or at the top and the bottom of the pixel array. In some aspects, it is desirable to control the amount of electromagnetic radiation, e.g., light, that is exposed to a pixel, thereby integrated or accumulated by the pixel. It will be appreciated that photons are elementary particles of electromagnetic radiation. Photons are integrated, absorbed, or accumulated by each pixel and converted into an electrical charge or current. An electronic shutter or rolling shutter is used to start the integration time (2219a-c) by resetting the pixel. The light will then integrate until the next readout phase. The position of the electronic shutter can be moved between two readout cycles 2202 in order to control the pixel saturation for a given amount of light. Alternatively, when lacking an electronic shutter, the integration time 2219a-c of the incoming light starts during a first readout cycle 2202 and ends at the next readout cycle 2202, which also defines the start of the next integration. The amount of light accumulated by each pixel is controlled by a time during which light is pulsed 2230a-d during the blanking times 2216a-d. This ensures that all rows see the same light issued from the same light pulse 2230a-c. In other words, each row will start its integration in a first dark environment 2231, which is at the optical black back row 2220 of read out frame (m) for a maximum light pulse width, and will then receive a light strobe and will end its integration in a second dark environment 2232, which may be at the optical black front row 2218 of the next succeeding read out frame (m + 1) for a maximum light pulse width. Thus, the image generated from the light pulse 2230a-c will be solely available during frame (m+1) readout without any interference with frames (m) and (m+2).

It should be noted that the condition to have a light pulse 2230a-c to be read out only in one frame and not interfere with neighboring frames is to have the given light pulse 2230a-c firing during the blanking time 2216. Because the optical black rows 2218, 2220 are insensitive to light, the optical black back rows 2220 time of frame (m) and the optical black front rows 2218 time of frame (m+1) can be added to the blanking time 2216 to determine the maximum range of the firing time of the light pulse 2230.

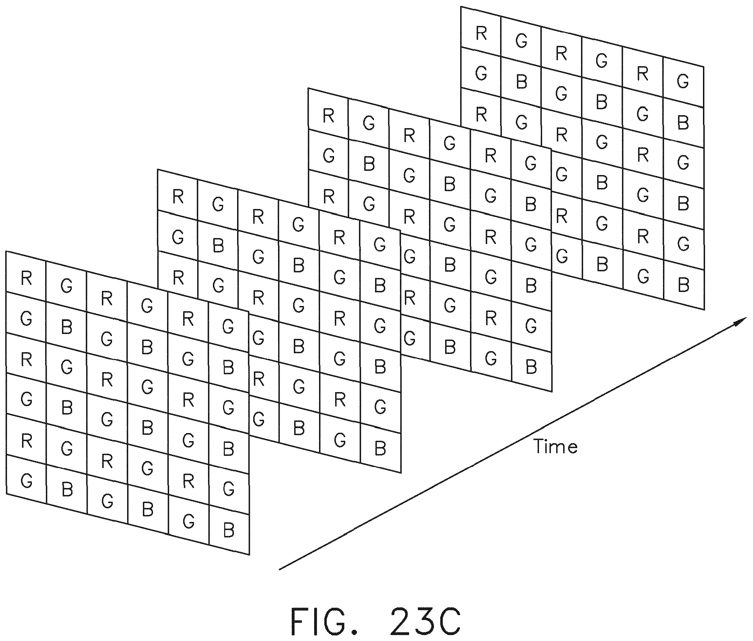

FIG. 23B depicts an example of a timing diagram for sequential frame captures by a conventional CMOS sensor. Such a CMOS sensor incorporates a Bayer pattern of color filters, as depicted in FIG. 23C. It is recognized that the Bayer pattern provides for greater luminance detail than chrominance. It is further recognized that the sensor has a reduced spatial resolution since a total of 4 adjacent pixels are required to produce the color information for the aggregate spatial portion of the image. In an alternative approach, the color image is constructed by rapidly strobing the visualized area at high speed with a variety of optical sources (either laser or light-emitting diodes) having different central optical wavelengths.

The optical strobing system is under the control of the camera system, and includes a specially designed CMOS sensor with high speed readout. The principal benefit is that the sensor can accomplish the same spatial resolution with significantly fewer pixels compared with conventional Bayer or 3-sensor cameras. Therefore, the physical space occupied by the pixel array is reduced. The actual pulse periods (2230a-c) differ within the repeating pattern, as illustrated in FIG. 23B. This is useful for, e.g., apportioning greater time to the components that require the greater light energy or those having the weaker sources. As long as the average captured frame rate is an integer multiple of the requisite final system frame rate, the data is simply buffered in the signal processing chain as appropriate.

The facility to reduce the CMOS sensor chip-area to the extent allowed by combining all of these methods is particularly attractive for small diameter (~3-10 mm) endoscopy. In particular, it allows for endoscope designs in which the sensor is located in the space-constrained distal end, thereby greatly reducing the complexity and cost of the optical section, while providing high definition video. A consequence of this approach is that to reconstruct each final, full color image, requires that data be fused from three separate snapshots in time. Any motion within the scene, relative to the optical frame of reference of the endoscope, will generally degrade the perceived resolution, since the edges of objects appear at slightly different locations within each captured component. In this disclosure, a means of diminishing this issue is described which exploits the fact that spatial resolution is much more important for luminance information, than for chrominance.

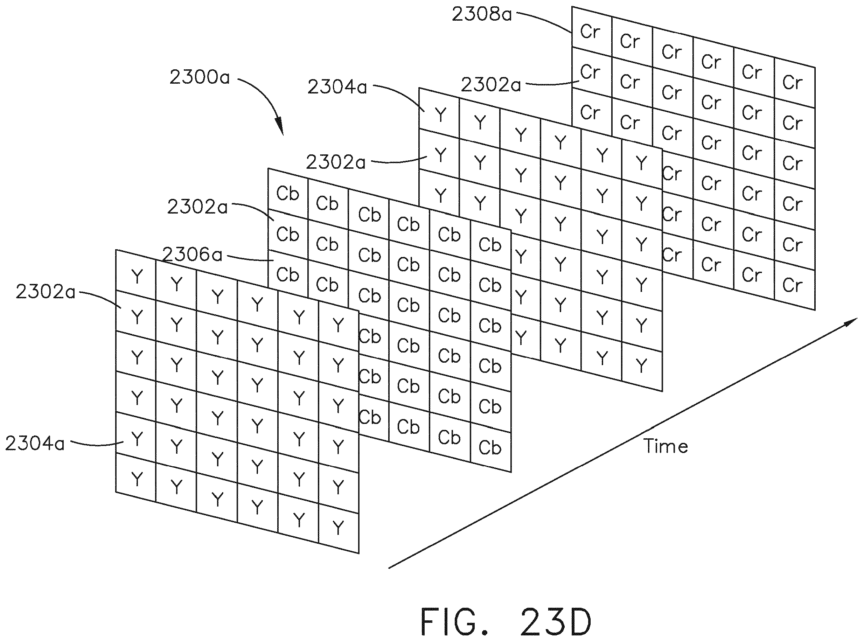

The basis of the approach is that, instead of firing monochromatic light during each frame, combinations of the three wavelengths are used to provide all of the luminance information within a single image. The chrominance information is derived from separate frames with, e.g., a repeating pattern such as Y-Cb-Y-Cr (FIG. 23D). While it is possible to provide pure luminance data by a shrewd choice of pulse ratios, the same is not true of chrominance.

As illustrated in FIG. 23D, an endoscopic system 2300a comprises a pixel array 2302a having uniform pixels and the system 2300a may be operated to receive Y (luminance pulse) 2304a, Cb (ChromaBlue) 2306a and Cr (ChromaRed) 2308a pulses.

To complete a full color image requires that the two components of chrominance also be provided. However, the same algorithm that was applied for luminance cannot be directly applied for chrominance images since it is signed, as reflected in the fact that some of the RGB coefficients are negative. The solution to this is to add a degree of luminance of sufficient magnitude that all of the final pulse energies become positive. As long as the color fusion process in the ISP is aware of the composition of the chrominance frames, they can be decoded by subtracting the appropriate amount of luminance from a neighboring frame. The pulse energy proportions are given by:

$$Y = 0.183 \cdot R + 0.614 \cdot G + 0.062 \cdot B$$

$$Cb = \lambda \cdot Y - 0.101 \cdot R - 0.339 \cdot G + 0.439 \cdot B$$

$$Cr = \delta \cdot Y + 0.439 \cdot R - 0.399 \cdot G - 0.040 \cdot B$$

where

$$\lambda \geq 0.399/0.614 = 0.552$$

$$\delta \geq 0.399/0.614 = 0.650$$

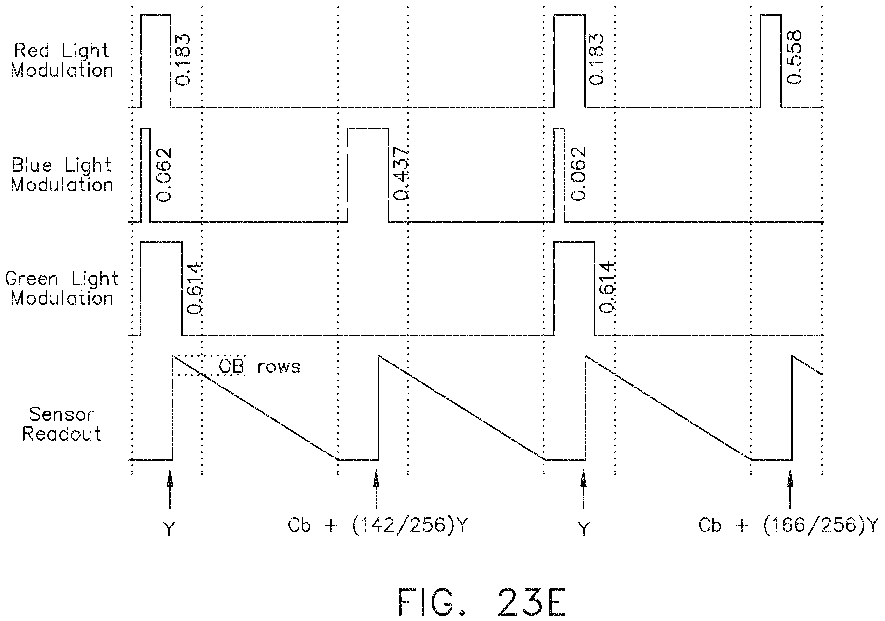

It turns out that if the $\lambda$ factor is equal to 0.552; both the red and the green components are exactly cancelled, in which case the Cb information can be provided with pure blue light. Similarly, setting $\delta = 0.650$ cancels out the blue and green components for Cr which becomes pure red. This particular example is illustrated in FIG. 23E, which also depicts $\lambda$ and $\delta$ as integer multiples of $1/2^8$. This is a convenient approximation for the digital frame reconstruction.

In the case of the Y-Cb-Y-Cr pulsing scheme, the image data is already in the YCbCr space following the color fusion. Therefore, in this case it makes sense to perform luminance and chrominance based operations up front, before converting back to linear RGB to perform the color correction etc.

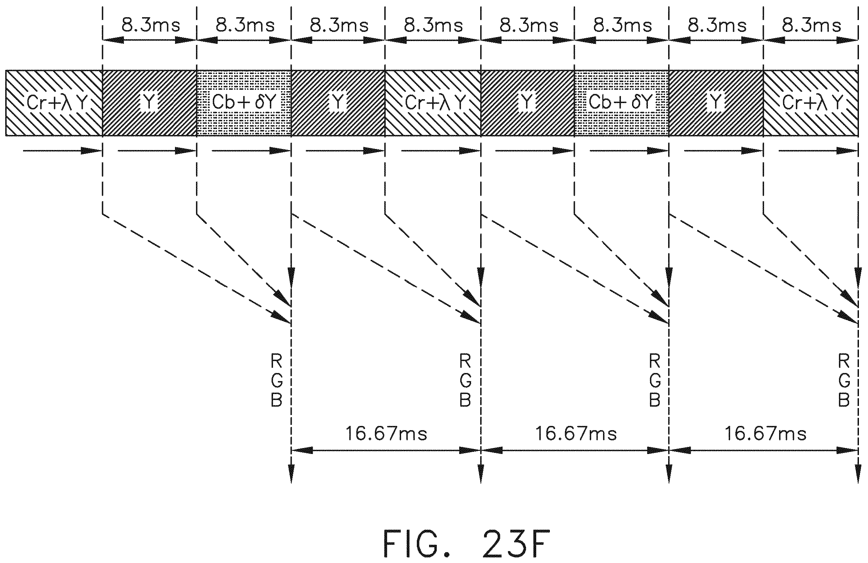

The color fusion process is more straightforward than de-mosaic, which is necessitated by the Bayer pattern (see FIG. 23C), since there is no spatial interpolation. It does require buffering of frames though in order to have all of the necessary information available for each pixel. Data for the Y-Cb-Y-Cr pattern is pipelined to yield one full color image per two raw captured images. This is accomplished by using each chrominance sample twice. In FIG. 23F the specific example of a 120 Hz frame capture rate providing 60 Hz final video is depicted.

Additional disclosures regarding the control of the laser components of an illumination system as depicted in FIGS. 23B - 23F for use in a surgical visualization system 108 may be found in U.S. Patent Application Publication No. 2014/0160318, titled YCBCR PULSED ILLUMINATION SCHEME IN A LIGHT DEFICIENT ENVIRONMENT, filed on July 26, 2013, which issued on December 6, 2016 as U.S. Patent No. 9,516,239, and U.S. Patent Application Publication No. 2014/0160319, titled CONTINUOUS VIDEO IN A LIGHT DEFICIENT ENVIRONMENT, filed on July 26, 2013, which issued on August 22, 2017 as U.S. Patent No. 9,743,016.

## Subsurface Vascular Imaging

During a surgical procedure, a surgeon is required to manipulate tissues to effect a desired medical outcome. The actions of the surgeon are limited by what is visually observable in the surgical site. Thus, the surgeon may not be aware, for example, of the disposition of vascular structures that underlie the tissues being manipulated during the procedure.

Since the surgeon is unable to visualize the vasculature beneath a surgical site, the surgeon may accidentally sever one or more critical blood vessels during the procedure.

Therefore, it is desirable to have a surgical visualization system that can acquire imaging data of the surgical site for presentation to a surgeon in which the presentation can include information related to the presence of vascular structures located beneath the surface of a surgical site.

Some aspects of the present disclosure further provide for a control circuit configured to control the illumination of a surgical site using one or more illumination sources such as laser light sources and to receive imaging data from one or more image sensors. In some aspects, the present disclosure provides for a non-transitory computer readable medium storing computer readable instructions that, when executed, cause a device to detect a blood vessel in a tissue and determine its depth below the surface of the tissue.

In some aspects, a surgical image acquisition system may include a plurality of illumination sources wherein each illumination source is configured to emit light having a specified central wavelength, a light sensor configured to receive a portion of the light reflected from a tissue sample when illuminated by the one or more of the plurality of illumination sources, and a computing system. The computing system may be configured to: receive data from the light sensor when the tissue sample is illuminated by each of the plurality of illumination sources; determine a depth location of a structure within the tissue sample based on the data received by the light sensor when the tissue sample is illuminated by each of the plurality of illumination sources, and calculate visualization data regarding the structure and the depth location of the structure. In some aspects, the visualization data may have a data format that may be used by a display system, and the structure may comprise one or more vascular tissues.

Vascular Imaging Using NIR Spectroscopy

In one aspect, a surgical image acquisition system may include an independent color cascade of illumination sources comprising visible light and light outside of the visible range to image one or more tissues within a surgical site at different times and at different depths. The surgical image acquisition system may further detect or calculate characteristics of the light reflected and/or refracted from the surgical site. The characteristics of the light may be used to provide a composite image of the tissue within the surgical site as well as provide an analysis of underlying tissue not directly visible at the surface of the surgical site. The surgical image acquisition system may determine tissue depth location without the need for separate measurement devices.

In one aspect, the characteristic of the light reflected and/or refracted from the surgical site may be an amount of absorbance of light at one or more wavelengths. Various chemical components of individual tissues may result in specific patterns of light absorption that are wavelength dependent.

In one aspect, the illumination sources may comprise a red laser source and a near infrared laser source, wherein the one or more tissues to be imaged may include vascular tissue such as veins or arteries. In some aspects, red laser sources (in the visible range) may be used to image some aspects of underlying vascular tissue based on spectroscopy in the visible red range. In some non-limiting examples, a red laser light source may source illumination having a peak wavelength that may range between 635 nm and 660 nm, inclusive. Non-limiting examples of a red laser peak wavelength may include about 635 nm, about 640 nm, about 645 nm, about 650 nm, about 655 nm, about 660 nm, or any value or range of values therebetween. In some other aspects, near infrared laser sources may be used to image underlying vascular tissue based on near infrared spectroscopy. In some non-limiting examples, a near infrared laser source may emit illumination have a wavelength that may range between 750-3000 nm, inclusive. Non-limiting examples of an infra red laser peak wavelength may include about 750 nm, about 1000 nm, about 1250 nm, about 1500 nm, about 1750 nm, about 2000 nm, about 2250 nm, about 2500 nm, about 2750 nm, 3000 nm, or any value or range of values therebetween. It may be recognized that underlying vascular tissue may be probed using a combination of red and infrared spectroscopy. In some examples, vascular tissue may be probed using a red laser source having a peak wavelength at about 660 nm and a near IR laser source having a peak wavelength at about 750 nm or at about 850 nm.

Near infrared spectroscopy (NIRS) is a noninvasive technique that allows determination of tissue oxygenation based on spectro-photometric quantitation of oxy- and deoxyhemoglobin within a tissue. In some aspects, NIRS can be used to image vascular tissue directly based on the difference in illumination absorbance between the vascular tissue and non-vascular tissue. Alternatively, vascular tissue can be indirectly visualized based on a difference of illumination absorbance of blood flow in the tissue before and after the application of physiological interventions, such as arterial and venous occlusions methods.

Instrumentation for near-IR (NIR) spectroscopy may be similar to instruments for the UV-visible and mid-IR ranges. Such spectroscopic instruments may include an illumination source, a detector, and a dispersive element to select a specific near-IR wavelength for illuminating the tissue sample. In some aspects, the source may comprise an incandescent light source or a quartz halogen light source. In some aspects, the detector may comprise semiconductor (for example, an InGaAs) photodiode or photo array. In some aspects, the dispersive element may comprise a prism or, more commonly, a diffraction grating. Fourier transform NIR instruments using an interferometer are also common, especially for wavelengths greater than about 1000 nm. Depending on the sample, the spectrum can be measured in either reflection or transmission mode.

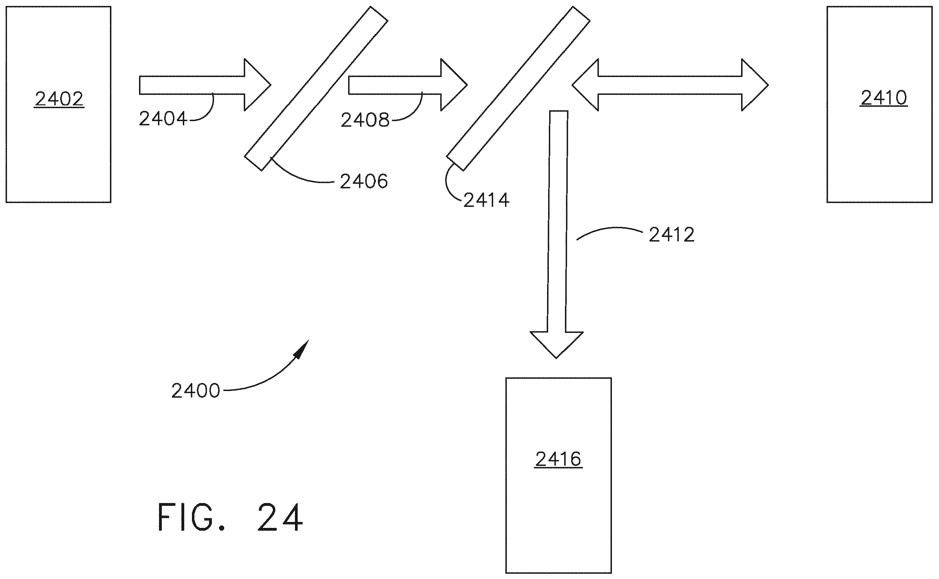

FIG. 24 depicts schematically one example of instrumentation 2400 similar to instruments for the UV-visible and mid-IR ranges for NIR spectroscopy. A light source 2402 emits a broad spectral range of illumination 2404 that impinges upon a dispersive element 2406 (such as a prism or a diffraction grating). The dispersive element 2406 operates to select a narrow wavelength portion 2408 of the light emitted by the broad spectrum light source 2402, and the selected portion 2408 of the light illuminates the tissue 2410. The light reflected from the tissue 2412 is directed to a detector 2416 (for example, by means of a dichroic mirror 2414) and the intensity of the reflected light 2412 is recorded. The wavelength of the light illuminating the tissue 2410 is selected by the dispersive element 2406. The tissue 2410 is illuminated only by a single narrow wavelength portion 2408 selected by the dispersive element 2406 form the light source 2402. The tissue 2410 is scanned with a variety of narrow wavelength portions 2408 selected by the dispersive element 2406. In this manner, a spectroscopic analysis of the tissue 2410 is obtained over a range of NIR wavelengths.

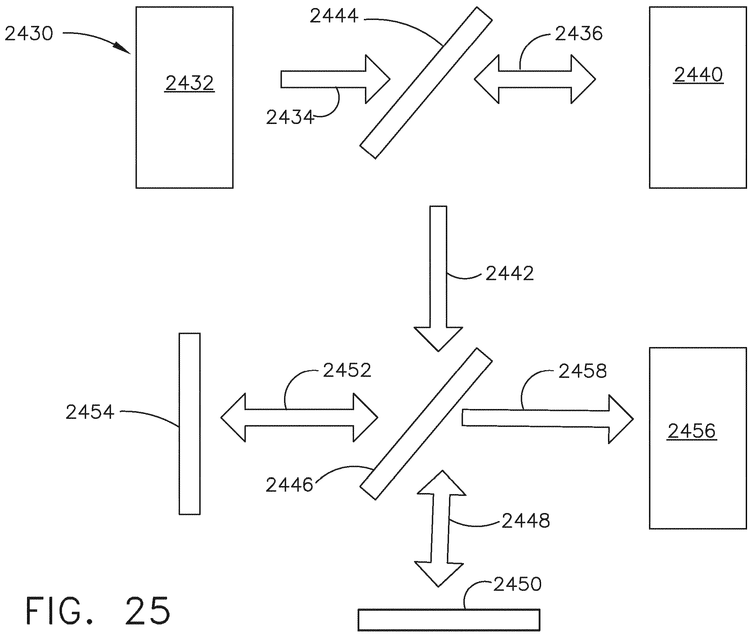

FIG. 25 depicts schematically one example of instrumentation 2430 for determining NIRS based on Fourier transform infrared imaging. In FIG. 25, a laser source emitting 2432 light in the near IR range 2434 illuminates a tissue sample 2440. The light reflected 2436 by the tissue 2440 is reflected 2442 by a mirror, such as a dichroic mirror 2444, to a beam splitter 2446. The beam splitter 2446 directs one portion of the light 2448 reflected 2436 by the tissue 2440 to a stationary mirror 2450 and one portion of the light 2452 reflected 2436 by the tissue 2440 a moving mirror 2454. The moving mirror 2454 oscillates in position based on an affixed piezoelectric transducer activated by a sinusoidal voltage having a voltage frequency. The position of the moving mirror 2454 in space corresponds to the frequency of the sinusoidal activation voltage of the piezoelectric transducer. The light reflected from the moving mirror and the stationary mirror are recombined 2458 at the beam splitter 2446 and directed to a detector 2456. Computational components receive the signal output of the detector 2456 and perform a Fourier transform (in time) of the received signal. Because the wavelength of the light received from the moving mirror 2454 varies in time with respect to the wavelength of the light received from the stationary mirror 2450, the time-based Fourier transform of the recombined light corresponds to a wavelength-based Fourier transform of the recombined light 2458. In this manner, a wavelength-based spectrum of the light reflected from the tissue 2440 is determined and spectral characteristics of the light reflected 2436 from the tissue 2440 are obtained. Changes in the absorbance of the illumination in spectral components from the light reflected from the tissue 2440 thus indicate the presence or absence of tissue having specific light absorbing properties (such as hemoglobin).

An alternative to near infrared light to determine hemoglobin oxygenation would be the use of monochromatic red light to determine the red light absorbance characteristics of hemoglobin. The absorbance characteristics of red light having a central wavelength of about 660 nm by the hemoglobin may indicate if the hemoglobin is oxygenated (arterial blood) or deoxygenated (venous blood).

In some alternative surgical procedures, contrasting agents can be used to improve the data that is collected on oxygenation and tissue oxygen consumption. In one non-limiting example, NIRS techniques may be used in conjunction with a bolus injection of a near-IR contrast agent such as indocyanine green (ICG) which has a peak absorbance at about 800 nm. ICG has been used in some medical procedures to measure cerebral blood flow.

Vascular Imaging Using Laser Doppler Flowmetry

In one aspect, the characteristic of the light reflected and/or refracted from the surgical site may be a Doppler shift of the light wavelength from its illumination source.

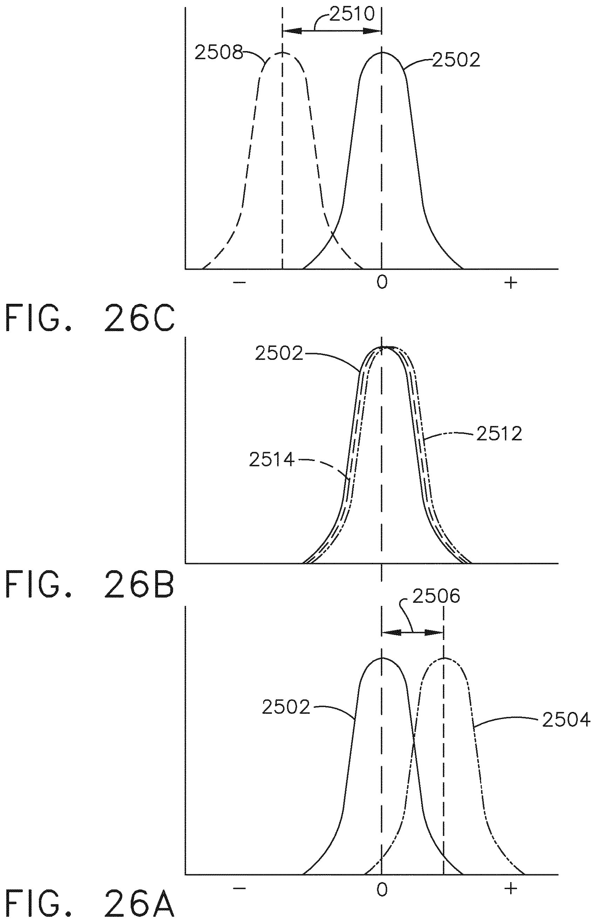

Laser Doppler flowmetry may be used to visualize and characterized a flow of particles moving relative to an effectively stationary background. Thus, laser light scattered by moving particles, such as blood cells, may have a different wavelength than that of the original illuminating laser source. In contrast, laser light scattered by the effectively stationary background (for example, the vascular tissue) may have the same wavelength of that of the original illuminating laser source. The change in wavelength of the scattered light from the blood cells may reflect both the direction of the flow of the blood cells relative to the laser source as well as the blood cell velocity. FIGS. 26A-C illustrate the change in wavelength of light scattered from blood cells that are moving away from (FIG. 26A) or towards (FIG. 26C) the laser light source.

In each of FIGS. 26A-C, the original illuminating light 2502 is depicted having a relative central wavelength of 0. It is observed from FIG. 26A that light scattered from blood cells moving away from the laser source 2504 has a wavelength shifted by some amount 2506 to a greater wavelength relative to that of the laser source (and is thus red shifted). It is also observed from FIG. 26C that light scattered from blood cells moving towards from the laser source 2508 has a wavelength shifted by some amount 2510 to a shorter wavelength relative to that of the laser source (and is thus blue shifted). The amount of wavelength shift (for example 2506 or 2510) is dependent on the velocity of the motion of the blood cells. An amount of a red shift (2506) of some blood cells is about the same as the amount of blue shift (2510) of some other blood cells. Alternatively, an amount of a red shift (2506) of some blood cells differs from the amount of blue shift (2510) of some other blood cells Thus, the velocity of the blood cells flowing away from the laser source as depicted in FIG. 26A is less than the velocity of the blood cells flowing towards the laser source as depicted in FIG. 26C based on the relative magnitude of the wavelength shifts (2506 and 2510). In contrast, and as depicted in FIG. 26B, light scattered from tissue not moving relative to the laser light source (for example blood vessels 2512 or non-vascular tissue 2514) does not demonstrate any change in wavelength.

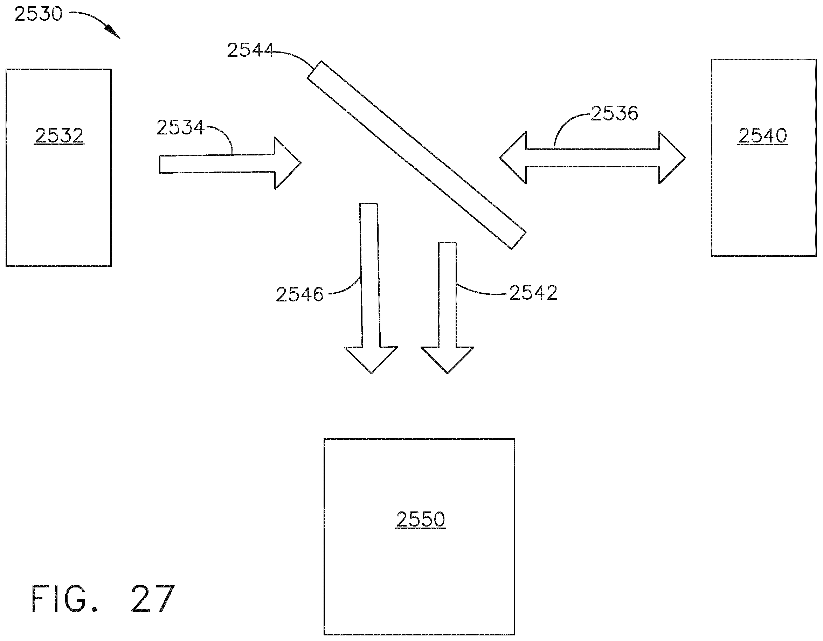

FIG. 27 depicts an aspect of instrumentation 2530 that may be used to detect a Doppler shift in laser light scattered from portions of a tissue 2540. Light 2534 originating from a laser 2532 passes through a beam splitter 2544. Some portion of the laser light 2536 is transmitted by the beam splitter 2544 and illuminates tissue 2540. Another portion of the laser light is reflected 2546 by the beam splitter 2544 to impinge on a detector 2550. The light back-scattered 2542 by the tissue 2540 is directed by the beam splitter 2544 and also impinges on the detector 2550. The combination of the light 2534 originating from the laser 2532 with the light back-scattered 2542 by the tissue 2540 results in an interference pattern detected by the detector 2550. The interference pattern received by the detector 2550 includes interference fringes resulting from the combination of the light 2534 originating from the laser 2532 and the Doppler shifted (and thus wavelength shifted) light back-scattered 2452 from the tissue 2540.

It is recognized that back-scattered light 2542 from the tissue 2540 also includes back scattered light from boundary layers within the tissue 2540 and/or wavelength-specific light absorption by material within the tissue 2540. As a result, the interference pattern observed at the detector 2550 incorporates interference fringe features from these additional optical effects and therefore confounds the calculation of the Doppler shift unless properly analyzed.

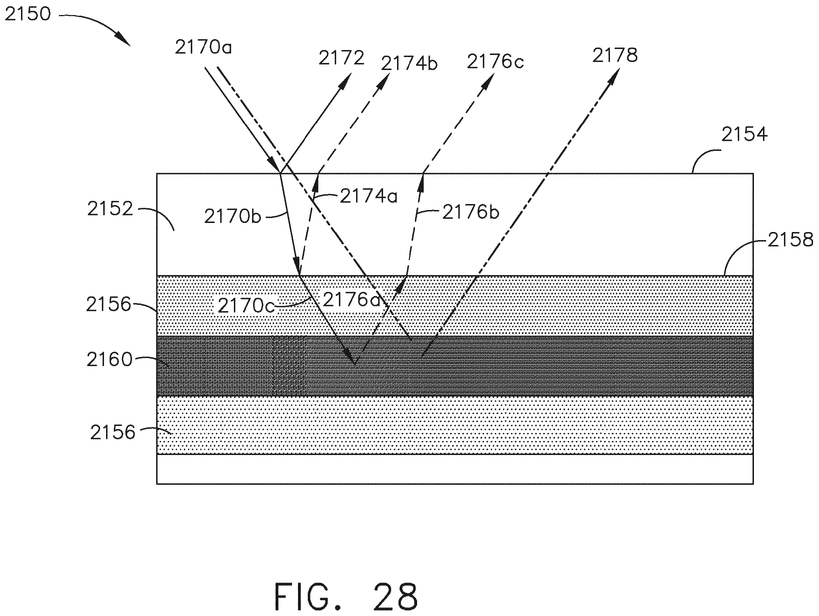

FIG. 28 depicts some of these additional optical effects. It is well known that light traveling through a first optical medium having a first refractive index, n1, is reflected at an interface with a second optical medium having a second refractive index, n2. The light transmitted through the second optical medium will have a transmission angle relative to the interface that differs from the angle of the incident light based on a difference between the refractive indices n1 and n2 (Snell's Law). FIG. 28 illustrates the effect of Snell's Law on light impinging on the surface of a multi-component tissue 2150, as is presented in a surgical field. The multi-component tissue 2150 is composed of an outer tissue layer 2152 having a refractive index n1 and a buried tissue, such as a blood vessel having a vessel wall 2156. The blood vessel wall 2156 is characterized by a refractive index n2. Blood flows within the lumen of the blood vessel 2160. It is important during a surgical procedure to determine the position of the blood vessel 2160 below the surface 2154 of the outer tissue layer 2152 and to characterize the blood flow using Doppler shift techniques.

An incident laser light 2170a is used to probe for the blood vessel 2160 and is directed on the top surface 2154 of the outer tissue layer 2152. A portion 2172 of the incident laser light 2170a is reflected at the top surface 2154. Another portion 2170b of the incident laser light 2170a penetrates the outer tissue layer 2152. The reflected portion 2172 at the top surface 2154 of the outer tissue layer 2152 has the same path length of the incident light 2170a, and therefore has the same wavelength and phase of the incident light 2170a. However, the portion 2170b of light transmitted into the outer tissue layer 2152 will have a transmission angle that differs from the incidence angle of the light impinging on the tissue surface because the outer tissue layer 2152 has an index of refraction n1 that differs from the index of refraction of air.

If the portion of light transmitted through the outer tissue layer 2152 impinges on a second tissue surface 2158, for example of the blood vessel wall 2156, some portion 2174a,b of light will be reflected back towards the source of the incident light 2170a. The light thus reflected 2174a at the interface between the outer tissue layer 2152 and the blood vessel wall 2156 will have the same wavelength as the incident light 2170a, but will be phase shifted due to the change in the light path length. Projecting the light reflected 2174a,b from the interface between the outer tissue layer 2152 and the blood vessel wall 2156 along with the incident light on the sensor, will produce an interference pattern based on the phase difference between the two light sources.

Further, a portion of the incident light 2170c is transmitted through the blood vessel wall 2156 and penetrate into the blood vessel lumen 2160. This portion of the incident light 2170c interacts with the moving blood cells in the blood vessel lumen 2160 and is reflected back 2176a-c towards the source of the impinging light having a wavelength Doppler shifted according to the velocity of the blood cells, as disclosed above. The Doppler shifted light reflected 2176a-c from the moving blood cells are projected along with the incident light on the sensor, resulting in an interference pattern having a fringe pattern based on the wavelength difference between the two light sources.

In FIG. 28, a light path 2178 is presented of light impinging on the red blood cells in the blood vessel lumen 2160 if there are no changes in refractive index between the emitted light and the light reflected by the moving blood cells. In this example, only a Doppler shift in the reflected light wavelength can be detected. However, the light reflected by the blood cells (2176a-c) incorporates phase changes due to the variation in the tissue refractive indices in addition to the wavelength changes due to the Doppler Effect.

Thus, it is understood that if the light sensor receives the incident light, the light reflected from one or more tissue interfaces (2172, and 2174a,b) and the Doppler shifted light from the blood cells (2176a-c), the interference pattern thus produced on the light sensor includes the effects due to the Doppler shift (change in wavelength) as well as the effects due to the change in refractive index within the tissue (change in phase). As a result, a Doppler analysis of the light reflected by the tissue sample produces erroneous results if the effects due to changes in the refractive index within the sample are not compensated for.

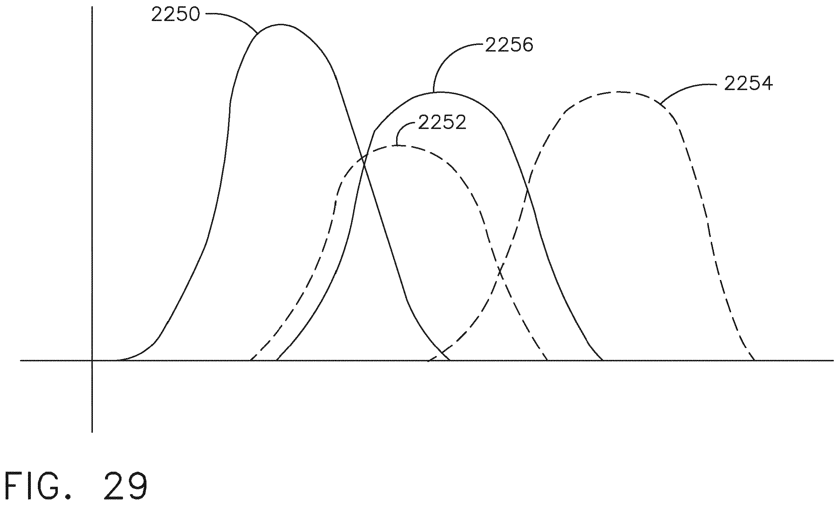

FIG. 29 illustrates an example of the effects on a Doppler analysis of light that impinge 2250 on a tissue sample to determine the depth and location of an underlying blood vessel. If there is no intervening tissue between the blood vessel and the tissue surface, the interference pattern detected at the sensor is due primarily to the change in wavelength reflected from the moving blood cells. As a result, a spectrum 2252 derived from the interference pattern generally reflects only the Doppler shift of the blood cells. However, if there is intervening tissue between the blood vessel and the tissue surface, the interference pattern detected at the sensor is due to a combination of the change in wavelength reflected from the moving blood cells and the phase shift due to the refractive index of the intervening tissue. A spectrum 2254 derived from such an interference pattern, results in the calculation of the Doppler shift that is confounded due to the additional phase change in the reflected light. If information regarding the characteristics (thickness and refractive index) of the intervening tissue is known, the resulting spectrum 2256 is corrected to provide a more accurate calculation of the change in wavelength.

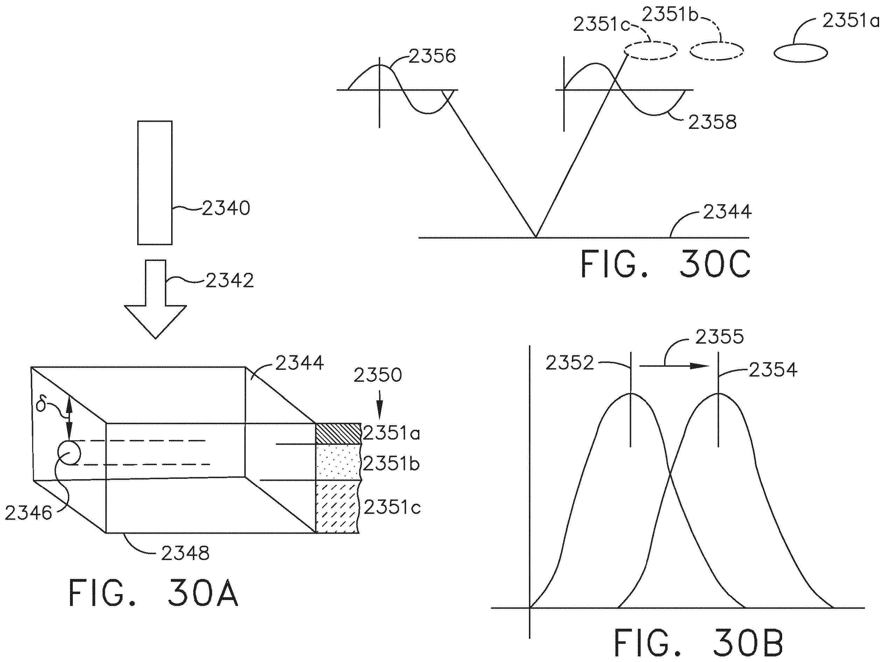

It is recognized that the tissue penetration depth of light is dependent on the wavelength of the light used. Thus, the wavelength of the laser source light is chosen to detect particle motion (such a blood cells) at a specific range of tissue depth. FIGS. 30A-C depict schematically a means for detect moving particles such as blood cells at a variety of tissue depths based on the laser light wavelength. As illustrated in FIG. 30A, a laser source 2340 directs an incident beam of laser light 2342 onto a surface 2344 of a surgical site. A blood vessel 2346 (such as a vein or artery) is disposed within the tissue 2348 at some depth $\delta$ from the tissue surface. The penetration depth 2350 of a laser into a tissue 2348 is dependent at least in part on the laser wavelength. Thus, laser light having a wavelength in the red range of about 635 nm to about 660 nm, penetrates the tissue 2351a to a depth of about 1 mm. Laser light having a wavelength in the green range of about 520 nm to about 532 nm penetrates the tissue 2351b to a depth of about 2-3 mm. Laser light having a wavelength in the blue range of about 405 nm to about 445 nm penetrates the tissue 2351c to a depth of about 4 mm or greater. In the example depicted in FIGS. 30A-C, a blood vessel 2346 is located at a depth $\delta$ of about 2-3 mm below the tissue surface. Red laser light will not penetrate to this depth and thus will not detect blood cells flowing within this vessel. However, both green and blue laser light can penetrate this depth. Therefore, scattered green and blue laser light from the blood cells within the blood vessel 2346 demonstrates a Doppler shift in wavelength.

FIG. 30B illustrates how a Doppler shift 2355 in the wavelength of reflected laser light appears. The emitted light (or laser source light 2342) impinging on a tissue surface 2344 has a central wavelength 2352. For example, light from a green laser has a central wavelength 2352 within a range of about 520 nm to about 532 nm. The reflected green light has a central wavelength 2354 shifted to a longer wavelength (red shifted) if the light was reflected from a particle such as a red blood cell that is moving away from the detector. The difference between the central wavelength 2352 of the emitted laser light and the central wavelength 2354 of the emitted laser light comprises the Doppler shift 2355.

As disclosed above with respect to FIGS. 28 and 29, laser light reflected from structures within a tissue 2348 also show a phase shift in the reflected light due to changes in the index of refraction arising from changes in tissue structure or composition. The emitted light (or laser source light 2342) impinging on a tissue surface 2344 has a first phase characteristic 2356. The reflected laser light has a second phase characteristic 2358. It is recognized that blue laser light that can penetrate tissue to a depth of about 4 mm or greater 2351c encounters a greater variety of tissue structures than red laser light (about 1 mm 2351a) or green laser light (about 2-3 mm 2351b). Consequently, as illustrated in FIG. 30C, the phase shift 2358 of reflected blue laser light is significant at least due to the depth of penetration.

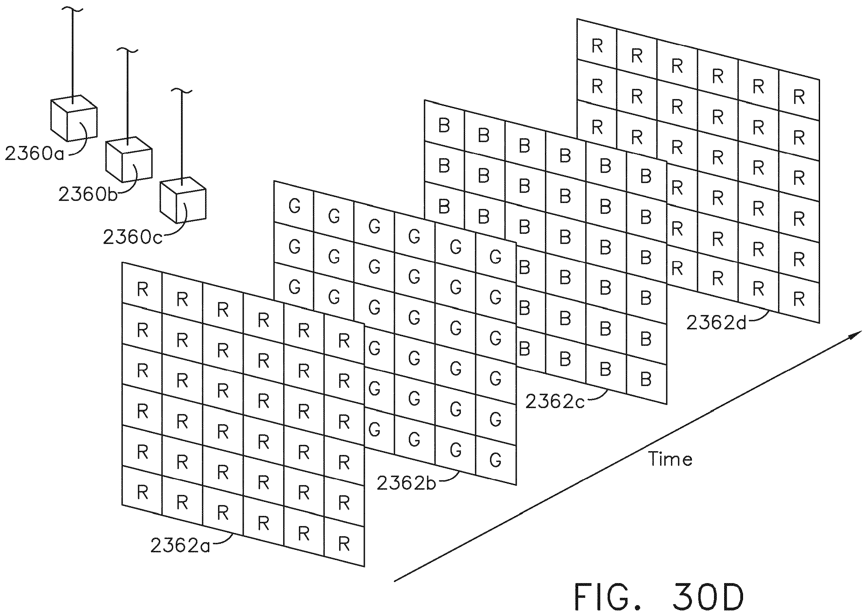

FIG. 30D illustrates illuminating tissue by red 2360a, green 2360b and blue 2360c laser light in a sequential manner. A tissue is probed by red 2360a, green 2360b and blue 2360c laser illumination in a sequential manner. Alternatively, one or more combinations of red 2360a, green 2360b, and blue 2360c laser light, as depicted in FIGS. 23D - 23F and disclosed above, are used to illuminate the tissue according to a defined illumination sequence. 30D illustrates the effect of such illumination on a CMOS imaging sensor 2362a-d over time. Thus, at a first time $t_1$, the CMOS sensor 2362a is illuminated by the red 2360a laser. At a second time $t_2$ the CMOS sensor 2362b is illuminated by the green 2360b laser. At a third time $t_3$, the CMOS sensor 2362c is illuminated by the blue 2360c laser. The illumination cycle is then repeated starting at a fourth time $t_4$ in which the CMOS sensor 2362d is illuminated by the red 2360a lase again. It is recognized that sequential illumination of the tissue by laser illumination at differing wavelengths may permit a Doppler analysis at varying tissue depths over time. Although red 2360a, green 2360b and blue 2360c laser sources are used to illuminate the surgical site, it is recognized that other wavelengths outside of visible light (such as in the infra red or ultraviolet regions) are used to illuminate the surgical site for Doppler analysis.

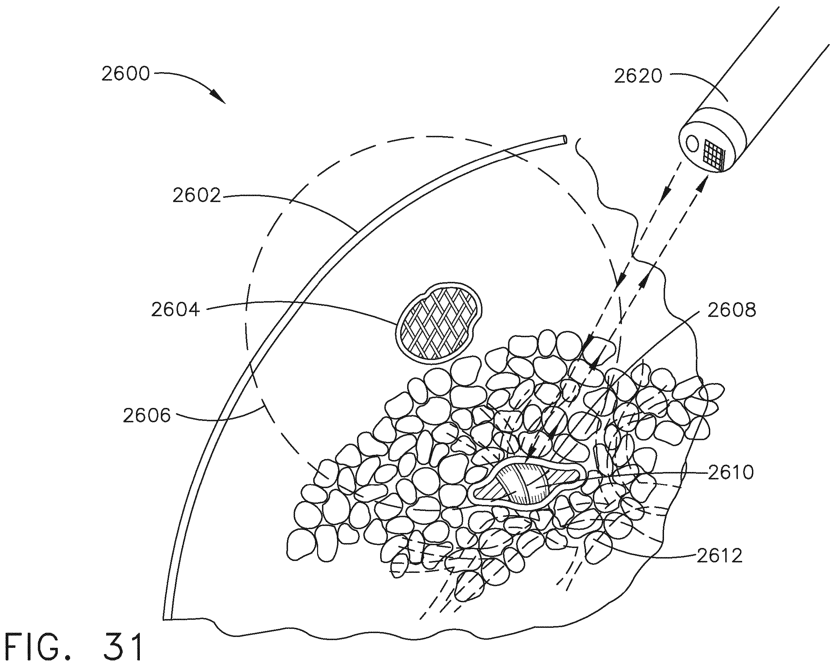

FIG. 31 illustrates an example of a use of Doppler imaging to detect the present of blood vessels not otherwise viewable at a surgical site 2600. In FIG. 31, a surgeon wishes to excise a tumor 2602 found in the right superior posterior lobe 2604 of a lung. Because the lungs are highly vascular, care must be taken to identify only those blood vessels associate with the tumor and to seal only those vessels without compromising the blood flow to the non-affected portions of the lung. In FIG. 31, the surgeon has identified the margin 2606 of the tumor 2604. The surgeon may then cut an initial dissected area 2608 in the margin region 2606, and exposed blood vessels 2610 may be observed for cutting and sealing. The Doppler imaging detector 2620 is used to locate and identify blood vessels not observable 2612 in the dissected area. An imaging system receives data from the Doppler imaging detector 2620 for analysis and display of the data obtained from the surgical site 2600. The imaging system includes a display to illustrate the surgical site 2600 including a visible image of the surgical site 2600 along with an image overlay of the hidden blood vessels 2612 on the image of the surgical site 2600.

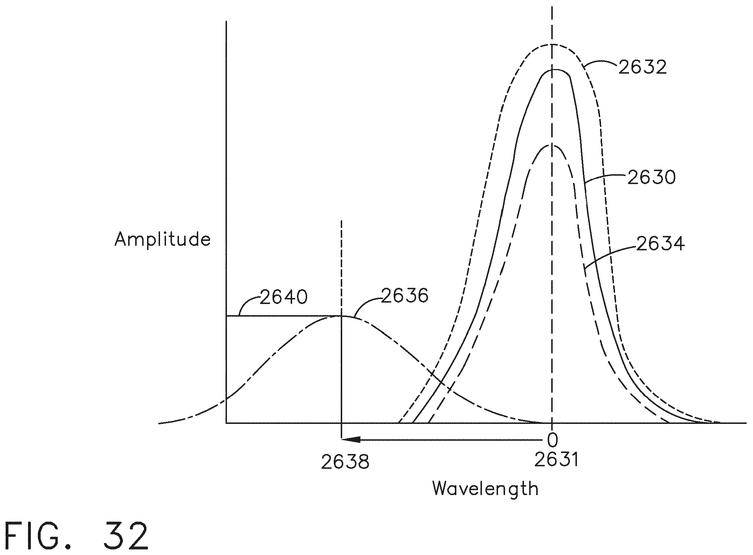

In the scenario disclosed above regarding FIG. 31, a surgeon wishes to sever blood vessels that supply oxygen and nutrients to a tumor while sparing blood vessels associated with non-cancerous tissue. Additionally, the blood vessels may be disposed at different depths in or around the surgical site 2600. The surgeon must therefore identify the position (depth) of the blood vessels as well as determine if they are appropriate for resection. FIG. 32 illustrates one method for identifying deep blood vessels based on a Doppler shift of light from blood cells flowing therethrough. As disclosed above, red laser light has a penetration depth of about 1mm and green laser light has a penetration depth of about 2-3 mm. However, a blood vessel having a below-surface depth of 4mm or more will be outside the penetration depths at these wavelengths. Blue laser light, however, can detect such blood vessels based on their blood flow.

FIG. 32 depicts the Doppler shift of laser light reflected from a blood vessel at a specific depth below a surgical site. The site may be illuminated by red laser light, green laser light, and blue laser light. The central wavelength 2630 of the illuminating light is normalized to a relative central 3631. If the blood vessel lies at a depth of 4 or more mm below the surface of the surgical site, neither the red laser light nor the green laser light will be reflected by the blood vessel. Consequently, the central wavelength 2632 of the reflected red light and the central wavelength 2634 of the reflected green light will not differ much from the central wavelength 2630 of the illuminating red light or green light, respectively. However, if the site is illuminated by blue laser light, the central wavelength 2638 of the reflected blue light 2636 will differ from the central wavelength 2630 of the illuminating blue light. The amplitude of the reflected blue light 2636 is also significantly reduced from the amplitude of the illuminating blue light. A surgeon thus determines the presence of a deep lying blood vessel along with its approximate depth, and thereby avoiding the deep blood vessel during surface tissue dissection.

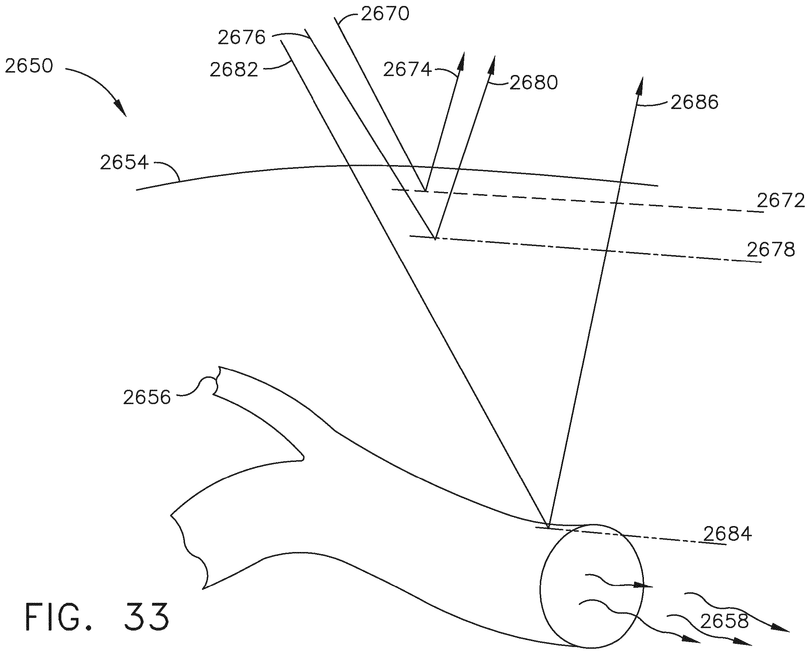

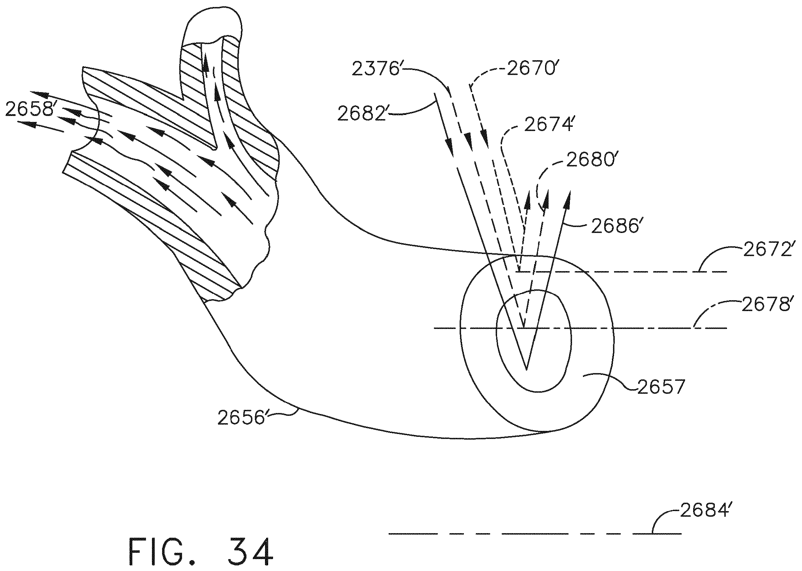

FIGS. 33 and 34 illustrates schematically the use of laser sources having differing central wavelengths (colors) for determining the approximate depth of a blood vessel beneath the surface of a surgical site. FIG. 33 depicts a first surgical site 2650 having a surface 2654 and a blood vessel 2656 disposed below the surface 2654. In one method, the blood vessel 2656 is identified based on a Doppler shift of light impinging on the flow 2658 of blood cells within the blood vessel 2656. The surgical site 2650 is illuminated by light from a number of lasers 2670, 2676, 2682, each laser being characterized by emitting light at one of several different central wavelengths. As noted above, illumination by a red laser 2670 can only penetrate tissue by about 1 mm. Thus, if the blood vessel 2656 was located at a depth of less than 1 mm 2672 below the surface 2654, the red laser illumination would be reflected 2674 and a Doppler shift of the reflected red illumination 2674 may be determined. Further, as noted above, illumination by a green laser 2676 can only penetrate tissue by about 2-3 mm. If the blood vessel 2656 was located at a depth of about 2-3 mm 2678 below the surface 2654, the green laser illumination would be reflected 2680 while the red laser illumination 2670 would not, and a Doppler shift of the reflected green illumination 2680 may be determined. However, as depicted in FIG. 33, the blood vessel 2656 is located at a depth of about 4 mm 2684 below the surface 2654. Therefore, neither the red laser illumination 2670 nor the green laser illumination 2676 would be reflected. Instead, only the blue laser illumination would be reflected 2686 and a Doppler shift of the reflected blue illumination 2686 is determined.

In contrast to the blood vessel 2656 depicted in FIG. 33, the blood vessel 2656' depicted in FIG. 34 is located closer to the surface of the tissue at the surgical site. Blood vessel 2656' is also distinguished from blood vessel 2656 in that blood vessel 2656' is illustrated to have a much thicker wall 2657. Thus, blood vessel 2656' is an example of an artery while blood vessel 2656 may be an example of a vein because arterial walls are known to be thicker than venous walls. Arterial walls have a thickness of about 1.3 mm. As disclosed above, red laser illumination 2670' can penetrate tissue to a depth of about 1mm 2672'. Thus, even if a blood vessel 2656' is exposed at a surgical site (see 2610 at FIG. 31), red laser light that is reflected 2674' from the surface of the blood vessel 2656', is not be able to visualize blood flow 2658' within the blood vessel 2656' under a Doppler analysis due to the thickness of the blood vessel wall 2657. However, as disclosed above, green laser light impinging 2676' on the surface of a tissue penetrates to a depth of about 2-3mm 2678'. Further, blue laser light impinging 2682' on the surface of a tissue penetrates to a depth of about 4 mm 2684'. Consequently, green laser light is reflected 2680' from the blood cells flowing 2658' within the blood vessel 2656' and blue laser light be reflected 2686' from the blood cells flowing 2658' within the blood vessel 2656'. As a result, a Doppler analysis of the reflected green light 2680' and reflected blue light 2686' provides information regarding blood flow in near-surface blood vessel, especially the approximate depth of the blood vessel.

As disclosed above, the depth of blood vessels below the surgical site is probed based on wavelength-dependent Doppler imaging. The amount of blood flow through such a blood vessel is also determined by speckle contrast (interference) analysis. Doppler shift indicate a moving particle with respect to a stationary light source. As disclosed above, the Doppler wavelength shift is an indication of the velocity of the particle motion. Individual particles such as blood cells are not separately observable. However, the velocity of each blood cell will produce a proportional Doppler shift. An interference pattern is generated by the combination of the light back-scattered from multiple blood cells due to the differences in the Doppler shift of the back-scattered light from each of the blood cells. The interference pattern is an indication of the number density of blood cells within a visualization frame. The interference pattern is termed speckle contrast. Speckle contrast analysis may be calculated using a full frame 300x300 CMOS imaging array, and the speckle contrast is directly related to the amount of moving particles (for example blood cells) interacting with the laser light over a given exposure period.

A CMOS image sensor is coupled to a digital signal processor (DSP). Each pixel of the sensor may be multiplexed and digitized. The Doppler shift in the light is analyzed by looking at the source laser light in comparison to the Doppler shifted light. A greater Doppler shift and speckle is related to a greater number of blood cells and their velocity in the blood vessel.

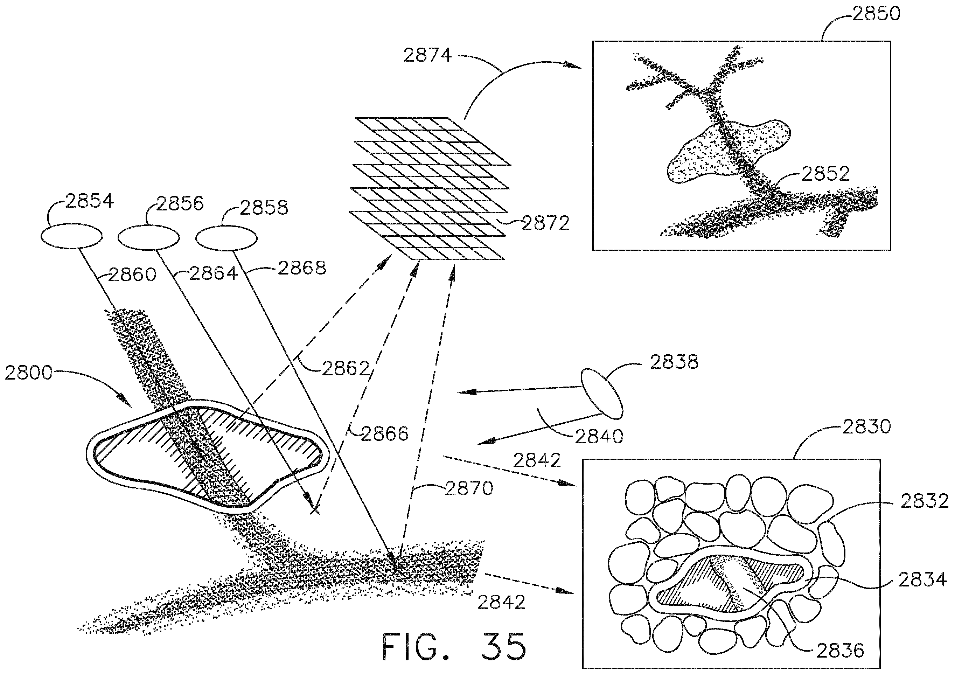

FIG. 35 depicts an aspect of a composite visual display 2800 that is presented a surgeon during a surgical procedure. The composite visual display 2800 is constructed by overlaying a white light image 2830 of the surgical site with a Doppler analysis image 2850.

In some aspects, the white light image 2830 may portray the surgical site 2832, one or more surgical incisions 2834, and the tissue 2836 readily visible within the surgical incision 2834. The white light image 2830 may be generated by illuminating 2840 the surgical site 2832 with a white light source 2838 and receiving the reflected white light 2842 by an optical detector. Although a white light source 2838 may be used to illuminate the surface of the surgical site, in one aspect, the surface of the surgical site may be visualized using appropriate combinations of red 2854, green 2856, and blue 2858 laser light as disclosed above with respect to FIGS. 23C -23F.

In some aspects, the Doppler analysis image 2850 may include blood vessel depth information along with blood flow information 2852 (from speckle analysis). As disclosed above, blood vessel depth and blood flow velocity may be obtained by illuminating the surgical site with laser light of multiple wavelengths, and determining the blood vessel depth and blood flow based on the known penetration depth of the light of a particular wavelength. In general, the surgical site 2832 may be illuminated by light emitted by one or more lasers such as a red leaser 2854, a green laser 2856, and a blue laser 2858. A CMOS detector 2872 may receive the light reflected back (2862, 2866, 2870) from the surgical site 2832 and its surrounding tissue. The Doppler analysis image 2850 may be constructed 2874 based on an analysis of the multiple pixel data from the CMOS detector 2872.

In one aspect, a red laser 2854 may emit red laser illumination 2860 on the surgical site 2832 and the reflected light 2862 may reveal surface or minimally subsurface structures. In one aspect, a green laser 2856 may emit green laser illumination 2864 on the surgical site 2832 and the reflected light 2866 may reveal deeper subsurface characteristics. In another aspect, a blue laser 2858 may emit blue laser illumination 2868 on the surgical site 2832 and the reflected light 2870 may reveal, for example, blood flow within deeper vascular structures. In addition, the speckle contrast analysis my present the surgeon with information regarding the amount and velocity of blood flow through the deeper vascular structures.

Although not depicted in FIG. 35, it may be understood that the imaging system may also illuminate the surgical site with light outside of the visible range. Such light may include infra red light and ultraviolet light. In some aspects, sources of the infra red light or ultra-

violet light may include broad-band wavelength sources (such as a tungsten source, a tungsten-halogen source, or a deuterium source). In some other aspects, the sources of the infra red or ultraviolet light may include narrow-band wavelength sources (IR diode lasers, UV gas lasers or dye lasers).

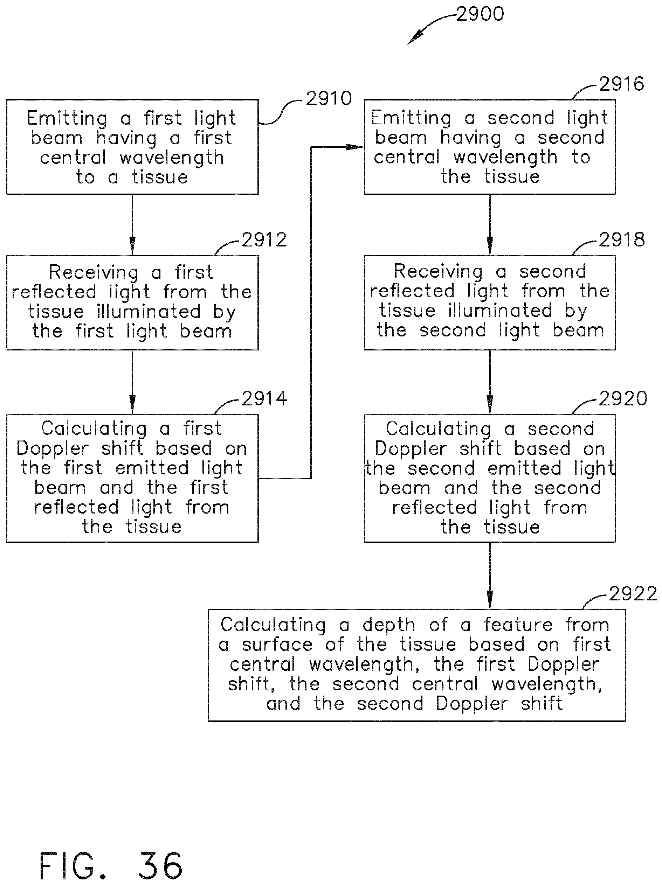

FIG. 36 is a flow chart 2900 of a method for determining a depth of a surface feature in a piece of tissue. An image acquisition system illuminates 2910 a tissue with a first light beam having a first central frequency and receive 2912 a first reflected light from the tissue illuminated by the first light beam. The image acquisition system then calculates 2914 a first Doppler shift based on the first light beam and the first reflected light. The image acquisition system then illuminates 2916 the tissue with a second light beam having a second central frequency and receive 2918 a second reflected light from the tissue illuminated by the second light beam. The image acquisition system then calculates 2920 a second Doppler shift based on the second light beam and the second reflected light. The image acquisition system then calculates 2922 a depth of a tissue feature based at least in part on the first central wavelength, the first Doppler shift, the second central wavelength, and the second Doppler shift. The tissue features may include the presence of moving particles, such as blood cells moving within a blood vessel, and a direction and velocity of flow of the moving particles. It is understood that the method is extended to include illumination of the tissue by any one or more additional light beams. Further, the system calculates an image comprising a combination of an image of the tissue surface and an image of the structure disposed within the tissue.

In some aspects, multiple visual displays may be used. For example, a 3D display may provide a composite image displaying the combined white light (or an appropriate combination of red, green, and blue laser light) and laser Doppler image. Additional displays may provide only the white light display or a displaying showing a composite white light display and an NIRS display to visualize only the blood oxygenation response of the tissue. However, the NIRS display may not be required every cycle allowing for response of tissue.

### Subsurface Tissue Characterization Using Multispectral OCT

During a surgical procedure, the surgeon may employ "smart" surgical devices for the manipulation of tissue. Such devices may be considered "smart" in that they include automated features to direct, control, and/or vary the actions of the devices based parameters relevant to their uses. The parameters may include the type and/or composition of the tissue being manipulated. If the type and/or composition of the tissue being manipulated is unknown, the actions of the smart devices may be inappropriate for the tissue being manipulated. As a result, tissues may be damaged or the manipulation of the tissue may be ineffective due to inappropriate settings of the smart device.

The surgeon may manually attempt to vary the parameters of the smart device in a trial-and-error manner, resulting in an inefficient and lengthy surgical procedure.

Therefore, it is desirable to have a surgical visualization system that can probe tissue structures underlying a surgical site to determine their structural and compositional characteristics, and to provide such data to smart surgical instruments being used in a surgical procedure.

Some aspects of the present disclosure further provide for a control circuit configured to control the illumination of a surgical site using one or more illumination sources such as laser light sources and to receive imaging data from one or more image sensors. In some aspects, the present disclosure provides for a non-transitory computer readable medium storing computer readable instructions that, when executed, cause a device to characterize structures below the surface at a surgical site and determine the depth of the structures below the surface of the tissue.

In some aspects, a surgical image acquisition system may comprise a plurality of illumination sources wherein each illumination source is configured to emit light having a specified central wavelength, a light sensor configured to receive a portion of the light reflected from a tissue sample when illuminated by the one or more of the plurality of illumination sources, and a computing system. The computing system may be configured to receive data from the light sensor when the tissue sample is illuminated by each of the plurality of illumination sources, calculate structural data related to a characteristic of a structure within the tissue sample based on the data received by the light sensor when the tissue sample is illuminated by each of the illumination sources, and transmit the structural data related to the characteristic of the structure to be received by a smart surgical device. In some aspects, the characteristic of the structure is a surface characteristic or a structure composition.

In one aspect, a surgical system may include multiple laser light sources and may receive laser light reflected from a tissue. The light reflected from the tissue may be used by the system to calculate surface characteristics of components disposed within the tissue. The characteristics of the components disposed within the tissue may include a composition of the components and/or a metric related to surface irregularities of the components.

In one aspect, the surgical system may transmit data related to the composition of the components and/or metrics related to surface irregularities of the components to a second instrument to be used on the tissue to modify the control parameters of the second instrument.

In some aspects, the second device may be an advanced energy device and the modifications of the control parameters may include a clamp pressure, an operational power level, an operational frequency, and a transducer signal amplitude.

As disclosed above, blood vessels may be detected under the surface of a surgical site base on the Doppler shift in light reflected by the blood cells moving within the blood vessels.

Laser Doppler flowmetry may be used to visualize and characterized a flow of particles moving relative to an effectively stationary background. Thus, laser light scattered by moving particles, such as blood cells, may have a different wavelength than that of the original illuminating laser source. In contrast, laser light scattered by the effectively stationary background (for example, the vascular tissue) may have the same wavelength of that of the original illuminating laser source. The change in wavelength of the scattered light from the blood cells may reflect both the direction of the flow of the blood cells relative to the laser source as well as the blood cell velocity. As previously disclosed, FIGS. 26A-C illustrate the change in wavelength of light scattered from blood cells that is moving away from (FIG. 26A) or towards (FIG. 26C) the laser light source.

In each of FIGS. 26A-C, the original illuminating light 2502 is depicted having a relative central wavelength of 0. It is observed from FIG. 26A that light scattered from blood cells moving away from the laser source 2504 has a wavelength shifted by some amount 2506 to a greater wavelength relative to that of the laser source (and is thus red shifted). It is also observed from FIG. 24C that light scattered from blood cells moving towards from the laser source 2508 has a wavelength shifted by some amount 2510 to a shorter wavelength relative to that of the laser source (and is thus blue shifted). The amount of wavelength shift (for example 2506 or 2510) is dependent on the velocity of the motion of the blood cells. An amount of a red shift (2506) of some blood cells is about the same as the amount of blue shift (2510) of some other blood cells. Alternatively, an amount of a red shift (2506) of some blood cells differs from the amount of blue shift (2510) of some other blood cells Thus, the velocity of the blood cells flowing away from the laser source as depicted in FIG. 24A is less than the velocity of the blood cells flowing towards the laser source as depicted in FIG. 26C based on the relative magnitude of the wavelength shifts (2506 and 2510). In contrast, and as depicted in FIG. 26B, light scattered from tissue not moving relative to the laser light source (for example blood vessels 2512 or non-vascular tissue 2514) does not demonstrate any change in wavelength.

As previously disclosed, FIG. 27 depicts instrumentation 2530 that is used to detect a Doppler shift in laser light scattered from portions of a tissue 2540. Light 2534 originating from a laser 2532 passes through a beam splitter 2544. Some portion of the laser light 2536 is transmitted by the beam splitter 2544 and illuminates tissue 2540. Another portion of the laser light is reflected 2546 by the beam splitter 2544 to impinge on a detector 2550. The light back-scattered 2542 by the tissue 2540 is directed by the beam splitter 2544 and also impinges on the detector 2550. The combination of the light 2534 originating from the laser 2532 with the light back-scattered 2542 by the tissue 2540 results in an interference pattern detected by the detector 2550. The interference pattern received by the detector 2550 includes interference fringes resulting from the combination of the light 2534 originating from the laser 2532 and the Doppler shifted (and thus wavelength shifted) light back-scattered 2452 from the tissue 2540.

It is recognized that back-scattered light 2542 from the tissue 2540 also includes back scattered light from boundary layers within the tissue 2540 and/or wavelength-specific light absorption by material within the tissue 2540. As a result, the interference pattern observed at the detector 2550 incorporates interference fringe features from these additional optical effects and therefore confounds the calculation of the Doppler shift unless properly analyzed.

It may be recognized that light reflected from the tissue may also include back scattered light from boundary layers within the tissue and/or wavelength-specific light absorption by material within the tissue. As a result, the interference pattern observed at the detector may incorporate fringe features that may confound the calculation of the Doppler shift unless properly analyzed.

As previously disclosed, FIG. 28 depicts some of these additional optical effects. It is well known that light traveling through a first optical medium having a first refractive index, n1, is reflected at an interface with a second optical medium having a second refractive index, n2. The light transmitted through the second optical medium will have a transmission angle relative to the interface that differs from the angle of the incident light based on a difference between the refractive indices n1 and n2 (Snell's Law). FIG. 26 illustrates the effect of Snell's Law on light impinging on the surface of a multi-component tissue 2150, as is presented in a surgical field. The multi-component tissue 2150 is composed of an outer tissue layer 2152 having a refractive index n1 and a buried tissue, such as a blood vessel having a vessel wall 2156. The blood vessel wall 2156 is characterized by a refractive index n2. Blood flows within the lumen of the blood vessel 2160. It is important during a surgical procedure to determine the position of the blood vessel 2160 below the surface 2154 of the outer tissue layer 2152 and to characterize the blood flow using Doppler shift techniques.

An incident laser light 2170a is used to probe for the blood vessel 2160 and is directed on the top surface 2154 of the outer tissue layer 2152. A portion 2172 of the incident laser light 2170a is reflected at the top surface 2154. Another portion 2170b of the incident laser light 2170a penetrates the outer tissue layer 2152. The reflected portion 2172 at the top surface 2154 of the outer tissue layer 2152 has the same path length of the incident light 2170a, and therefore has the same wavelength and phase of the incident light 2170a. However, the portion 2170b of light transmitted into the outer tissue layer 2152 will have a transmission angle that differs from the incidence angle of the light impinging on the tissue surface because the outer tissue layer 2152 has an index of refraction n1 that differs from the index of refraction of air.

If the portion of light transmitted through the outer tissue layer 2152 impinges on a second tissue surface 2158, for example of the blood vessel wall 2156, some portion 2174a,b of light will be reflected back towards the source of the incident light 2170a. The light thus reflected 2174a at the interface between the outer tissue layer 2152 and the blood vessel wall 2156 will have the same wavelength as the incident light 2170a, but will be phase shifted due to the change in the light path length. Projecting the light reflected 2174a,b from the interface between the outer tissue layer 2152 and the blood vessel wall 2156 along with the incident light on the sensor, will produce an interference pattern based on the phase difference between the two light sources.

Further, a portion of the incident light 2170c is transmitted through the blood vessel wall 2156 and penetrate into the blood vessel lumen 2160. This portion of the incident light 2170c interacts with the moving blood cells in the blood vessel lumen 2160 and is reflected back 2176a-c towards the source of the impinging light having a wavelength Doppler shifted according to the velocity of the blood cells, as disclosed above. The Doppler shifted light reflected 2176a-c from the moving blood cells are projected along with the incident light on the sensor, resulting in an interference pattern having a fringe pattern based on the wavelength difference between the two light sources.

In FIG. 28, a light path 2178 is presented of light impinging on the red blood cells in the blood vessel lumen 2160 if there are no changes in refractive index between the emitted light and the light reflected by the moving blood cells. In this example, only a Doppler shift in the reflected light wavelength can be detected. However, the light reflected by the blood cells (2176a-c) incorporates phase changes due to the variation in the tissue refractive indices in addition to the wavelength changes due to the Doppler Effect.

Thus, it may be understood that if the light sensor receives the incident light, the light reflected from one or more tissue interfaces (2172, and 2174a,b) and the Doppler shifted light from the blood cells (2176a-c), the interference pattern thus produced on the light sensor includes the effects due to the Doppler shift (change in wavelength) as well as the effects due to the change in refractive index within the tissue (change in phase). As a result, a Doppler analysis of the light reflected by the tissue sample produces erroneous results if the effects due to changes in the refractive index within the sample are not compensated for.

As previously disclosed, FIG. 29 illustrates an example of the effects on a Doppler analysis of light that impinge 2250 on a tissue sample to determine the depth and location of an underlying blood vessel. If there is no intervening tissue between the blood vessel and the tissue surface, the interference pattern detected at the sensor is due primarily to the change in wavelength reflected from the moving blood cells. As a result, a spectrum 2252 derived from the interference pattern generally reflects only the Doppler shift of the blood cells. However, if there is intervening tissue between the blood vessel and the tissue surface, the interference pattern detected at the sensor is due to a combination of the change in wavelength reflected from the moving blood cells and the phase shift due to the refractive index of the intervening tissue. A spectrum 2254 derived from such an interference pattern, results in the calculation of the Doppler shift that is confounded due to the additional phase change in the reflected light. In some aspects, if information regarding the characteristics (thickness and refractive index) of the intervening tissue is known, the resulting spectrum 2256 is corrected to provide a more accurate calculation of the change in wavelength.

It may be recognized that the phase shift in the reflected light from a tissue may provide additional information regarding underlying tissue structures, regardless of Doppler effects.

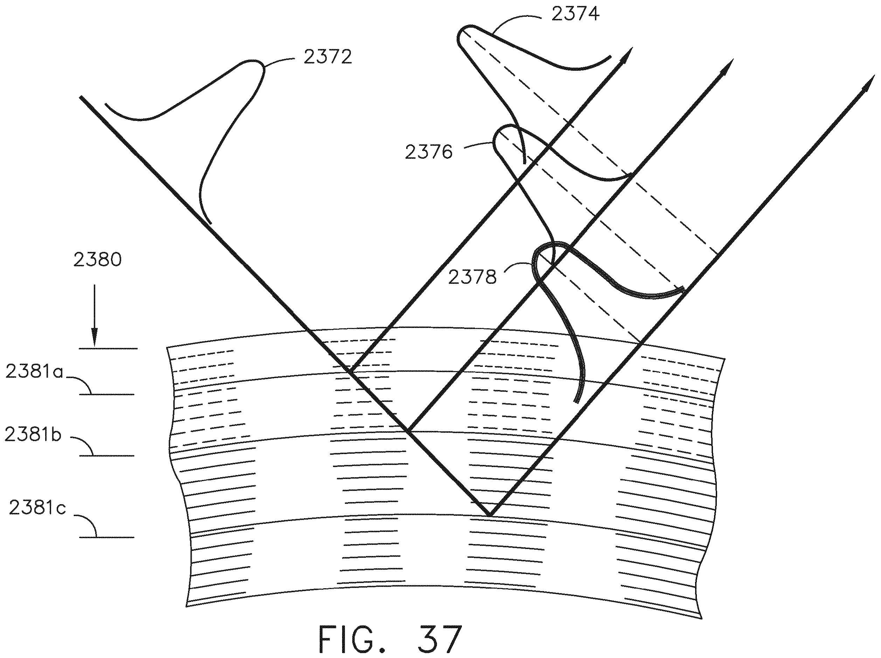

FIG. 37 illustrates that the location and characteristics of non-vascular structures are determined based on the phase difference between the incident light 2372 and the light reflected from the deep tissue structures (2374, 2376, 2378). As noted above, the penetration depth of light impinging on a tissue is dependent on the wavelength of the impinging illumination. Red laser light (having a wavelength in the range of about 635 nm to about 660 nm) penetrates the tissue to a depth of about 1 mm. Green laser light (having a wavelength in the range of about 520 nm to about 532 nm) penetrates the tissue to a depth of about 2-3 mm. Blue laser light (having a wavelength in the range of about 405 nm to about 445 nm) penetrates the tissue to a depth of about 4 mm or greater. In one aspect, an interface 2381a between two tissues differing in refractive index that is located less than or about 1 mm below a tissue surface 2380 reflects 2374 red, green, or blue laser light. The phase of the reflected light 2374 is compared to the incident light 2372 and thus the difference in the refractive index of the tissues at the interface 2381a is determined. An interface 2381b between two tissues differing in refractive index that is located between 2 and 3 mm 2381b below a tissue surface 2380 reflects 2376 green or blue laser light, but not red light. The phase of the reflected light 2376 is compared to the incident light 2372 and thus the difference in the refractive index of the tissues at the interface 2381 b is determined. In yet another aspect, an interface 2381c between two tissues differing in refractive index that is located between 3 and 4 mm 2381c below a tissue surface 2380 reflects 2378 only blue laser light, but not red or green light. The phase of the reflected light 2378 is compared to the incident light 2372 and thus the difference in the refractive index of the tissues at the interface 2381c is determined.

A phase interference measure of a tissue illuminated by light having different wavelengths may therefore provide information regarding the relative indices of refraction of the reflecting tissue as well as the depth of the tissue. The indices of refraction of the tissue may be assessed using the multiple laser sources and their intensity, and thereby relative indices of refraction may be calculated for the tissue. It is recognized that different tissues may have different refractive indices. For example, the refractive index may be related to the relative composition of collagen and elastin in a tissue or the amount of hydration of the tissue. Therefore, a technique to measure relative tissue index of refraction may result in the identification of a composition of the tissue.

In some aspects, smart surgical instruments include algorithms to determine parameters associated with the function of the instruments. One non-limiting example of such parameters may be the pressure of an anvil against a tissue for a smart stapling device. The amount of pressure of an anvil against a tissue may depend on the type and composition of the tissue. For example, less pressure may be required to staple a highly compressive tissue, while a greater amount of pressure may be required to stable a more non-compressive tissue. Another non-limiting example of a parameter associated with a smart surgical device may include a rate of firing of an i-beam knife to cut the tissue. For example, a stiff tissue may require more force and a slower cutting rate than a less stiff tissue. Another non-limiting example of such parameters may be the amount of current provided to an electrode in a smart cauterizing or RF sealing device. Tissue composition, such as percent tissue hydration, may determine an amount of current necessary to heat seal the tissue. Yet another non-limiting example of such parameters may be the amount of power provided to an ultrasonic transducer of a smart ultrasound cutting device or the driving frequency of the cutting device. A stiff tissue may require more power for cutting, and contact of the ultrasonic cutting tool with a stiff tissue may shift the resonance frequency of the cutter.

It may be recognized that a tissue visualization system that can identify tissue type and depth may provide such data to one or more smart surgical devices. The identification and location data may then be used by the smart surgical devices to adjust one or more of their operating parameters thereby allowing them to optimize their manipulation of the tissue. It may be understood that an optical method to characterize a type of tissue may permit automation of the operating parameters of the smart surgical devices. Such automation of the operation of smart surgical instruments may be preferable to relying on human estimation to determine the operational parameters of the instruments.

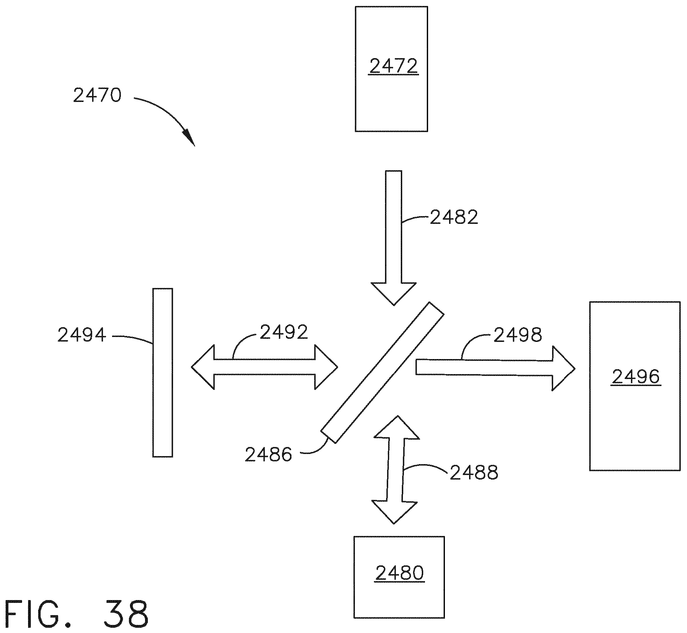

Optical Coherence Tomography (OCT) is a technique that can visual subsurface tissue structures based on the phase difference between an illuminating light source, and light reflected from structures located within the tissue. FIG. 38 depicts schematically one example of instrumentation 2470 for Optical Coherence Tomography. In FIG. 38, a laser source 2472 emits light 2482 according to any optical wavelength of interest (red, green, blue, infrared, or ultraviolet). The light 2482 is directed to a beam splitter 2486. The beam splitter 2486 directs one portion of the light 2488 to a tissue sample 2480. The beam splitter 2486 also directs a portion of the light 2492 to a stationary reference mirror 2494. The light reflected from the tissue sample 2480 and from the stationary mirror 2494 is recombined 2498 at the beam splitter 2486 and directed to a detector 2496. The phase difference between the light from the reference mirror 2494 and from the tissue sample 2480 is detected at the detector 2496 as an interference pattern. Appropriate computing devices then calculate phase information from the interference pattern. Additional computation then provides information regarding structures below the surface of the tissue sample. Additional depth information is also obtained by comparing the interference patterns generated from the sample when illuminated at different wavelengths of laser light.

As disclosed above, depth information regarding subsurface tissue structures is ascertained from a combination of laser light wavelength and the phase of light reflected from a deep tissue structure. Additionally, local tissue surface inhomogeneity is ascertained by comparing the phase as well as amplitude difference of light reflected from different portions of the same subsurface tissues. Measurements of a difference in the tissue surface properties at a defined location compared to those at a neighboring location may be indicative of adhesions, disorganization of the tissue layers, infection, or a neoplasm in the tissue being probed.

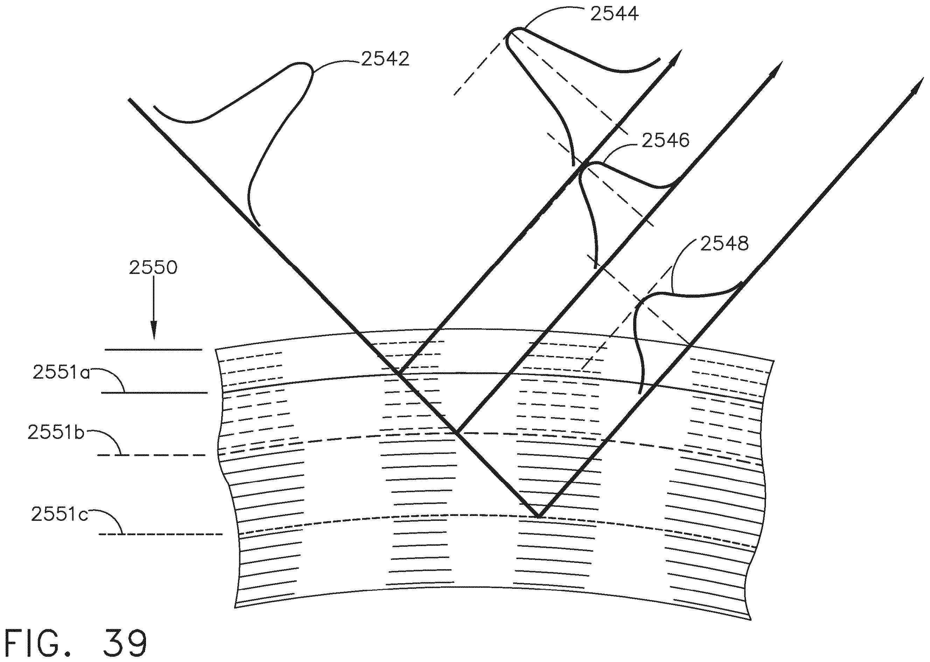

FIG. 39 illustrates this effect. The surface characteristics of a tissue determine the angle of reflection of light impinging on the surface. A smooth surface 2551a reflects the light essentially with the same spread 2544 as the light impinging on the surface 2542 (specular reflection). Consequently, the amount of light received by a light detector having a known fixed aperture effectively receives the entire amount of light reflected 2544 from the smooth surface 2551a. However, increased surface roughness at a tissue surface results in an increase spread in the reflected light with respect to the incident light (diffuse reflection).

Some amount of the reflected light 2546 from a tissue surface having some amount of surface irregularities 2551b will fall outside the fixed aperture of the light detector due to the increased spread of the reflected light 2546. As a result, the light detector will detect less light (shown in FIG. 39 as a decrease in the amplitude of the reflected light signal 2546). It is understood that the amount of reflected light spread will increase as the surface roughness of a tissue increases. Thus, as depicted in FIG. 39, the amplitude of light reflected 2548 from a surface 2551c having significant surface roughness has a smaller amplitude than the light reflected 2544 from a smooth surface 2551a, or light reflected 2546 form a surface having only a moderate amount of surface roughness 2551b. Therefore, in some aspects, a single laser source may be used to investigate the quality of a tissue surface or subsurface by comparing the optical properties of reflected light from the tissue with the optical properties of reflected light from adjacent surfaces.

In other aspects, light from multiple laser sources (for example, lasers emitting light having different central wavelengths) may be used sequentially to probe tissue surface characteristics at a variety of depths below the surface 2550. As disclosed above (with reference to FIG. 37), the absorbance profile of a laser light in a tissue is dependent on the central wavelength of the laser light. Laser light having a shorter (more blue) central wavelength can penetrate tissue deeper than laser light having a longer (more red) central wavelength. Therefore, measurements related to light diffuse reflection made at different light wavelengths can indicate both an amount of surface roughness as well as the depth of the surface being measured.

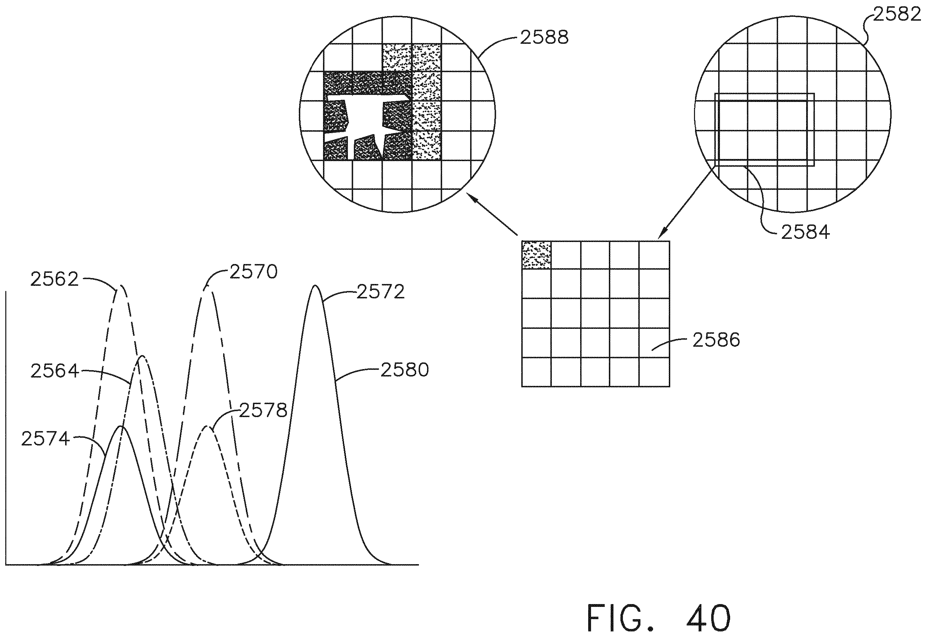

FIG. 40 illustrates one method of displaying image processing data related to a combination of tissue visualization modalities. Data used in the display is derived from image phase data related to tissue layer composition, image intensity (amplitude) data related to tissue surface features, and image wavelength data related to tissue mobility (such as blood cell transport) as well as tissue depth. As one example, light emitted by a laser in the blue optical region 2562 impinges on blood flowing at a depth of about 4 mm below the surface of the tissue. The reflected light 2564 be red shifted due to the Doppler effect of the blood flow. As a result, information is obtained regarding the existence of a blood vessel and its depth below the surface.

A layer of tissue may lie at a depth of about 2-3 mm below the surface of the surgical site. This tissue includes surface irregularities indicative of scarring or other pathologies. Emitted red light 2572 does not penetrate to the 2-3 mm depth, so consequently, the reflected red light 2580 has about the same amplitude of the emitted red light 2572 because it is unable to probe structures more than 1 mm below the top surface of the surgical site. However, green light reflected from the tissue 2578 reveals the existence of the surface irregularities at that depth in that the amplitude of the reflected green light 2578 is less than the amplitude of the emitted green light 2570. Similarly, blue light reflected from the tissue 2574 reveals the existence of the surface irregularities at that depth in that the amplitude of the reflected blue light 2574 is less than the amplitude of the emitted blue light 2562.In an image processing step, the image 2582 is smoothed using a moving window filter 2584 to reduce inter-pixel noise as well as reduce small local tissue anomalies 2586 that hides more important features 2588.

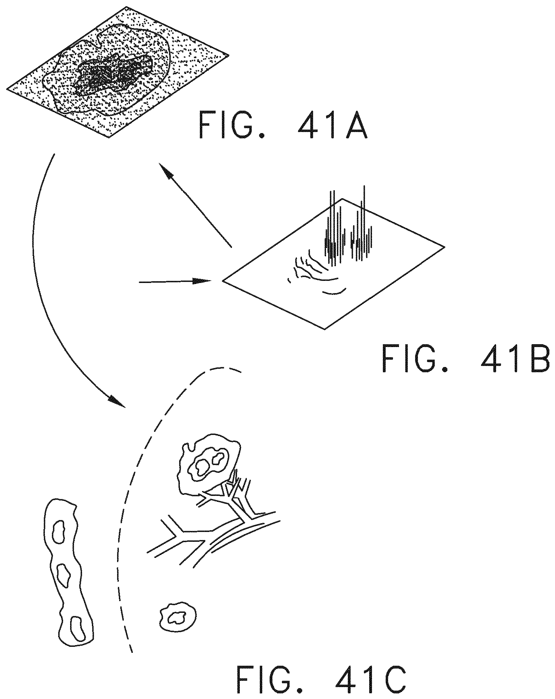

FIGS. 41A-C illustrate several aspects of displays that provides to a surgeon for a visual identification of surface and sub-surface structures of a tissue in a surgical site. FIG. 41A represents a surface map of the surgical site with color coding to indicate structures located at varying depths below the surface of the surgical site. FIG. 41B depicts an example of one of several horizontal slices through the tissue at varying depths, which are color coded to indicate depth and further include data associated with differences in tissue surface anomalies (for example, as displayed in a 3D bar graph). FIG. 41C depicts yet another visual display in which surface irregularities as well as Doppler shift flowmetry data indicate sub-surface vascular structures as well as tissue surface characteristics.

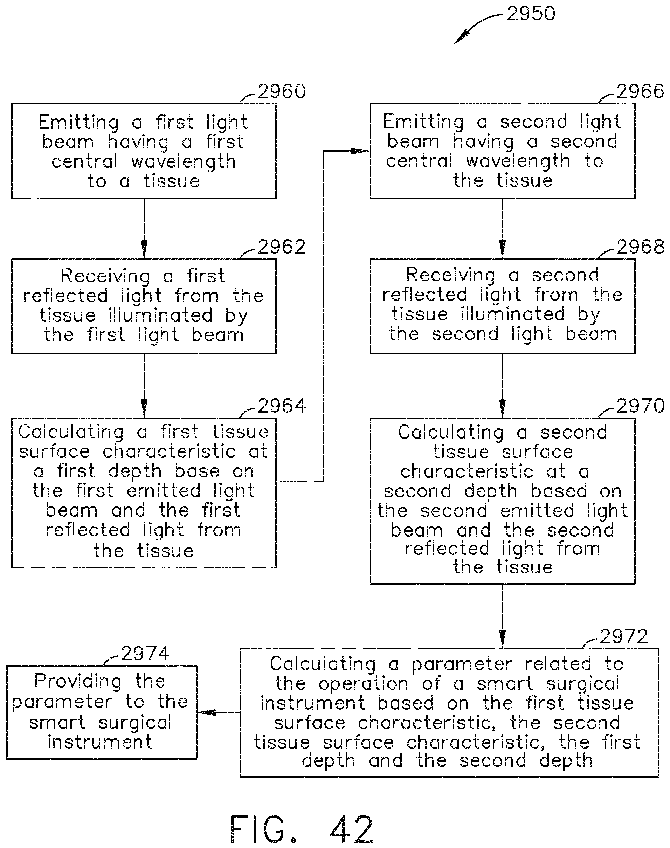

FIG. 42 is a flow chart 2950 of a method for providing information related to a characteristic of a tissue to a smart surgical instrument. An image acquisition system illuminates 2960 a tissue with a first light beam having a first central frequency and receive 2962 a first reflected light from the tissue illuminated by the first light beam. The image acquisition system then calculates 2964 a first tissue surface characteristic at a first depth based on the first emitted light beam and the first reflected light from the tissue. The image acquisition system then illuminates 2966 the tissue with a second light beam having a second central frequency and receives 2968 a second reflected light from the tissue illuminated by the second light beam. The image acquisition system then calculates 2970 a second tissue surface characteristic at a second depth based on the second emitted light beam and the second reflected light from the tissue. Tissue features that include a tissue type, a tissue composition, and a tissue surface roughness metric are determined from the first central light frequency, the second central light frequency, the first reflected light from the tissue, and the second reflected light from the tissue. The tissue characteristic is used to calculate 2972 one or more parameters related to the function of a smart surgical instrument such as jaw pressure, power to effect tissue cauterization, or current amplitude and/or frequency to drive a piezoelectric actuator to cut a tissue. In some additional examples, the parameter is transmitted 2974 either directly or indirectly to the smart surgical instrument which modifies its operating characteristics in response to the tissue being manipulated.

Multifocal Minimally Invasive Camera

In a minimally invasive procedure, e.g., laparoscopic, a surgeon may visualize the surgical site using imaging instruments including a light source and a camera. The imaging instruments may allow the surgeon to visualize the end effector of a surgical device during the procedure. However, the surgeon may need to visualize tissue away from the end effector to prevent unintended damage during the surgery. Such distant tissue may lie outside the field of view of the camera system when focused on the end effector. The imaging instrument may be moved in order to change the field of view of the camera, but it may be difficult to return the camera system back to its original position after being moved.

The surgeon may attempt to move the imaging system within the surgical site to visualize different portions of the site during the procedure. Repositioning of the imaging system is time consuming and the surgeon is not guaranteed to visualize the same field of view of the surgical site when the imaging system is returned to its original location.

It is therefore desirable to have a medical imaging visualization system that can provide multiple fields of view of the surgical site without the need to reposition the visualization system. Medical imaging devices include, without limitation, laparoscopes, endoscopes, thoracoscopes, and the like, as described herein. In some aspects, a single display system may display each of the multiple fields of view of the surgical site at about the same time. The display of each of the multiple fields of view may be independently updated depending on a display control system composed of one or more hardware modules, one or more software modules, one or more firmware modules, or any combination or combinations thereof.

Some aspects of the present disclosure further provide for a control circuit configured to control the illumination of a surgical site using one or more illumination sources such as laser light sources and to receive imaging data from one or more image sensors. In some aspects, the control circuit may be configured to control the operation of one or more light sensor modules to adjust a field of view. In some aspects, the present disclosure provides for a non-transitory computer readable medium storing computer readable instructions that, when executed, cause a device to adjust one or more components of the one or more light sensor modules and to process an image from each of the one or more light sensor modules.

An aspect of a minimally invasive image acquisition system may comprise a plurality of illumination sources wherein each illumination source is configured to emit light having a specified central wavelength, a first light sensing element having a first field of view and configured to receive illumination reflected from a first portion of the surgical site when the first portion of the surgical site is illuminated by at least one of the plurality of illumination sources, a second light sensing element having a second field of view and configured to receive illumination reflected from a second portion of the surgical site when the second portion of the surgical site is illuminated by at least one of the plurality of illumination sources, wherein the second field of view overlaps at least a portion of the first field of view; and a computing system.

The computing system may be configured to receive data from the first light sensing element, receive data from the second light sensing element, compute imaging data based on the data received from the first light sensing element and the data received from the second light sensing element, and transmit the imaging data for receipt by a display system.

A variety of surgical visualization systems have been disclosed above. Such systems provide for visualizing tissue and sub-tissue structures that may be encountered during one or more surgical procedures. Non-limiting examples of such systems may include: systems to determine the location and depth of subsurface vascular tissue such as veins and arteries; systems to determine an amount of blood flowing through the subsurface vascular tissue; systems to determine the depth of non-vascular tissue structures; systems to characterize the composition of such non-vascular tissue structures; and systems to characterize one or more surface characteristics of such tissue structures.

It is recognized that a single surgical visualization system incorporates components of any one or more of these visualization modalities. FIGS. 22A-D depict some examples of such a surgical visualization system 2108.

As disclosed above, a surgical visualization system 2108 includes an imaging control unit 2002 and a hand unit 2020. The hand unit 2020 includes a body 2021, a camera scope cable 2015 attached to the body 2021, and an elongated camera probe 2024. The elongated camera probe 2024 also terminates at its distal end with at least one window. In some non-limiting examples, a light sensor 2030 is incorporated in the hand unit 2020, for example either in the body of the hand unit 2032b, or at a distal end 2032a of the elongated camera probe, as depicted in FIG. 22C. The light sensor 2030 is fabricated using a CMOS sensor array or a CCD sensor array. As illustrated in FIG. 23C, a typical CMOS or CCD sensor array generates an RGB (red-green-blue) image from light impinging on a mosaic of sensor elements, each sensor element having one of a red, green, or blue optical filter.

Alternatively, the illumination of the surgical site may be cycled among visible illumination sources as depicted in FIG. 30D. In some example, the illumination sources may include any one or more of a red laser 2360a, a green laser 2360b, or a blue laser 2360c. In some non-limiting examples, a red laser 2360a light source may source illumination having a peak wavelength that may range between 635 nm and 660 nm, inclusive. Non-limiting examples of a red laser peak wavelength may include about 635 nm, about 640 nm, about 645 nm, about 650 nm, about 655 nm, about 660 nm, or any value or range of values therebetween. In some non-limiting examples, a green laser 2360b light source may source illumination having a peak wavelength that may range between 520 nm and 532 nm, inclusive. Non-limiting examples of a red laser peak wavelength may include about 520 nm, about 522 nm, about 524 nm, about 526 nm, about 528 nm, about 530 nm, about 532 nm, or any value or range of values therebetween. In some non-limiting examples, the blue laser 2360c light source may source illumination having a peak wavelength that may range between 405 nm and 445 nm, inclusive. Non-limiting examples of a blue laser peak wavelength may include about 405 nm, about 410 nm, about 415 nm, about 420 nm, about 425 nm, about 430 nm, about 435 nm, about 440 nm, about 445 nm, or any value or range of values therebetween.

Additionally, illumination of the surgical site may be cycled to include non-visible illumination sources that may supply infra red or ultraviolet illumination. In some non-limiting examples, an infra red laser light source may source illumination having a peak wavelength that may range between 750 nm and 3000 nm, inclusive. Non-limiting examples of an infra red laser peak wavelength may include about 750 nm, about 1000 nm, about 1250 nm, about 1500 nm, about 1750 nm, about 2000 nm, about 2250 nm, about 2500 nm, about 2750 nm, 3000 nm, or any value or range of values therebetween. In some non-limiting examples, an ultraviolet laser light source may source illumination having a peak wavelength that may range between 200 nm and 360 nm, inclusive. Non-limiting examples of an ultraviolet laser peak wavelength may include about 200 nm, about 220 nm, about 240 nm, about 260 nm, about 280 nm, about 300 nm, about 320 nm, about 340 nm, about 360 nm, or any value or range of values therebetween.

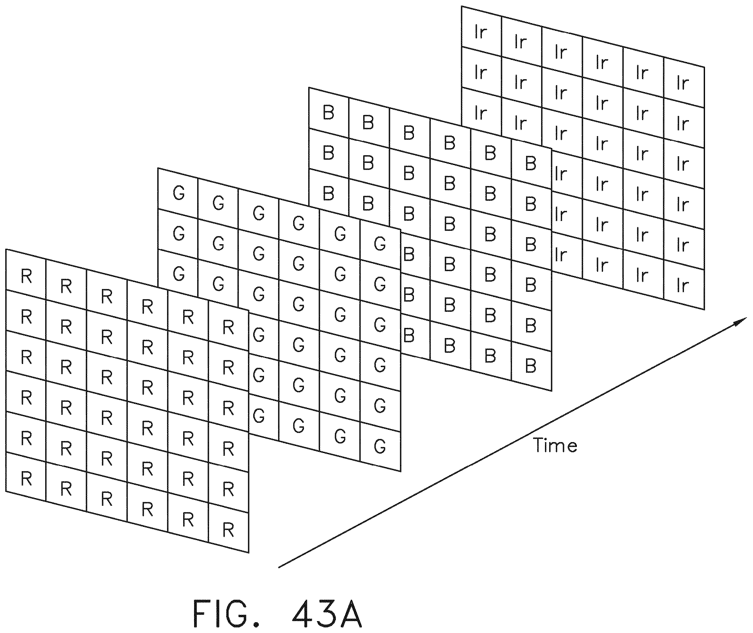

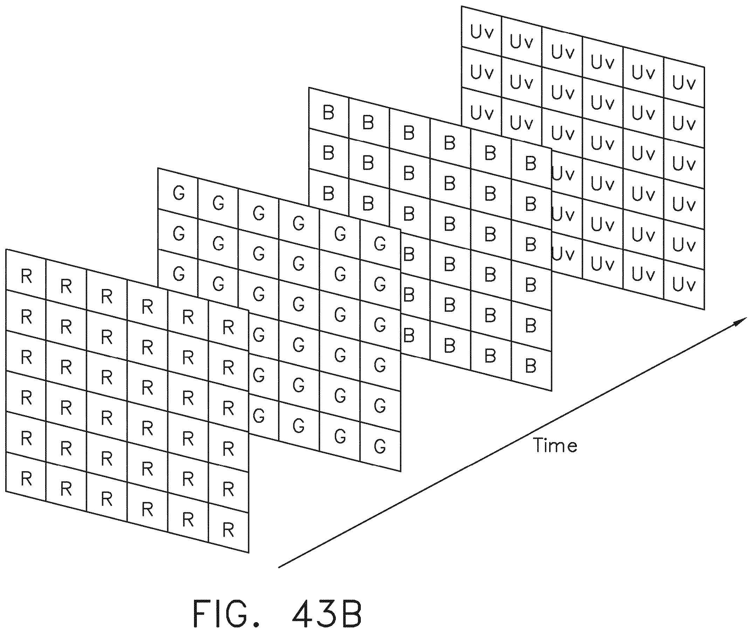

The outputs of the sensor array under the different illumination wavelengths are combined to form the RGB image, for example, if the illumination cycle time is sufficiently fast and the laser light is in the visible range. FIGS. 43A and 43B illustrate a multi-pixel light sensor receiving by light reflected by a tissue illuminated, for example, by sequential exposure to red, green, blue, infra red, (FIG. 43A) or red, green, blue, and ultraviolet laser light sources (FIG. 43B).

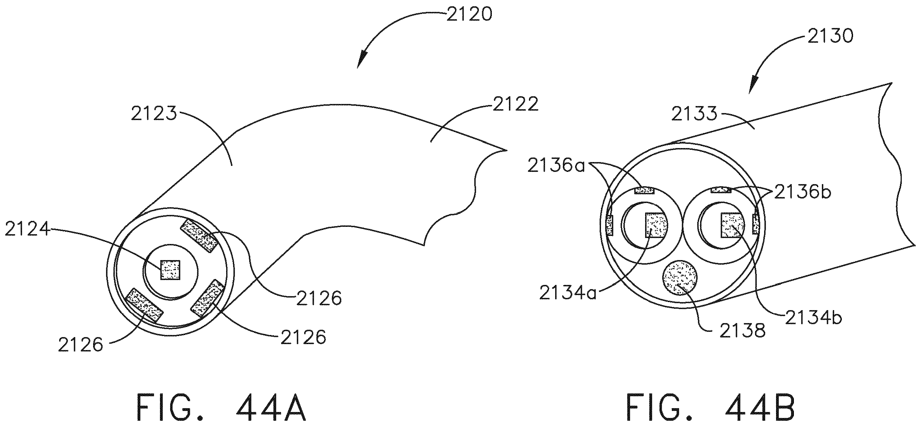

FIG. 44A depicts the distal end of a flexible elongated camera probe 2120 having a flexible camera probe shaft 2122 and a single light sensor module 2124 disposed at the distal end 2123 of the flexible camera probe shaft 2122. In some non-limiting examples, the flexible camera probe shaft 2122 may have an outer diameter of about 5 mm. The outer diameter of the flexible camera probe shaft 2122 may depend on geometric factors that may include, without limitation, the amount of allowable bend in the shaft at the distal end 2123. As depicted in FIG. 44A, the distal end 2123 of the flexible camera probe shaft 2122 may bend about 90° with respect to a longitudinal axis of an un-bent portion of the flexible camera probe shaft 2122 located at a proximal end of the elongated camera probe 2120. It may be recognized that the distal end 2123 of the flexible camera probe shaft 2122 may bend any appropriate amount as may be required for its function. Thus, as non-limiting examples, the distal end 2123 of the flexible camera probe shaft 2122 may bend any amount between about 0° and about 90°. Non-limiting examples of the bend angle of the distal end 2123 of the flexible camera probe shaft 2122 may include about 0°, about 10°, about 20°, about 30°, about 40°, about 50°, about 60°, about 70°, about 80°, about 90°, or any value or range of values therebetween. In some examples, the bend angle of the distal end 2123 of the flexible camera probe shaft 2122 may be set by a surgeon or other health care professional prior to or during a surgical procedure. In some other example, the bend angle of the distal end 2123 of the flexible camera probe shaft 2122 may be a fixed angle set at a manufacturing site.

The single light sensor module 2124 may receive light reflected from the tissue when illuminated by light emitted by one or more illumination sources 2126 disposed at the distal end of the elongated camera probe. In some examples, the light sensor module 2124 may be a 4 mm sensor module such as 4 mm mount 2136b, as depicted in FIG. 22D. It may be recognized that the light sensor module 2124 may have any appropriate size for its intended function. Thus, the light sensor module 2124 may include a 5.5 mm mount 2136a, a 2.7 mm mount 2136c, or a 2 mm mount 2136d as depicted in FIG. 22D.

It may be recognized that the one or more illumination sources 2126 may include any number of illumination sources 2126 including, without limitation, one illumination source, two illumination sources, three illumination sources, four illumination sources, or more than four illumination sources. It may be further understood that each illumination source may source illumination having any central wavelength including a central red illumination wavelength, a central green illumination wavelength, a central blue illumination wavelength, a central infrared illumination wavelength, a central ultraviolet illumination wavelength, or any other wavelength. In some examples, the one or more illumination sources 2126 may include a white light source, which may illuminate tissue with light having wavelengths that may span the range of optical white light from about 390 nm to about 700 nm.

FIG. 44B depicts the distal end 2133 of an alternative elongated camera probe 2130 having multiple light sensor modules, for example the two light sensor modules 2134a,b, each disposed at the distal end 2133 of the elongated camera probe 2130. In some non-limiting examples, the alternative elongated camera probe 2130 may have an outer diameter of about 7 mm. In some examples, the light sensor modules 2134a,b may each comprise a 4mm sensor module, similar to light sensor module 2124 in FIG. 44A. Alternatively, each of the light sensor modules 2134a,b may comprise a 5.5 mm light sensor module, a 2.7 mm light sensor module, or a 2 mm light sensor module as depicted in FIG. 22D. In some examples, both light sensor modules 2134a,b may have the same size. In some examples, the light sensor modules 2134a,b may have different sizes. As one non-limiting example, an alternative elongated camera probe 2130 may have a first 4 mm light sensor and two additional 2 mm light sensors. In some aspects, a visualization system may combine the optical outputs from the multiple light sensor modules 2134a,b to form a 3D or quasi-3D image of the surgical site. In some other aspects, the outputs of the multiple light sensor modules 2134a,b may be combined in such a manner as to enhance the optical resolution of the surgical site, which may not be otherwise practical with only a single light sensor module.

Each of the multiple light sensor modules 2134a,b receives light reflected from the tissue when illuminated by light emitted by one or more illumination sources 2136a,b disposed at the distal end 2133 of the alternative elongated camera probe 2130. The light emitted by all of the illumination sources 2136a,b is derived from the same light source (such as a laser). The illumination sources 2136a surrounding a first light sensor module 2134a emit light at a first wavelength and the illumination sources 2136b surrounding a second light sensor module 2134b emit light at a second wavelength. It is further understood that each illumination source 2136a,b has source illumination having any central wavelength including a central red illumination wavelength, a central green illumination wavelength, a central blue illumination wavelength, a central infrared illumination wavelength, a central ultraviolet illumination wavelength, or any other wavelength. The one or more illumination sources 2136a,b include a white light source, which illuminates tissue with light having wavelengths that spans the range of optical white light from about 390 nm to about 700 nm.

The distal end 2133 of the alternative elongated camera probe 2130 includes one or more working channels 2138. Such working channels 2138 are in fluid communication with an aspiration port of a device to aspirate material from the surgical site, thereby permitting the removal of material that potentially obscures the field of view of the light sensor modules 2134a,b. Alternatively, such working channels 2138 are in fluid communication with an fluid source port of a device to provide a fluid to the surgical site, to flush debris or material away from the surgical site. Such fluids are used to clear material from the field of view of the light sensor modules 2134a,b.

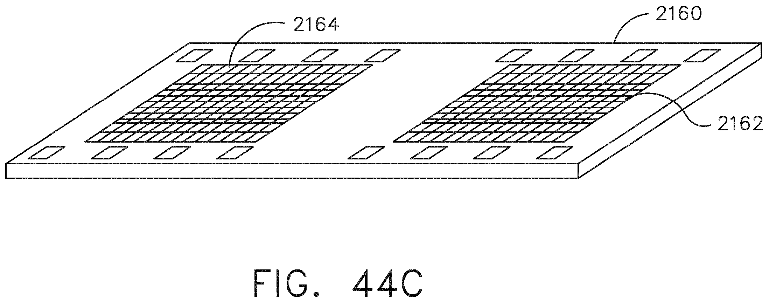

FIG. 44C depicts a perspective view of an aspect of a monolithic sensor 2160 having a plurality of pixel arrays for producing a three dimensional image in accordance with the teachings and principles of the disclosure. Such an implementation is desirable for three dimensional image capture, wherein the two pixel arrays 2162 and 2164 are offset during use. A first pixel array 2162 and a second pixel array 2164 are dedicated to receiving a predetermined range of wave lengths of electromagnetic radiation, wherein the first pixel array 2162 is dedicated to a different range of wave length electromagnetic radiation than the second pixel array 2164.

Additional disclosures regarding a dual sensor array may be found in U.S. Patent Application Publication No. 2014/0267655, titled SUPER RESOLUTION AND COLOR MOTION ARTIFACT CORRECTION IN A PULSED COLOR IMAGING SYSTEM, filed on March 14, 2014, which issued on May 2, 2017 as U.S. Patent No. 9,641,815.

In some aspects, a light sensor module comprises a multi-pixel light sensor such as a CMOS array in addition to one or more additional optical elements such as a lens, a reticle, and a filter.

In some alternative aspects, the one or more light sensors may be located within the body 2021 of the hand unit 2020. Light reflected from the tissue may be acquired at a light receiving surface of one or more optical fibers at the distal end of the elongated camera probe 2024. The one or more optical fibers may conduct the light from the distal end of the elongated camera probe 2024 to the one or more light sensors, or to additional optical elements housed in the body of the hand unit 2020 or in the imaging control unit 2002. The additional optical elements may include, without limitation, one or more dichroic mirrors, one or more reference mirrors, one or more moving mirrors, and one or more beam splitters and/or combiners, and one or more optical shutters. In such alternative aspects, the light sensor module may include any one or more of a lens, a reticle and a filter, disposed at the distal end of the elongated camera probe 2024.

Images obtained from each of the multiple light sensors for example 2134a,b may be combined or processed in several different manners, either in combination or separately, and then displayed in a manner to allow a surgeon to visualize different aspects of the surgical site.

In one non-limiting example, each light sensor may have an independent field of view. In some additional examples, the field of view of a first light sensor may partially or completely overlap the field of view of a second light sensor.

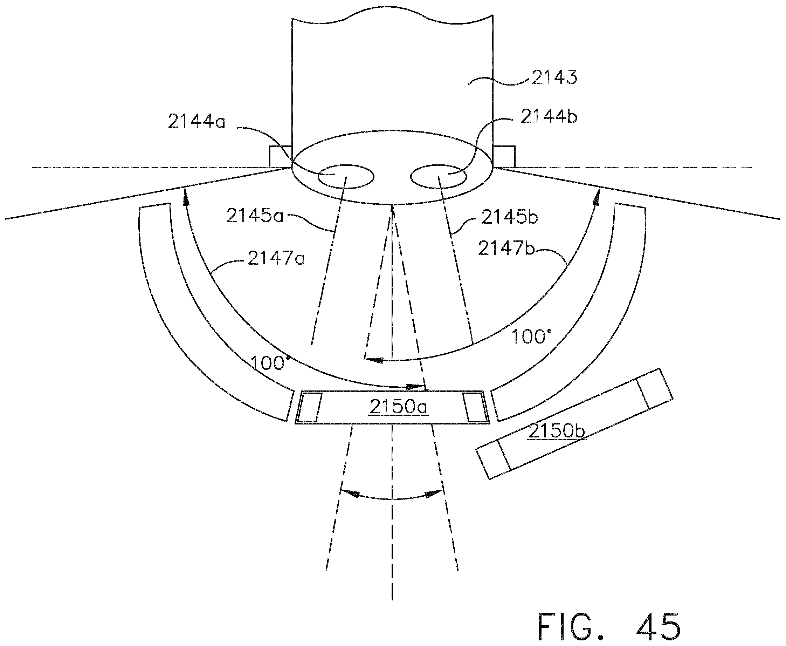

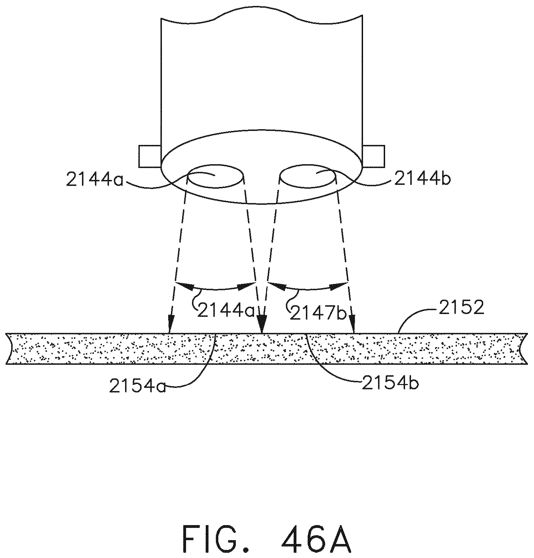

As disclosed above, an imaging system may include a hand unit 2020 having an elongated camera probe 2024 with one or more light sensor modules 2124, 2134a,b disposed at its distal end 2123, 2133. The elongated camera probe 2024 has two light sensor modules 2134a,b, although it may be recognized that there may be three, four, five, or more light sensor modules at the distal end of the elongated camera probe 2024. Although FIGS. 45 and 46A-D depict examples of the distal end of an elongated camera probe having two light sensor modules, it is recognized that the description of the operation of the light sensor modules is not limited to solely two light sensor modules. As depicted in FIGS. 45, and 46A-D, the light sensor modules include an image sensor, such as a CCD or CMOS sensor that is composed of an array of light sensing elements (pixels). The light sensor modules also includes additional optical elements, such as lenses. Each lens is adapted to provide a field of view for the light sensor of the respective light sensor module.

FIG. 45 depicts a generalized view of a distal end 2143 of an elongated camera probe having multiple light sensor modules 2144a,b. Each light sensor module 2144a,b is composed of a CCD or CMOS sensor and one or more optical elements such as filters, lenses, shutters, and similar. The components of the light sensor modules 2144a,b are fixed within the elongated camera probe. One or more of the components of the light sensor modules 2144a,b is/are adjustable. For example, the CCD or CMOS sensor of a light sensor module 2144a,b is mounted on a movable mount to permit automated adjustment of the center 2145a,b of a field of view 2147a,b of the CCD or CMOS sensor. The CCD or CMOS sensor is fixed, but a lens in each light sensor modules 2144a,b is adjustable to change the focus. The light sensor modules 2144a,b include adjustable irises to permit changes in the visual aperture of the sensor modules 2144a,b.

As depicted in FIG. 45, each of the sensor modules 2144a,b has a field of view 2147a,b having an acceptance angle. As depicted in FIG. 45, the acceptance angle for each sensor modules 2144a,b has an acceptance angle of greater than 90°. The acceptance angle is about 100°. The acceptance angle may be about 120°. If the sensor modules 2144a,b has an acceptance angle of greater than 90° (for example, 100°), the fields of view 2147a and 2147b form an overlap region 2150a,b. An optical field of view having an acceptance angle of 100° or greater may be called a "fish-eyed" field of view. A visualization system control system associated with such an elongated camera probe includes computer readable instructions that may permit the display of the overlap region 2150a,b in such a manner so that the extreme curvature of the overlapping fish-eyed fields of view is corrected, and a sharpened and flattened image is displayed. In FIG. 45, the overlap region 2150a represents a region wherein the overlapping fields of view 2147a,b of the sensor modules 2144a,b have their respective centers 2145a,b directed in a forward direction. However, if any one or more components of the sensor modules 2144a,b is adjustable, it is recognized that the overlap region 2150b is directed to any attainable angle within the fields of view 2147a,b of the sensor modules 2144a,b.

FIGS. 46A-D depict a variety of examples of an elongated light probe having two light sensor modules 2144a,b with a variety of fields of view. The elongated light probe is directed to visualize a surface 2152 of a surgical site.

In FIG. 46A, the first light sensor module 2144a has a first sensor field of view 2147a of a tissue surface 2154a, and the second light sensor module 2144b has a second sensor field of view 2147b of a tissue surface 2154b. As depicted in FIG. 46A, the first field of view 2147a and the second field of view 2147b have approximately the same angle of view. Additionally, the first sensor field of view 2147a is adjacent to but does not overlap the second sensor field of view 2147b. The image received by the first light sensor module 2144a is displayed separately from the image received by the second light sensor module 2144b, or the images are combined to form a single image. The angle of view of a lens associated with the first light sensor module 2144a and the angle of view of a lens associated with the second light sensor module 2144b is somewhat narrow, and image distortion may not be great at the periphery of their respective images. Therefore, the images may be easily combined edge to edge.

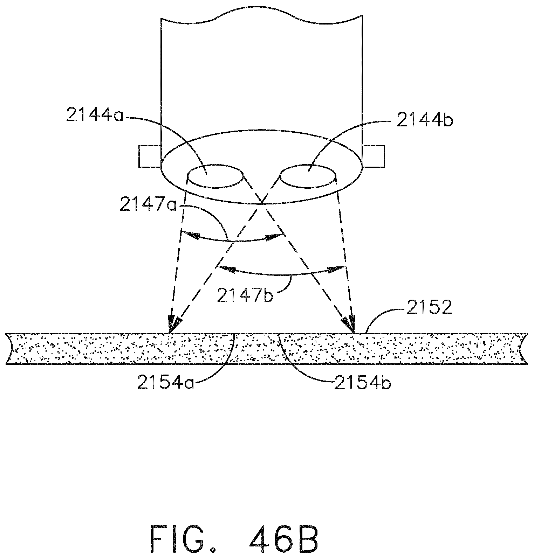

As depicted in FIG. 46B, the first field of view 2147a and the second field of view 2147b have approximately the same angular field of view, and the first sensor field of view 2147a overlaps completely the second sensor field of view 2147b. This results in a first sensor field of view 2147a of a tissue surface 2154a being identical to the view of a tissue surface 2154b as obtained by the second light sensor module 2144b from the second sensor field 2147b of view. This configuration is useful for applications in which the image from the first light sensor module 2144a is processed differently than the image from the second light sensor module 2144b. The information in the first image complements the information in the second image and refers to the same portion of tissue.

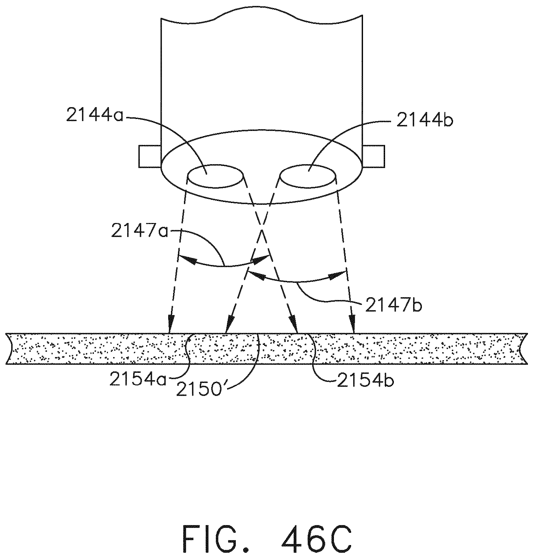

As depicted in FIG. 46C, the first field of view 2147a and the second field of view 2147b have approximately the same angular field of view, and the first sensor field of view 2147a partially overlaps the second sensor field of view 2147b. A lens associated with the first light sensor module 2144a and a lens associated with the second light sensor module 2144b may be wide angle lenses. These lenses permit the visualization of a wider field of view than that depicted in FIG. 46A. Wide angle lenses are known to have significant optical distortion at their periphery. Appropriate image processing of the images obtained by the first light sensor module 2144a and the second light sensor module 2144b permits the formation of a combined image in which the central portion of the combined image is corrected for any distortion induced by either the first lens or the second lens. It is understood that a portion of the first sensor field of view 2147a of a tissue surface 2154a thus has some distortion due to the wide angle nature of a lens associated with the first light sensor module 2144a and a portion of the second sensor field of view 2147b of a tissue surface 2154b thus has some distortion due to the wide angle nature of a lens associated with the second light sensor module 2144b. However, a portion of the tissue viewed in the overlap region 2150' of the two light sensor modules 2144a,b is corrected for any distortion induced by either of the light sensor modules 2144a,b. The configuration depicted in FIG. 46C is useful for applications in which it is desired to have a wide field of view of the tissue around a portion of a surgical instrument during a surgical procedure. Lenses associated with each light sensor module 2144a,b are independently controllable, thereby controlling the location of the overlap region 2150' of view within the combined image.

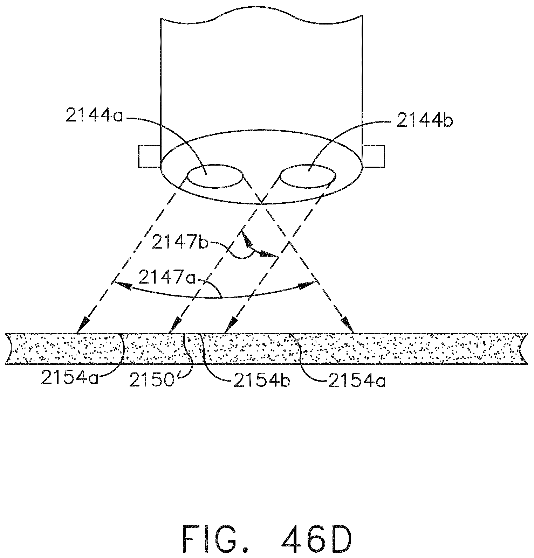

As depicted in FIG. 46D, the first light sensor module 2144a has a first angular field of view 2147a that is wider than the second angular field of view 2147b of the second light sensor module 2144b. The second sensor field of view 2147b is totally disposed within the first sensor field of view 2147a. Alternatively, the second sensor field of view lies outside of or tangent to the wide angle field of view 2147a of the first sensor 2144a. A display system that uses the configuration depicted in FIG. 46D displays a wide angle portion of tissue 2154a imaged by the first sensor module 2144a along with a magnified second portion of tissue 2154b imaged by the second sensor module 2144b and located in an overlap region 2150" of the first field of view 2147a and the second field of view 2147b. This configuration is useful to present a surgeon with a close-up image of tissue proximate to a surgical instrument (for example, imbedded in the second portion of tissue 2154b) and a wide-field image of the tissue surrounding the immediate vicinity of the medical instrument (for example, the proximal first portion of tissue 2154a). In some non-limiting examples, the image presented by the narrower second field of view 2147b of the second light sensor module 2144b is a surface image of the surgical site. The image presented in the first wide field view 2147a of the first light sensor module 2144a includes a display based on a hyperspectral analysis of the tissue visualized in the wide field view.

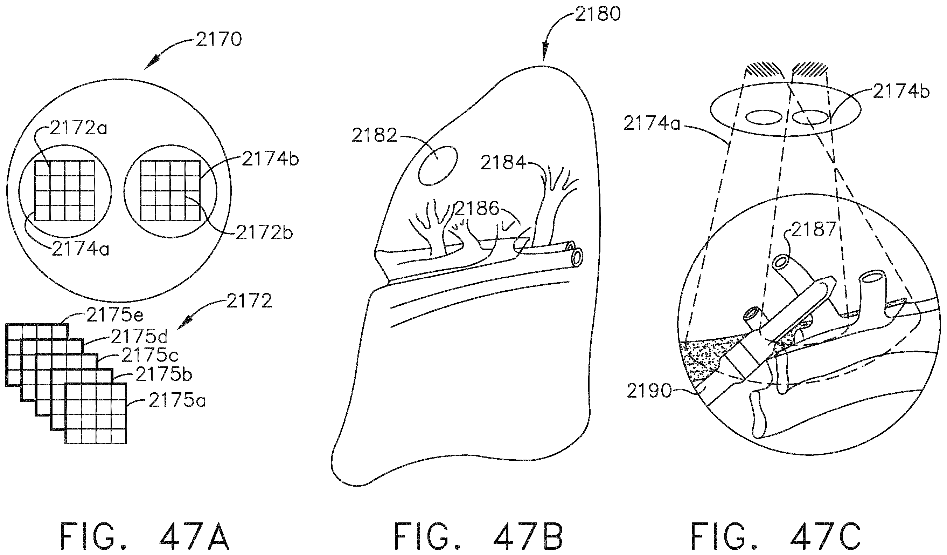

FIGS. 47A-C illustrate an example of the use of an imaging system incorporating the features disclosed in FIG 46D. FIG. 47A illustrates schematically a proximal view 2170 at the distal end of the elongated camera probe depicting the light sensor arrays 2172a,b of the two light sensor modules 2174a,b. A first light sensor module 2174a may include a wide angle lens, and the second light sensor module 2174b may include a narrow angle lens. In some aspects, the second light sensor module 2174b may have a narrow aperture lens. In other aspects, the second light sensor module 2174b may have a magnifying lens. The tissue may be illuminated by the illumination sources disposed at the distal end of the elongated camera probe. The light sensor arrays 2172' (either light sensor array 2172a or 2172b, or both 2172a and 2172b) may receive the light reflected from the tissue upon illumination. The tissue may be illuminated by light from a red laser source, a green laser source, a blue laser source, an infra red laser source, and/or an ultraviolet laser source. In some aspects, the light sensor arrays 2172' may sequentially receive the red laser light 2175a, green laser light 2175b, blue laser light 2175c, infrared laser light 2175d, and the ultra-violet laser light 2175e. The tissue may be illuminated by any combination of such laser sources simultaneously, as depicted in FIGS. 23E and 23F. Alternatively, the illuminating light may be cycled among any combination of such laser sources, as depicted for example in FIGS. 23D, and FIGS. 43A and 43B.

FIG. 47B schematically depicts a portion of lung tissue 2180 which contains a tumor 2182. The tumor 2182 is in communication with blood vessels including one or more veins 2184 and/or arteries 2186. In some surgical procedures, the blood vessels (veins 2184 and arteries 2186) associated with the tumor 2182 require resection and/or cauterization prior to the removal of the tumor.

FIG. 47C illustrates the use of a dual imaging system as disclosed above with respect to FIG. 47A. The first light sensor module 2174a acquires a wide angle image of the tissue surrounding a blood vessel 2187 to be severed with a surgical knife 2190. The wide angle image permits the surgeon to verify the blood vessel to be severed 2187. In addition, the second light sensor module 2174b acquires a narrow angle image of the specific blood vessel 2187 to be manipulated. The narrow angle image shows the surgeon the progress of the manipulation of the blood vessel 2187. In this manner, the surgeon is presented with the image of the vascular tissue to be manipulated as well as its environs to assure that the correct blood vessel is being manipulated.

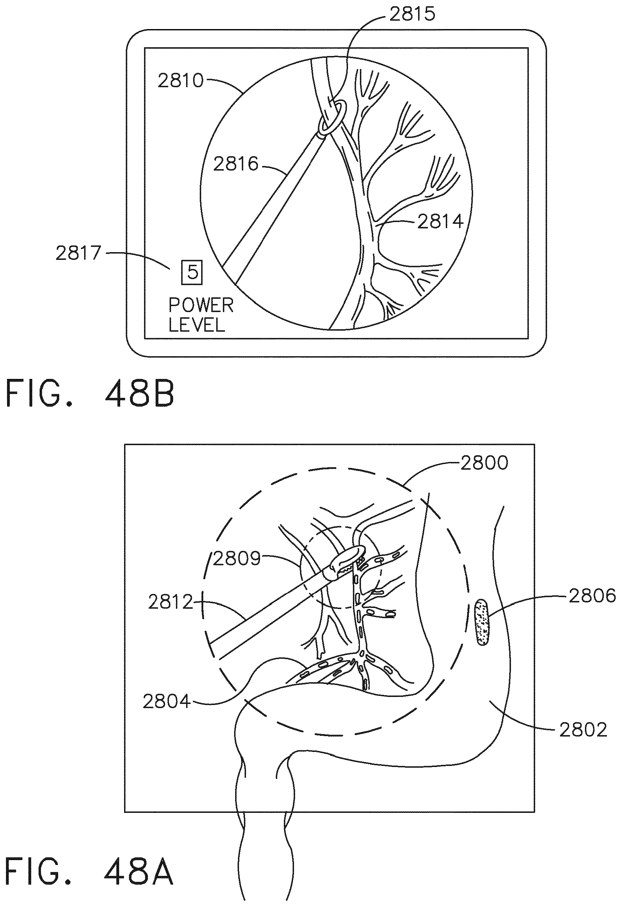

FIGS. 48A and 48B depict another example of the use of a dual imaging system. FIG. 48A depicts a primary surgical display providing an image of a section of a surgical site. The primary surgical display depicts a wide view image 2800 of a section of intestine 2802 along with its vasculature 2804. The wide view image 2800 includes a portion of the surgical field 2809 that is separately displayed as a magnified view 2810 in a secondary surgical display (FIG. 48B). As disclosed above with respect to surgery to remove a tumor from a lung (FIGS. 47A-C), it is necessary to dissect blood vessels supplying a tumor 2806 before removing the cancerous tissue. The vasculature 2804 supplying the intestines 2802 is complex and highly ramified. It is necessary to determine which blood vessels supply the tumor 2806 and to identify blood vessels supplying blood to healthy intestinal tissue. The wide view image 2800 permits a surgeon to determine which blood vessel suppliesy the tumor 2806. The surgeon then tests a blood vessel using a clamping device 2812 to determine if the blood vessel supplies the tumor 2806 or not.

FIG. 48B depicts a secondary surgical display that only displays a narrow magnified view image 2810 of one portion of the surgical field 2809. The narrow magnified view image 2810 presents a close-up view of the vascular tree 2814 so that the surgeon can focus on dissecting only the blood vessel of interest 2815. For resecting the blood vessel of interest 2815, a surgeon uses a smart RF cautery device 2816. It is understood that any image obtained by the visualization system includes not only images of the tissue in the surgical site but also images of the surgical instruments inserted therein. Such a surgical display (either the primary display in FIG. 48A or the secondary display in FIG. 48B) also includes indicia 2817 related to functions or settings of any surgical device used during the surgical procedure. For example, the indicia 2817 includes a power setting of the smart RF cautery device 2816. Such smart medical devices may transmit data related to their operating parameters to the visualization system to incorporate in display data to be transmitted to one or more display devices.

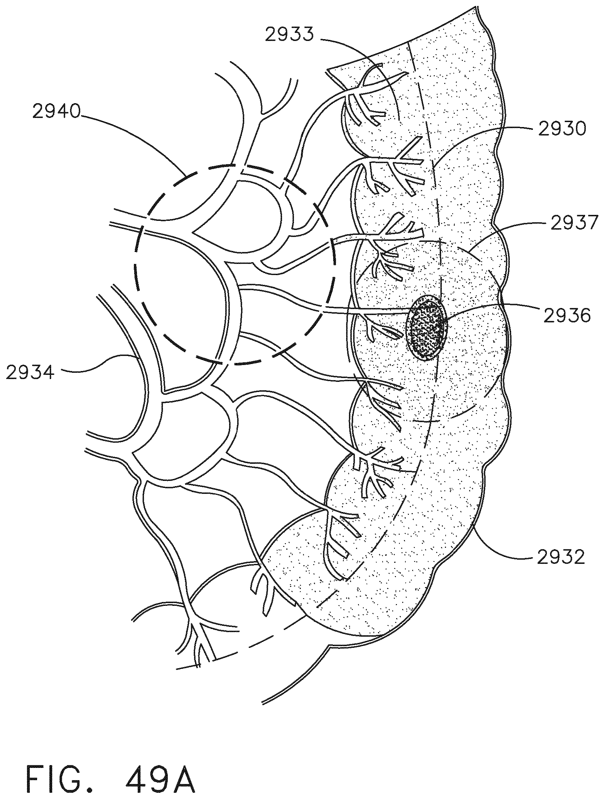

FIGS. 49A-C illustrate examples of a sequence of surgical steps for the removal of an intestinal/colon tumor and which benefit from the use of multi-image analysis at the surgical site. FIG. 49A depicts a portion of the surgical site, including the intestines 2932 and the ramified vasculature 2934 supplying blood and nutrients to the intestines 2932. The intestines 2932 have a tumor 2936 surrounded by a tumor margin 2937. A first light sensor module of a visualization system has a wide field of view 2930, and it provides imaging data of the wide field of view 2930 to a display system. A second light sensor module of the visualization system may has a narrow or standard field of view 2940, and it provides imaging data of the narrow field of view 2940 to the display system. The wide field image and the narrow field image are displayed by the same display device. Alternatively, the wide field image and the narrow field image are displayed by separate display devices.

During the surgical procedure, it is important to remove not just the tumor 2936 but the margin 2937 surrounding it to assure complete removal of the tumor. A wide angle field of view 2930 is used to image both the vasculature 2934 as well as the section of the intestines 2932 surrounding the tumor 2936 and the margin 2637. As noted above, the vasculature feeding the tumor 2936 and the margin 2637 should be removed, but the vasculature feeding the surrounding intestinal tissue must be preserved to provide oxygen and nutrients to the surrounding tissue. Transection of the vasculature feeding the surrounding colon tissue will remove oxygen and nutrients from the tissue, leading to necrosis. Laser Doppler imaging of the tissue visualized in the wide angle field 2630 is analyzed to provide a speckle contrast analysis 2933, indicating the blood flow within the intestinal tissue.

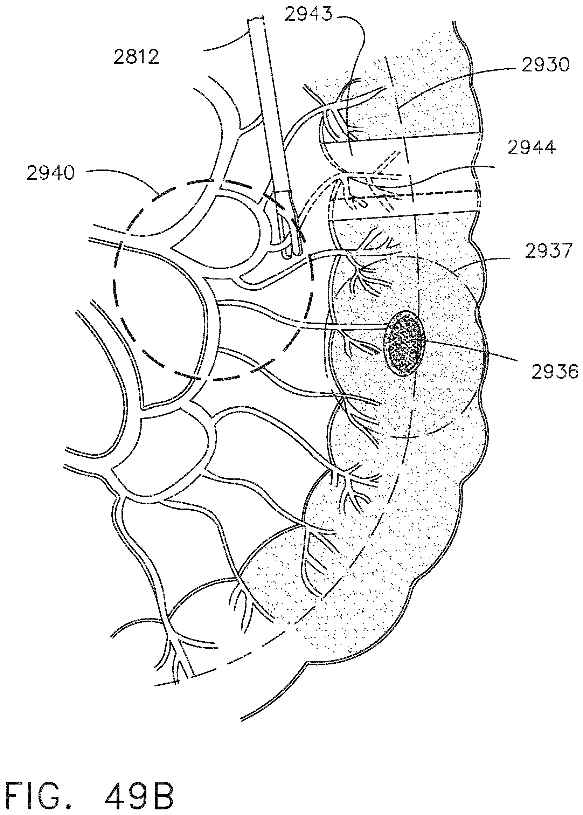

FIG. 49B illustrates a step during the surgical procedure. The surgeon is uncertain which part of the vascular tree supplies blood to the tumor 2936. The surgeon tests a blood vessel 2944 to determine if it feeds the tumor 2936 or the healthy tissue. The surgeon clamps a blood vessel 2944 with a clamping device 2812 and determines the section of the intestinal tissue 2943 that is no longer perfused by means of the speckle contrast analysis. The narrow field of view 2940 displayed on an imaging device assists the surgeon in the close-up and detailed work required to visualize the single blood vessel 2944 to be tested. When the suspected blood vessel 2944 is clamped, a portion of the intestinal tissue 2943 is determined to lack perfusion based on the Doppler imaging speckle contras analysis. As depicted in FIG. 29B, the suspected blood vessel 2944 does not supply blood to the tumor 2935 or the tumor margin 2937, and therefore is recognized as a blood vessel to be spared during the surgical procedure.

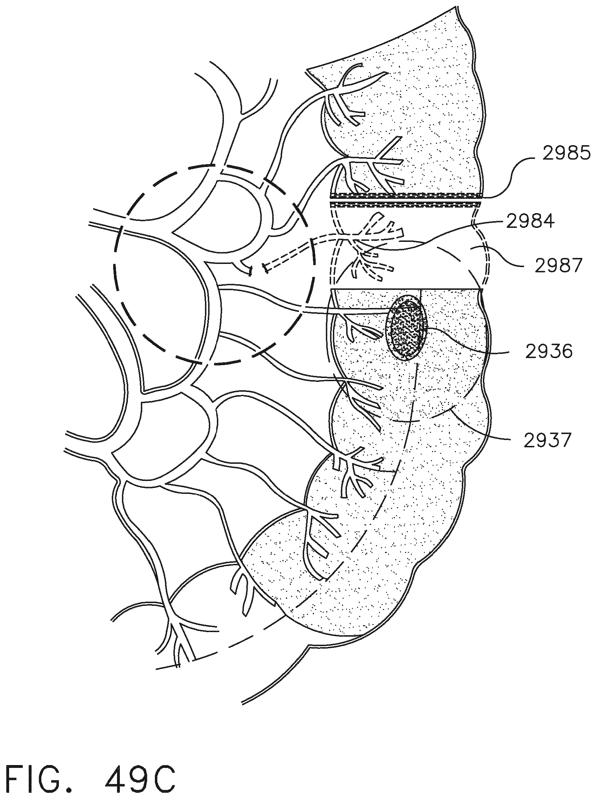

FIG. 49C depicts a following stage of the surgical procedure. In stage, a supply blood vessel 2984 has been identified to supply blood to the margin 2937 of the tumor. When this supply blood vessel 2984 has been severed, blood is no longer supplied to a section of the intestine 2987 that includes at least a portion of the margin 2937 of the tumor 2936. The lack of perfusion to the section 2987 of the intestines is determined by means of a speckle contrast analysis based on a Doppler analysis of blood flow into the intestines. The non-perfused section 2987 of the intestines is then isolated by a seal 2985 applied to the intestine. In this manner, only those blood vessels perfusing the tissue indicated for surgical removal are identified and sealed, thereby sparing healthy tissue from unintended surgical consequences.

In some additional aspects, a surgical visualization system may permit imaging analysis of the surgical site.

In some aspects, the surgical site may be inspected for the effectiveness of surgical manipulation of a tissue. Non-limiting examples of such inspection may include the inspection of surgical staples or welds used to seal tissue at a surgical site. Cone beam coherent tomography using one or more illumination sources may be used for such methods.

In some additional aspects, an image of a surgical site may have landmarks denoted in the image. In some examples, the landmarks may be determined through image analysis techniques. In some alternative examples, the landmarks may be denoted through a manual intervention of the image by the surgeon.

In some additional aspects, non-smart ready visualizations methods may be imported for used in Hub image fusion techniques.

In additional aspects, instruments that are not integrated in the Hub system may be identified and tracked during their use within the surgical site. In this aspect, computational and/or storage components of the Hub or in any of its components (including, for example, in the cloud system) may include a database of images related to EES and competitive surgical instruments that are identifiable from one or more images acquired through any image acquisition system or through visual analytics of such alternative instruments. The imaging analysis of such devices may further permit identification of when an instrument is replaced with a different instrument to do the same or a similar job. The identification of the replacement of an instrument during a surgical procedure may provide information related to when an instrument is not doing the job or a failure of the device.

Situational Awareness

Situational awareness is the ability of a surgical system to determine or infer information related to a surgical procedure from data received from databases and/or instruments. The information includes the type of procedure being undertaken, the type of tissue being operated on, or the body cavity that is the subject of the procedure. With the contextual information related to the surgical procedure, the surgical system improves the manner in which it controls the modular devices (e.g. a robotic arm and/or robotic surgical tool) that are connected to it and provides contextualized information or suggestions to the surgeon during the course of the surgical procedure.

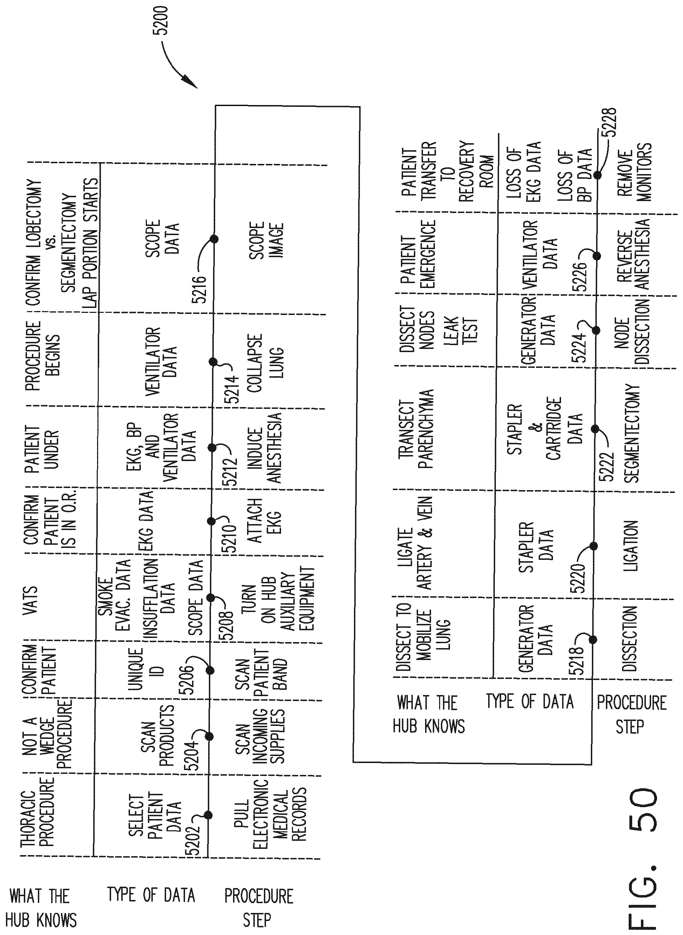

Referring now to FIG. 50, a timeline 5200 depicting situational awareness of a hub, such as the surgical hub 106 or 206, for example, is depicted. The timeline 5200 is an illustrative surgical procedure and the contextual information that the surgical hub 106, 206 derives from the data received from the data sources at each step in the surgical procedure. The timeline 5200 depicts the typical steps that would be taken by the nurses, surgeons, and other medical personnel during the course of a lung segmentectomy procedure, beginning with setting up the operating theater and ending with transferring the patient to a post-operative recovery room.

The situationally aware surgical hub 106, 206 receives data from the data sources throughout the course of the surgical procedure, including data generated each time medical personnel utilize a modular device that is paired with the surgical hub 106, 206. The surgical hub 106, 206 receives this data from the paired modular devices and other data sources and continually derive inferences (i.e., contextual information) about the ongoing procedure as new data is received, such as which step of the procedure is being performed at any given time. The situational awareness system of the surgical hub 106, 206 is able to, for example, record data pertaining to the procedure for generating reports, verify the steps being taken by the medical personnel, provide data or prompts (e.g., via a display screen) that are pertinent for the particular procedural step, adjust modular devices based on the context (e.g., activate monitors, adjust the field of view (FOV) of the medical imaging device, or change the energy level of an ultrasonic surgical instrument or RF electrosurgical instrument), and take any other such action described above.

As the first step 5202 in this illustrative procedure, the hospital staff members retrieve the patient's EMR from the hospital's EMR database. Based on select patient data in the EMR, the surgical hub 106, 206 determines that the procedure to be performed is a thoracic procedure.

Second step 5204, the staff members scan the incoming medical supplies for the procedure. The surgical hub 106, 206 cross-references the scanned supplies with a list of supplies that are utilized in various types of procedures and confirms that the mix of supplies corresponds to a thoracic procedure. Further, the surgical hub 106, 206 is also able to determine that the procedure is not a wedge procedure (because the incoming supplies either lack certain supplies that are necessary for a thoracic wedge procedure or do not otherwise correspond to a thoracic wedge procedure).

Third step 5206, the medical personnel scan the patient band via a scanner that is communicably connected to the surgical hub 106, 206. The surgical hub 106, 206 then confirms the patient's identity based on the scanned data.

Fourth step 5208, the medical staff turns on the auxiliary equipment. The auxiliary equipment being utilized can vary according to the type of surgical procedure and the techniques to be used by the surgeon, but in this illustrative case they include a smoke evacuator, insufflator, and medical imaging device. When activated, the auxiliary equipment that are modular devices can automatically pair with the surgical hub 106, 206 that is located within a particular vicinity of the modular devices as part of their initialization process. The surgical hub 106, 206 then derives contextual information about the surgical procedure by detecting the types of modular devices that pair with it during this pre-operative or initialization phase. In this particular example, the surgical hub 106, 206 determines that the surgical procedure is a VATS procedure based on this particular combination of paired modular devices. Based on the combination of the data from the patient's EMR, the list of medical supplies to be used in the procedure, and the type of modular devices that connect to the hub, the surgical hub 106, 206 generally infers the specific procedure that the surgical team will be performing. Once the surgical hub 106, 206 knows what specific procedure is being performed, the surgical hub 106, 206 then retrieves the steps of that procedure from a memory or from the cloud and then cross-reference the data it subsequently receives from the connected data sources (e.g., modular devices and patient monitoring devices) to infer what step of the surgical procedure the surgical team is performing.

Fifth step 5210, the staff members attach the EKG electrodes and other patient monitoring devices to the patient. The EKG electrodes and other patient monitoring devices are able to pair with the surgical hub 106, 206. As the surgical hub 106, 206 begins receiving data from the patient monitoring devices, the surgical hub 106, 206 thus confirms that the patient is in the operating theater.

Sixth step 5212, the medical personnel induce anesthesia in the patient. The surgical hub 106, 206 infers that the patient is under anesthesia based on data from the modular devices and/or patient monitoring devices, including EKG data, blood pressure data, ventilator data, or combinations thereof, for example. Upon completion of the sixth step 5212, the pre-operative portion of the lung segmentectomy procedure is completed and the operative portion begins.

Seventh step 5214, the patient's lung that is being operated on is collapsed (while ventilation is switched to the contralateral lung). The surgical hub 106, 206 infers from the ventilator data that the patient's lung has been collapsed, for example. The surgical hub 106, 206 infers that the operative portion of the procedure has commenced as it can compare the detection of the patient's lung collapsing to the expected steps of the procedure (which can be accessed or retrieved previously) and thereby determine that collapsing the lung is the first operative step in this particular procedure.

Eighth step 5216, the medical imaging device (e.g., a scope) is inserted and video from the medical imaging device is initiated. The surgical hub 106, 206 receives the medical imaging device data (i.e., video or image data) through its connection to the medical imaging device. Upon receipt of the medical imaging device data, the surgical hub 106, 206 determines that the laparoscopic portion of the surgical procedure has commenced. Further, the surgical hub 106, 206 determines that the particular procedure being performed is a segmentectomy, as opposed to a lobectomy (note that a wedge procedure has already been discounted by the surgical hub 106, 206 based on data received at the second step 5204 of the procedure). The data from the medical imaging device 124 (FIG. 2) is utilized to determine contextual information regarding the type of procedure being performed in a number of different ways, including by determining the angle at which the medical imaging device is oriented with respect to the visualization of the patient's anatomy, monitoring the number or medical imaging devices being utilized (i.e., that are activated and paired with the surgical hub 106, 206), and monitoring the types of visualization devices utilized. For example, one technique for performing a VATS lobectomy places the camera in the lower anterior corner of the patient's chest cavity above the diaphragm, whereas one technique for performing a VATS segmentectomy places the camera in an anterior intercostal position relative to the segmental fissure. Using pattern recognition or machine learning techniques, for example, the situational awareness system is trained to recognize the positioning of the medical imaging device according to the visualization of the patient's anatomy. As another example, one technique for performing a VATS lobectomy utilizes a single medical imaging device, whereas another technique for performing a VATS segmentectomy utilizes multiple cameras. As yet another example, one technique for performing a VATS segmentectomy utilizes an infrared light source (which can be communicably coupled to the surgical hub as part of the visualization system) to visualize the segmental fissure, which is not utilized in a VATS lobectomy. By tracking any or all of this data from the medical imaging device, the surgical hub 106, 206 thereby determines the specific type of surgical procedure being performed and/or the technique being used for a particular type of surgical procedure.

Ninth step 5218, the surgical team begins the dissection step of the procedure. The surgical hub 106, 206 infers that the surgeon is in the process of dissecting to mobilize the patient's lung because it receives data from the RF or ultrasonic generator indicating that an energy instrument is being fired. The surgical hub 106, 206 cross-references the received data with the retrieved steps of the surgical procedure to determine that an energy instrument being fired at this point in the process (i.e., after the completion of the previously discussed steps of the procedure) corresponds to the dissection step. The energy instrument is an energy tool mounted to a robotic arm of a robotic surgical system.

Tenth step 5220, the surgical team proceeds to the ligation step of the procedure. The surgical hub 106, 206 infers that the surgeon is ligating arteries and veins because it receives data from the surgical stapling and cutting instrument indicating that the instrument is being fired. Similarly to the prior step, the surgical hub 106, 206 derives this inference by cross-referencing the receipt of data from the surgical stapling and cutting instrument with the retrieved steps in the process. The surgical instrument is a surgical tool mounted to a robotic arm of a robotic surgical system.

Eleventh step 5222, the segmentectomy portion of the procedure is performed. The surgical hub 106, 206 infers that the surgeon is transecting the parenchyma based on data from the surgical stapling and cutting instrument, including data from its cartridge. The cartridge data corresponds to the size or type of staple being fired by the instrument, for example. As different types of staples are utilized for different types of tissues, the cartridge data thus indicates the type of tissue being stapled and/or transected. In this case, the type of staple being fired is utilized for parenchyma (or other similar tissue types), which allows the surgical hub 106, 206 to infer that the segmentectomy portion of the procedure is being performed.

Twelfth step 5224, the node dissection step is then performed. The surgical hub 106, 206 infers that the surgical team is dissecting the node and performing a leak test based on data received from the generator indicating that an RF or ultrasonic instrument is being fired. For this particular procedure, an RF or ultrasonic instrument being utilized after parenchyma was transected corresponds to the node dissection step, which allows the surgical hub 106, 206 to make this inference. It should be noted that surgeons regularly switch back and forth between surgical stapling/cutting instruments and surgical energy (i.e., RF or ultrasonic) instruments depending upon the particular step in the procedure because different instruments are better adapted for particular tasks. Therefore, the particular sequence in which the stapling/cutting instruments and surgical energy instruments are used can indicate what step of the procedure the surgeon is performing. Moreover, in certain instances, robotic tools can be utilized for one or more steps in a surgical procedure and/or handheld surgical instruments are utilized for one or more steps in the surgical procedure. The surgeon(s) alternate between robotic tools and handheld surgical instruments and/or use the devices concurrently, for example. Upon completion of the twelfth step 5224, the incisions are closed up and the post-operative portion of the procedure begins.

Thirteenth step 5226, the patient's anesthesia is reversed. The surgical hub 106, 206 infers that the patient is emerging from the anesthesia based on the ventilator data (i.e., the patient's breathing rate begins increasing), for example.

Lastly, the fourteenth step 5228 is that the medical personnel remove the various patient monitoring devices from the patient. The surgical hub 106, 206 thus infers that the patient is being transferred to a recovery room when the hub loses EKG, BP, and other data from the patient monitoring devices. As can be seen from the description of this illustrative procedure, the surgical hub 106, 206 determines or infers when each step of a given surgical procedure is taking place according to data received from the various data sources that are communicably coupled to the surgical hub 106, 206.

Situational awareness is further described in U.S. Provisional Patent Application Serial No. 62/611,341, titled INTERACTIVE SURGICAL PLATFORM, filed December 28, 2017. Operation of a robotic surgical system, including the various robotic surgical systems disclosed hereincontrolled by the hub 106, 206 based on its situational awareness and/or feedback from the components thereof and/or based on information from the cloud 102.

Various aspects of the subject matter described herein are set out in the following numbered examples.

Example 1. A minimally invasive image acquisition system comprising: a plurality of illumination sources wherein each illumination source is configured to emit light having a specified central wavelength; a first light sensing element having a first field of view and configured to receive illumination reflected from a first portion of a surgical site when the first portion of the surgical site is illuminated by at least one of the plurality of illumination sources; a second light sensing element having a second field of view and configured to receive illumination reflected from a second portion of the surgical site when the second portion of the surgical site is illuminated by at least one of the plurality of illumination sources, wherein the second field of view overlaps at least a portion of the first field of view; and a computing system, wherein the computing system is configured to: receive data from the first light sensing element, receive data from the second light sensing element, compute imaging data based on the data received from the first light sensing element and the data received from the second light sensing element, and transmit the imaging data for receipt by a display system.

Example 2. The minimally invasive image acquisition system of any one of Example 1, wherein the first field of view has a first angle and the second field of view has a second angle and the first angle is the same as the second angle.

Example 3. The minimally invasive image acquisition system of any one of Examples 1-2, wherein the first field of view has a first angle and the second field of view has a second angle and the first angle differs from the second angle.

Example 4. The minimally invasive image acquisition system of any one of Examples 1-3, wherein the first light sensing element has an optical component configured to adjust the first field of view.

Example 5. The minimally invasive image acquisition system of any one of Examples 1-4, wherein the second light sensing element has an optical component configured to adjust the second field of view.

Example 6. The minimally invasive image acquisition system of any one of Examples 1-5, wherein the second field of view overlaps all of the first field of view.

Example 7. The minimally invasive image acquisition system of any one of Examples 1-6, wherein the first field of view is completely enclosed by the second field of view.

Example 8. The minimally invasive image acquisition system of any one of Examples 1-7, wherein the first light sensing element and the second light sensing element are at least partially disposed within an elongated camera probe.

Example 9. The minimally invasive image acquisition system of any one of Examples 1-8, wherein each of the plurality of illumination source is configured to emit light having a specified central wavelength within a visible spectrum.

Example 10. The minimally invasive image acquisition system of any one of Examples 1-9, wherein at least one of the plurality of illumination source is configured to emit light having a specified central wavelength outside of a visible spectrum.

Example 11. The minimally invasive image acquisition system of any one of Example 10, wherein the specified central wavelength outside of the visible spectrum is within an ultra-violet range.

Example 12. The minimally invasive image acquisition system of any one of Examples 10-11, wherein the specified central wavelength outside of the visible spectrum is within an infrared range.

Example 13. The minimally invasive image acquisition system of any one of Examples 1-12, wherein the computing system configured to compute imaging data based on the data received from the first light sensing element and the data received from the second light sensing element comprises a computing system configured to perform a first data analysis on the data received from the first light sensing element and a second data analysis on the data received from the second light sensing element.

Example 14. The minimally invasive image acquisition system of any one of Example 13, wherein the first data analysis differs from the second data analysis.

Example 15. A minimally invasive image acquisition system comprising: a processor; and a memory coupled to the processor, the memory storing instructions executable by the processor to: control an operation of a plurality of illumination sources of a tissue sample wherein each illumination source is configured to emit light having a specified central wavelength; receive, from a first light sensing element, first data related to illumination reflected from a first portion of a surgical site when the first portion of the surgical site is illuminated by at least one of the plurality of illumination source, receive, from a second light sensing element, second data related to illumination reflected from a second portion of the surgical site when the second portion of the surgical site is illuminated by at least one of the plurality of illumination sources, wherein the second field of view overlaps at least a portion of the first field of view, compute imaging data based on the first data received from the first light sensing element and the second data received from the second light sensing element, and transmit the imaging data for receipt by a display system.

Example 16. The minimally invasive image acquisition system of any one of Example 15, wherein the memory coupled to the processor further stores instructions executable by the processor to receive, from a surgical instrument, operational data related to a function or status of the surgical instrument.

Example 17. The minimally invasive image acquisition system of any one of Example 16, wherein the memory coupled to the processor further stores instructions executable by the processor to compute imaging data based on the first data received from the first light sensing element, the second data received from the second light sensing element, and the operational data related to the function or status of the surgical instrument.

Example 18. A minimally invasive image acquisition system comprising: a control circuit configured to: control an operation of a plurality of illumination sources of a tissue sample wherein each illumination source is configured to emit light having a specified central wavelength; receive, from a first light sensing element, first data related to illumination reflected from a first portion of a surgical site when the first portion of the surgical site is illuminated by at least one of the plurality of illumination source, receive, from a second light sensing element, second data related to illumination reflected from a second portion of the surgical site when the second portion of the surgical site is illuminated by at least one of the plurality of illumination sources, wherein the second field of view overlaps at least a portion of the first field of view, compute imaging data based on the first data received from the first light sensing element and the second data received from the second light sensing element, and transmit the imaging data for receipt by a display system.

Example 19. A non-transitory computer readable medium storing computer readable instructions which, when executed, causes a machine to: control an operation of a plurality of illumination sources of a tissue sample wherein each illumination source is configured to emit light having a specified central wavelength; receive, from a first light sensing element, first data related to illumination reflected from a first portion of a surgical site when the first portion of the surgical site is illuminated by at least one of the plurality of illumination source, receive, from a second light sensing element, second data related to illumination reflected from a second portion of the surgical site when the second portion of the surgical site is illuminated by at least one of the plurality of illumination sources, wherein the second field of view overlaps at least a portion of the first field of view, compute imaging data based on the first data received from the first light sensing element and the second data received from the second light sensing element, and transmit the imaging data for receipt by a display system.

While several forms have been illustrated and described, it is not the intention of the applicant to restrict or limit the scope of the appended claims to such detail. Numerous modifications, variations, changes, substitutions, combinations, and equivalents to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description is intended to cover all such modifications, combinations, and variations as falling within the scope of the disclosed forms.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor comprising one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet switched network. The communication devices may be capable of communicating with each other using a selected packet switched network communications protocol. One example communications protocol may include an Ethernet communications protocol which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard", published in December, 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame relay communications protocol. The frame relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001, and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

**Claims**

1. A minimally invasive image acquisition system comprising:

a plurality of illumination sources (2136) wherein each illumination source is configured to emit light having a specified central wavelength;
an elongated camera probe (2024) having a distal end (2143);
a first light sensing element (2144a) having a first field of view (2147a) and configured to receive illumination reflected from a first portion of a surgical site when the first portion of the surgical site is illuminated by at least one of the plurality of illumination sources, wherein the first light sensing element is disposed at the distal end of the elongated camera probe;
a second light sensing element (2144b) having a second field of view (2147b) and configured to receive illumination reflected from a second portion of the surgical site when the second portion of the surgical site is illuminated by at least one of the plurality of illumination sources, wherein the second field of view overlaps at least a portion of the first field of view, wherein the second light sensing element is disposed at the distal end of the elongated camera probe; and
a computing system, wherein the computing system is configured to:

receive data from the first light sensing element,
receive data from the second light sensing element,
compute imaging data based on the data received from the first light sensing element and the data received from the second light sensing element, and
transmit the imaging data for receipt by a display system,

**characterised in that** the first field of view is wider than the second field of view and the second field of view is totally disposed within the first field of view.

2. The minimally invasive image acquisition system of claim 1, wherein the first light sensing element has an optical component configured to adjust the first field of view.

3. The minimally invasive image acquisition system of claim 1 or claim 2, wherein the second light sensing element has an optical component configured to adjust the second field of view.

4. The minimally invasive image acquisition system of any preceding claim, wherein each of the plurality of illumination source is configured to emit light having a specified central wavelength within a visible spectrum.

5. The minimally invasive image acquisition system of any of claims 1 to 3, wherein at least one of the plurality of illumination source is configured to emit light having a specified central wavelength outside of a visible spectrum.

6. The minimally invasive image acquisition system of claim 5, wherein the specified central wavelength outside of the visible spectrum is within an ultra-violet range.

7. The minimally invasive image acquisition system of claim 5, wherein the specified central wavelength outside of the visible spectrum is within an infrared range.

8. The minimally invasive image acquisition system of any preceding claim, wherein the computing system is configured to compute imaging data based on the data received from the first light sensing element and the data received from the second light sensing element comprises a computing system configured to perform a first data analysis on the data received from the first light sensing element and a second data analysis on the data received from the second light sensing element.

9. The minimally invasive image acquisition system of claim 8, wherein the first data analysis differs from the second data analysis.

10. The minimally invasive image acquisition system of claim 1, the computer system comprising:

a processor; and
a memory coupled to the processor, the memory storing instructions executable by the processor to:

control an operation of the plurality of illumination sources;
receive, from the first light sensing element, first data related to illumination reflected from the first portion of a surgical site when the first portion of the surgical site is illuminated by at least one of the plurality of illumination source,
receive, from the second light sensing element, second data related to illumination reflected from the second portion of the surgical site when the second portion of the surgical site is illuminated by at least one of the plurality of illumination sources, compute the imaging data based on the first data received from the first light sensing element and the second data received from the second light sensing element, and transmit the imaging data for receipt by the display system.

11. The minimally invasive image acquisition system of claim 1, the computing system comprising a control circuit, wherein the control circuit is further configured to control an operation of the plurality of illumination sources.

12. A non-transitory computer readable medium storing computer readable instructions which, when executed, causes a machine to:

control an operation of a plurality of illumination sources of a tissue sample wherein each illumination source is configured to emit light having a specified central wavelength;
receive, from a first light sensing element disposed at a distal end of an elongated camera probe, first data related to illumination reflected from a first portion of a surgical site when the first portion of the surgical site is illuminated by at least one of the plurality of illumination source,
receive, from a second light sensing element disposed at a distal end of an elongated camera probe, second data related to illumination reflected from a second portion of the surgical site when the second portion of the surgical site is illuminated by at least one of the plurality of illumination sources, wherein the second field of view overlaps at least a portion of the first field of view, wherein the first field of view is wider than the second field of view and the second field of view is totally disposed within the first field of view, wherein the second light sensing element is disposed at the distal end of the elongated camera probe,
compute imaging data based on the first data received from the first light sensing element and the second data received from the second light sensing element, and
transmit the imaging data for receipt by a display system.

**Patentansprüche**

1. Minimalinvasives Bilderfassungssystem, umfassend:

eine Vielzahl von Beleuchtungsquellen (2136), wobei jede Beleuchtungsquelle konfiguriert ist, um Licht auszusenden, das eine bestimmte zentrale Wellenlänge aufweist;
eine längliche Kamerasonde (2024), die ein distales Ende (2143) aufweist;
ein erstes Lichtsensorelement (2144a), das ein erstes Sichtfeld (2147a) aufweist und konfiguriert ist, um eine Beleuchtung zu empfangen, die von einem ersten Abschnitt einer Operationsstelle reflektiert wird, wenn der erste Abschnitt der Operationsstelle durch mindestens eine der Vielzahl von Beleuchtungsquellen beleuchtet wird, wobei das erste Lichtsensorelement an dem distalen Ende der länglichen Kamerasonde angeordnet ist;
ein zweites Lichterfassungselement (2144b), das ein zweites Sichtfeld (2147b) aufweist und konfiguriert ist, um die Beleuchtung zu empfangen, die von einem zweiten Abschnitt der Operationsstelle reflektiert wird, wenn der zweite Abschnitt der Operationsstelle durch mindestens eine der Vielzahl von Beleuchtungsquellen beleuchtet wird, wobei das zweite Sichtfeld mindestens einen Abschnitt des ersten Sichtfelds überlappt, wobei das zweite Lichterfassungselement an dem distalen Ende der länglichen Kamerasonde angeordnet ist; und
ein Computersystem, wobei das Computersystem konfiguriert ist zum:

Empfangen von Daten von dem ersten Lichtsensorelement,
Empfangen von Daten von dem zweiten Lichtsensorelement,
Berechnen von Bilddaten basierend auf den Daten, die von dem ersten Lichtsensorelement empfangen werden, und den Daten, die von dem zweiten Lichtsensorelement empfangen werden, und
Übertragen der Bilddaten für einen Empfang durch ein Anzeigesystem,
**dadurch gekennzeichnet, dass** das erste Sichtfeld breiter als das zweite Sichtfeld ist und das zweite Sichtfeld vollständig innerhalb des ersten Sichtfelds liegt.

2. Minimalinvasives Bilderfassungssystem nach Anspruch 1, wobei das erste Lichterfassungselement eine optische Komponente aufweist, die konfiguriert ist, um das erste Sichtfeld anzupassen.

3. Minimalinvasives Bilderfassungssystem nach Anspruch 1 oder 2, wobei das zweite Lichterfassungselement eine optische Komponente aufweist, die konfiguriert ist, um das zweite Sichtfeld anzupassen.

4. Minimalinvasives Bilderfassungssystem nach einem der vorstehenden Ansprüche, wobei jede der Vielzahl von Beleuchtungsquellen konfiguriert ist, um Licht auszusenden, das eine bestimmte zentrale Wellenlänge innerhalb eines sichtbaren Spektrums aufweist.

5. Minimalinvasives Bilderfassungssystem nach einem der Ansprüche 1 bis 3, wobei mindestens eine der Vielzahl Beleuchtungsquellen konfiguriert ist, um Licht auszusenden, das eine bestimmte zentrale Wellenlänge außerhalb des sichtbaren Spektrums aufweist.

6. Minimalinvasives Bilderfassungssystem nach Anspruch 5, wobei die bestimmte zentrale Wellenlänge außerhalb des sichtbaren Spektrums innerhalb eines Ultraviolettbereichs liegt.

7. Minimalinvasives Bilderfassungssystem nach Anspruch 5, wobei die bestimmte zentrale Wellenlänge außerhalb des sichtbaren Spektrums innerhalb eines Infrarotbereichs liegt.

8. Minimalinvasives Bilderfassungssystem nach einem der vorstehenden Ansprüche, wobei das Computersystem konfiguriert ist, um Bilddaten basierend auf den Daten, die von dem ersten Lichtsensorelement empfangen werden, und den Daten, die von dem zweiten Lichtsensorelement empfangen werden, zu berechnen, ein Computersystem umfasst, das konfiguriert ist, um eine erste Datenanalyse an den Daten, die von dem ersten Lichtsensorelement empfangen werden, und eine zweite Datenanalyse an den Daten, die von dem zweiten Lichtsensorelement empfangen werden, durchzuführen.

9. Minimalinvasives Bilderfassungssystem nach Anspruch 8, wobei sich die erste Datenanalyse von der zweiten Datenanalyse unterscheidet.

10. Minimalinvasives Bilderfassungssystem nach Anspruch 1, das Computersystem umfassend:

einen Prozessor; und
einen Speicher, der mit dem Prozessor gekoppelt ist, wobei der Speicher Anweisungen speichert, die durch den Prozessor ausführbar sind zum:

Steuern eines Betriebs der Vielzahl von Beleuchtungsquellen;
Empfangen, von dem ersten Lichtsensorelement, von ersten Daten bezüglich der Beleuchtung, die von dem ersten Abschnitt einer Operationsstelle reflektiert wird, wenn der erste Abschnitt der Operationsstelle durch mindestens eine der mehreren Beleuchtungsquellen beleuchtet wird,
Empfangen, von dem zweiten Lichtsensorelement, von zweiten Daten bezüglich der Beleuchtung, die von dem zweiten Abschnitt der Operationsstelle reflektiert wird, wenn der zweite Abschnitt der Operationsstelle durch mindestens eine der mehreren Beleuchtungsquellen beleuchtet wird,
Berechnen der Bilddaten basierend auf den ersten Daten, die von dem ersten Lichtsensorelement empfangen werden, und den zweiten Daten, die von dem zweiten Lichtsensorelement empfangen werden, und
Übertragen der Bilddaten für den Empfang durch das Anzeigesystem.

11. Minimalinvasives Bilderfassungssystem nach Anspruch 1, das Computersystem umfassend eine Steuerschaltung, wobei die Steuerschaltung ferner konfiguriert ist, um einen Betrieb der Vielzahl von Beleuchtungsquellen zu steuern.

12. Nicht flüchtiges computerlesbares Medium, das computerlesbare Anweisungen speichert, die, wenn sie ausgeführt werden, eine Maschine veranlassen zum:

Steuern eines Betriebs einer Vielzahl von Beleuchtungsquellen einer Gewebeprobe, wobei jede Beleuchtungsquelle konfiguriert ist, um Licht auszusenden, das eine bestimmte zentrale Wellenlänge aufweist;
Empfangen, von einem ersten Lichtsensorelement, das an einem distalen Ende einer länglichen Kamerasonde angeordnet ist, von ersten Daten bezüglich der Beleuchtung, die von einem ersten Abschnitt einer Operationsstelle reflektiert wird, wenn der erste Abschnitt der Operationsstelle durch mindestens eine der Vielzahl von Beleuchtungsquellen beleuchtet wird,
Empfangen, von einem zweiten Lichtsensorelement, das an einem distalen Ende einer länglichen Kamerasonde angeordnet ist, von zweiten Daten bezüglich der Beleuchtung, die von einem zweiten Abschnitt der Operationsstelle reflektiert wird, wenn der zweite Abschnitt der Operationsstelle durch mindestens eine der Vielzahl von Beleuchtungsquellen beleuchtet wird, wobei das zweite Sichtfeld mindestens einen Abschnitt des ersten Sichtfelds überlappt, wobei das erste Sichtfeld breiter als das zweite Sichtfeld ist und sich das zweite Sichtfeld vollständig innerhalb des ersten Sichtfelds befindet, wobei das zweite Lichtsensorelement an dem distalen Ende der länglichen Kamerasonde angeordnet ist,
Berechnen von Bilddaten basierend auf den ersten Daten, die von dem ersten Lichtsensorelement empfangen werden, und den zweiten Daten, die von dem zweiten Lichtsensorelement empfangen werden, und

Übertragen der Bilddaten für den Empfang durch ein Anzeigesystem.

**Revendications**

1. Système d'acquisition d'image à invasivité minimale comprenant :

   une pluralité de sources d'illumination (2136) dans lequel chaque source d'illumination est configurée pour émettre de la lumière ayant une longueur d'onde centrale spécifiée ;
   une caméra sonde allongée (2024) ayant une extrémité distale (2143) ;
   un premier élément de détection de lumière (2144a) ayant un premier champ visuel (2147a) et configuré pour recevoir l'illumination réfléchie par une première partie d'un site chirurgical lorsque la première partie du site chirurgical est illuminée par au moins l'une parmi la pluralité de sources d'illumination, dans lequel le premier élément de détection de lumière est disposé au niveau de l'extrémité distale de la caméra sonde allongée ;
   un second élément de détection de lumière (2144b) ayant un second champ visuel (2147b) et configuré pour recevoir l'illumination réfléchie par une seconde partie du site chirurgical lorsque la seconde partie du site chirurgical est illuminée par au moins l'une parmi la pluralité de sources d'illumination, dans lequel le second champ visuel chevauche au moins une partie du premier champ visuel, dans lequel le second élément de détection de lumière est disposé au niveau de l'extrémité distale de la caméra sonde allongée ; et
   un système informatique, dans lequel le système informatique est configuré pour :

      recevoir des données en provenance du premier élément de détection de lumière,
      recevoir des données en provenance du second élément de détection de lumière,
      calculer des données d'imagerie en fonction des données reçues du premier élément de détection de lumière et des données reçues du second élément de détection de lumière, et
      transmettre les données d'imagerie pour réception par un système d'affichage, **caractérisé en ce que** le premier champ visuel est plus large que le second champ visuel et le second champ visuel est totalement disposé au sein du premier champ visuel.

2. Système d'acquisition d'image à invasivité minimale selon la revendication 1, dans lequel le premier élément de détection de lumière a un composant optique conçu pour ajuster le premier champ visuel.

3. Système d'acquisition d'image à invasivité minimale selon la revendication 1 ou la revendication 2, dans lequel le second élément de détection de lumière a un composant optique conçu pour ajuster le second champ visuel.

4. Système d'acquisition d'image à invasivité minimale selon l'une quelconque revendication précédente, dans lequel chacune parmi la pluralité de sources d'illumination est configurée pour émettre de la lumière ayant une longueur d'onde centrale spécifiée au sein d'un spectre visible.

5. Système d'acquisition d'image à invasivité minimale selon l'une quelconque des revendications 1 à 3, dans lequel au moins l'une parmi la pluralité de sources d'illumination est configurée pour émettre de la lumière ayant une longueur d'onde centrale spécifiée à l'extérieur d'un spectre visible.

6. Système d'acquisition d'image à invasivité minimale selon la revendication 5, dans lequel la longueur d'onde centrale spécifiée à l'extérieur du spectre visible est au sein d'une plage ultraviolette.

7. Système d'acquisition d'image à invasivité minimale selon la revendication 5, dans lequel la longueur d'onde centrale spécifiée à l'extérieur du spectre visible est au sein d'une plage infrarouge.

8. Système d'acquisition d'image à invasivité minimale selon l'une quelconque revendication précédente, dans lequel le système informatique qui est configuré pour calculer des données d'imagerie en fonction des données reçues du premier élément de détection de lumière et des données reçues du second élément de détection de lumière comprend un système informatique configuré pour mettre en œuvre une première analyse de données sur les données reçues du premier élément de détection de lumière et une seconde analyse de données sur les données reçues du second élément de détection de lumière.

9. Système d'acquisition d'image à invasivité minimale selon la revendication 8, dans lequel la première analyse de données diffère de la seconde analyse de données.

10. Système d'acquisition d'image à invasivité minimale selon la revendication 1, le système informatique comprenant :

    un processeur ; et

une mémoire couplée au processeur, la mémoire stockant des instructions exécutables par le processeur pour :

commander un fonctionnement de la pluralité de sources d'illumination ;

recevoir, en provenance du premier élément de détection de lumière, des premières données apparentées à une illumination réfléchie par la première partie d'un site chirurgical lorsque la première partie du site chirurgical est illuminée par au moins l'une parmi la pluralité de sources d'illumination,

recevoir, en provenance du second élément de détection de lumière, des secondes données apparentées à une illumination réfléchie par la seconde partie du site chirurgical lorsque la seconde partie du site chirurgical est illuminée par au moins l'une parmi la pluralité de sources d'illumination,

calculer les données d'imagerie en fonction des premières données reçues du premier élément de détection de lumière et des secondes données reçues du second élément de détection de lumière, et

transmettre les données d'imagerie pour réception par le système d'affichage.

11. Système d'acquisition d'image à invasivité minimale selon la revendication 1, le système informatique comprenant un circuit de commande, dans lequel le circuit de commande est configuré en outre pour commander un fonctionnement de la pluralité de sources d'illumination.

12. Support non transitoire lisible par ordinateur stockant des instructions lisibles par ordinateur qui, lorsqu'elles sont exécutées, amènent une machine à :

commander un fonctionnement d'une pluralité de sources d'illumination d'un échantillon de tissu dans lequel chaque source d'illumination est configurée pour émettre de la lumière ayant une longueur d'onde centrale spécifiée ;

recevoir, en provenance d'un premier élément de détection de lumière disposé au niveau d'une extrémité distale d'une caméra sonde allongée, des premières données apparentées à une illumination réfléchie par une première partie d'un site chirurgical lorsque la première partie du site chirurgical est illuminée par au moins l'une parmi la pluralité de source d'illumination,

recevoir, en provenance d'un second élément de détection de lumière disposé au niveau d'une extrémité distale d'une caméra sonde allongée, des secondes données apparentées à une illumination réfléchie par une seconde partie du site chirurgical lorsque la seconde partie du site chirurgical est illuminée par au moins l'une parmi la pluralité de sources d'illumination, dans lequel le second champ visuel chevauche au moins une partie du premier champ visuel, dans lequel le premier champ visuel est plus large que le second champ visuel et le second champ visuel est totalement disposé au sein du premier champ visuel, dans lequel le second élément de détection de lumière est disposé au niveau de l'extrémité distale de la caméra sonde allongée,

calculer des données d'imagerie en fonction des premières données reçues du premier élément de détection de lumière et des secondes données reçues du second élément de détection de lumière, et

transmettre les données d'imagerie pour réception par un système d'affichage.

FIG. 1

EP 3 505 041 B1

FIG. 2

102

135

MONITOR

106

138 — IMAGING MODULE

140 — GENERATOR MODULE

142 — MONOPOLAR

144 — BIPOLAR

146 — ULTRASONIC

126 — SMOKE EVACUATION MODULE

128 — SUCTION/IRRIGATION MODULE

130 — COMMUNICATION MODULE

132 — PROCESSOR MODULE

134 — STORAGE ARRAY

133 — OPERATING-ROOM MAPPING MODULE

136

VISUALIZATION SYSTEM
108

ROBOTIC SYSTEM
110

INTELLIGENT INSTRUMENT
112

FIG. 3

FIG. 5

FIG. 4

FIG. 6

FIG. 7

FIG. 8

**FIG. 9**

FIG. 10

FIG. 11

FIG. 12

PROCESSOR
502

INSTRUCTION
PROCESSING UNIT
506

CONTROL

ARITHMETIC UNIT
508

500

ADDRESSES

INSTRUCTIONS

DATA

MEMORY
504

FIG. 13

510

INPUT
514

COMBINATIONAL
LOGIC
512

OUTPUT
516

FIG. 14

520

INPUT
526

COMBINATIONAL
LOGIC
522

OUPUT
528

MEMORY
524

CLOCK
529

FIG. 15

FIG. 16

EP 3 505 041 B1

FIG. 17

FIG. 18

EP 3 505 041 B1

FIG. 19

FIG. 20

FIG. 21

FIG. 22A

FIG. 22B

FIG. 22C

FIG. 22D

FIG. 23A

FIG. 23B

FIG. 23C

FIG. 23D

FIG. 23E

| 8.3ms | 8.3ms | 8.3ms | 8.3ms | 8.3ms | 8.3ms | 8.3ms | 8.3ms |
|---|---|---|---|---|---|---|---|
| Cr+λ Y | Y | Cb+δY | Y | Cr+λ Y | Y | Cb+δY | Y | Cr+λ Y |

R
G
B

R
G
B

R
G
B

R
G
B

| 16.67ms | 16.67ms | 16.67ms |

FIG. 23F

FIG. 24

FIG. 25

FIG. 26C

FIG. 26B

FIG. 26A

FIG. 27

EP 3 505 041 B1

FIG. 28

EP 3 505 041 B1

FIG. 29

EP 3 505 041 B1

FIG. 30C

FIG. 30A

FIG. 30B

EP 3 505 041 B1

FIG. 30D

FIG. 31

FIG. 32

FIG. 33

FIG. 34

FIG. 35

—2900

Emitting a first light
beam having a first
central wavelength
to a tissue
—2910

Emitting a second light
beam having a second
central wavelength to
the tissue
—2916

Receiving a first
reflected light from the
tissue illuminated by
the first light beam
—2912

Receiving a second
reflected light from the
tissue illuminated by
the second light beam
—2918

Calculating a first
Doppler shift based on
the first emitted light
beam and the first
reflected light from
the tissue
—2914

Calculating a second
Doppler shift based on
the second emitted light
beam and the second
reflected light from
the tissue
—2920

Calculating a depth of a feature from
a surface of the tissue based on first
central wavelength, the first Doppler
shift, the second central wavelength,
and the second Doppler shift
—2922

FIG. 36

FIG. 37

EP 3 505 041 B1

FIG. 38

FIG. 39

FIG. 40

EP 3 505 041 B1

FIG. 41A

FIG. 41B

FIG. 41C

```
                                                        ⟋2950

        ⟋2960                                      ⟋2966
┌─────────────────────┐           ┌─────────────────────┐
│   Emitting a first  │           │ Emitting a second   │
│   light beam having │           │ light beam having   │
│   a first central   │           │ a second central    │
│   wavelength        │──────┐    │ wavelength to       │
│   to a tissue       │      │    │ the tissue          │
└─────────────────────┘      │    └─────────────────────┘
          │                  │              │
          ▼    ⟋2962         │              ▼    ⟋2968
┌─────────────────────┐      │    ┌─────────────────────┐
│  Receiving a first  │      │    │  Receiving a second │
│  reflected light    │      │    │  reflected light    │
│  from the tissue    │      │    │  from the tissue    │
│  illuminated by     │      │    │  illuminated by     │
│  the first light    │      │    │  the second light   │
│  beam               │      │    │  beam               │
└─────────────────────┘      │    └─────────────────────┘
          │                  │              │
          ▼    ⟋2964         │              ▼    ⟋2970
┌─────────────────────┐      │    ┌─────────────────────┐
│ Calculating a first │      │    │  Calculating a      │
│ tissue surface      │      │    │  second tissue      │
│ characteristic at   │      │    │  surface            │
│ a first depth base  │──────┘    │  characteristic at  │
│ on the first        │           │  a second depth     │
│ emitted light beam  │           │  based on the       │
│ and the first       │           │  second emitted     │
│ reflected light     │           │  light beam and     │
│ from the tissue     │           │  the second         │
└─────────────────────┘           │  reflected light    │
                                  │  from the tissue    │
                                  └─────────────────────┘
                                            │
                                            ▼      ⟋2972
   ⟋2974              ┌──────────────────────────────────┐
┌───────────────┐     │  Calculating a parameter related │
│ Providing the │     │  to the operation of a smart     │
│ parameter to  │     │  surgical instrument based on     │
│ the smart     │◄────│  the first tissue surface         │
│ surgical      │     │  characteristic, the second       │
│ instrument    │     │  tissue surface characteristic,   │
└───────────────┘     │  the first depth and the second   │
                      │  depth                            │
                      └──────────────────────────────────┘
```

# FIG.  42

FIG. 43A

FIG. 43B

FIG. 44A

FIG. 44B

112

FIG. 44C

EP 3 505 041 B1

FIG. 45

EP 3 505 041 B1

FIG. 46A

FIG. 46B

EP 3 505 041 B1

FIG. 46C

EP 3 505 041 B1

FIG. 46D

EP 3 505 041 B1

FIG. 47A

FIG. 47B

FIG. 47C

FIG. 48B

FIG. 48A

FIG. 49A

FIG. 49B

2985

2984

2987

2936

2937

FIG. 49C

FIG. 50

5200

| WHAT THE HUB KNOWS | THORACIC PROCEDURE | NOT A WEDGE PROCEDURE | CONFIRM PATIENT | VATS | CONFIRM PATIENT IS IN O.R. | PATIENT UNDER | PROCEDURE BEGINS | CONFIRM LOBECTOMY vs. SEGMENTECTOMY LAP PORTION STARTS |
|---|---|---|---|---|---|---|---|---|
| TYPE OF DATA | SELECT PATIENT DATA 5202 | SCAN PRODUCTS 5204 | UNIQUE ID 5206 | SMOKE EVAC. DATA INSUFFLATION DATA SCOPE DATA 5208 | EKG DATA 5210 | EKG, BP AND VENTILATOR DATA 5212 | VENTILATOR DATA 5214 | SCOPE DATA 5216 |
| PROCEDURE STEP | PULL ELECTRONIC MEDICAL RECORDS | SCAN INCOMING SUPPLIES | SCAN PATIENT BAND | TURN ON HUB AUXILIARY EQUIPMENT | ATTACH EKG | INDUCE ANESTHESIA | COLLAPSE LUNG | SCOPE IMAGE |

| WHAT THE HUB KNOWS | DISSECT TO MOBILIZE LUNG | LIGATE ARTERY & VEIN | TRANSECT PARENCHYMA | DISSECT NODES LEAK TEST | PATIENT EMERGENCE | PATIENT TRANSFER TO RECOVERY ROOM |
|---|---|---|---|---|---|---|
| TYPE OF DATA | GENERATOR DATA 5218 | STAPLER DATA 5220 | STAPLER & CARTRIDGE DATA 5222 | GENERATOR DATA 5224 | VENTILATOR DATA 5226 | LOSS OF EKG DATA LOSS OF BP DATA 5228 |
| PROCEDURE STEP | DISSECTION | LIGATION | SEGMENTECTOMY | NODE DISSECTION | REVERSE ANESTHESIA | REMOVE MONITORS |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8027710 B1
- EP 3225151 A1
- US 62611339
- US 62611340
- US 62611341
- US 7995045 B
- US 7982776 B
- US 20110306840
- US 20140243597
- US 20170296213
- US 9345481 B
- US 20140263552
- US 62817517
- US 63682917
- US 72085217
- US 63609617
- US 9060775 B
- US 20170086914
- US 20140268860
- US 9777913 B
- US 20140160318
- US 9516239 B
- US 20140160319
- US 9743016 B
- US 20140267655
- US 9641815 B

**Non-patent literature cited in the description**

- IEEE 802.3 Standard. *Institute of Electrical and Electronics Engineers (IEEE),* December 2008
- ATM-MPLS Network Interworking 2.0. *ATM Forum,* August 2001